# EUROPEAN PATENT APPLICATION

(11) **EP 1 806 334 A1**
(43) Date of publication of application: **11.07.2007**
(21) Application number: 05795839.9
(22) Date of filing: 18.10.2005
(51) Int. Cl.: C07C 211/61, C07C 211/55, C09K 11/06, H05B 33/14, H05B 33/22

(54) **AROMATIC AMINE COMPOUND AND ORGANIC ELECTROLUMINESCENT DEVICE USING SAME**

(30) Priority: 29.10.2004 JP 2004316937
(71) Applicant: IDEMITSU KOSAN CO., LTD., Tokyo 100-8321 (JP)
(72) Inventor: KAWAMURA, Masahiro, 2990293 (JP); KAWAMURA, Hisayuki, 2990293 (JP); HOSOKAWA, Chishio, 2990293 (JP)
(74) Representative: Gille Hrabal Struck Neidlein Prop Roos
(86) International application number: PCT/JP2005/019122
(87) International publication number: WO 2006/046441

(57) **Abstract**

An aromatic amine compound of a specific structure having at least one fluorene structure. An organic electroluminescence device which comprises one or more organic thin film layers comprising at least one light emitting layer sandwiched between a cathode and an anode, wherein at least one of the thin film layers comprises the aromatic amine compound according to claim 1 singly or as its mixture component. Such an organic electroluminescence device has various luminescent hues, high heat resistance, long lifetime, high luminance and high luminous efficiency. The above-mentioned novel aromatic amine compound enables to realize such an organic electroluminescence device.

## Description

### TECHNICAL FIELD

The present invention relates to an aromatic amine compound and an organic electroluminescent device using the same. More specifically, the present invention relates to an organic electroluminescence device showing various hues of light emission and having high heat resistance, a long lifetime, high emission luminance, and high emission efficiency and further, relates to a novel aromatic amine compound for realizing the organic electroluminescence device.

An organic electroluminescence (EL) device using an organic substance has been used as a light source such as a flat light emitter for a wall television or a backlight for a display, and has been vigorously developed. The electroluminescence phenomenon of an organic substance was observed in 1963 by Pope et al. in an anthracene single crystal (Non-Patent Document 1). In 1965, Helfinch and Schneider succeeded in observing relatively strong injection type EL by using a solution electrode system having good injection efficiency (Non-Patent Document 2). As reported since then, research has been conducted on the formation of an organic luminous substance using a conjugate organic host substance and a conjugate organic activator having a fused benzene ring. Examples of the organic host substance include naphthalene, anthracene, phenanthrene, tetracene, pyrene, benzopyrene, chrysene, picene, carbazole, fluorene, biphenyl, terphenyl, triphenylene oxide, dihalobiphenyl, trans-stilbene, and 1,4-diphenylbutadiene. Examples of the activator include anthracene, tetracene, and pentacene. However, each of those organic luminous substances is present in the form of a single layer having a thickness in excess of 1 µm, so a high electric field has been needed to cause each of the substances to emit light. In view of the foregoing, research on a thin-film device produced by a vacuum vapor deposition method has been advanced (for example, Non-Patent Document 3). However, a reduction in thickness of a device did not succeed in providing the device with high luminance at a practical level, though the reduction was effective in reducing the voltage at which the device was driven. In view of the foregoing, Tang et al. have devised an organic EL device obtained with laminating by vacuum vapor deposition, two extremely thin films (a hole transporting layer and a light emitting layer) between an anode and a cathode, and have realized high luminance while driving the device at a low voltage (Non-Patent Document 4 or Patent Document 1). After that, the development of organic compounds for use in the hole transporting layer and the light emitting layer has been advanced for a dozen or so years, whereby a lifetime and luminous efficiency at practical levels have been achieved. As a result, the organic EL device has started to find use in practical applications typified by the display portion of a car stereo or of a portable phone.
However, the properties of the device such as emission luminance and durability against deterioration over time due to long-term use are not high enough to allow the device to be put into practical use, and additional improvements in such properties have been requested.

To solve such problems, an oligomer (trimer or tetramer) amine has been used as a hole injecting/transporting material for improving a glass transition temperature (Tg). For example, Patent Document 2 discloses a compound represented by the following general formula (A): 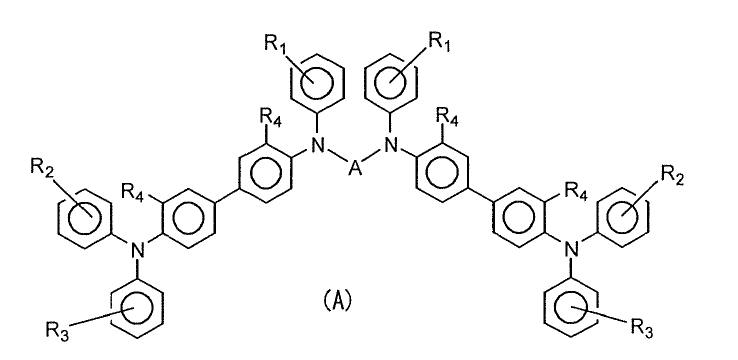 wherein R₁, R₂, and R₃ may be identical to or different from one another, and each represent a hydrogen atom, a lower alkyl group, or a lower alkoxy group; and R₄ represents a hydrogen atom, a lower alkyl group, a lower alkoxy group, or a chlorine atom; A is expressed by any one of the following structures: 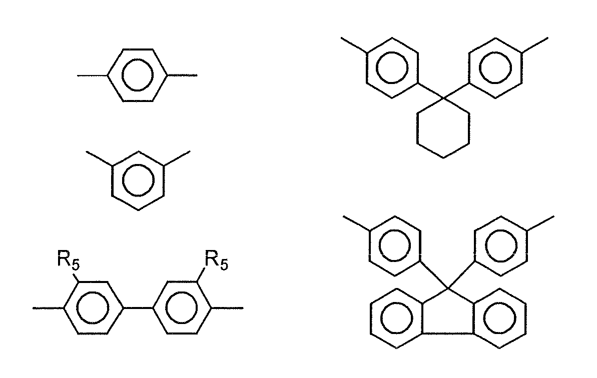 ; and
R₅ represents a hydrogen atom, a lower alkyl group, a lower alkoxy group, or a chlorine atom.

Patent Document 3 discloses a compound expressed by the following general formula (B):

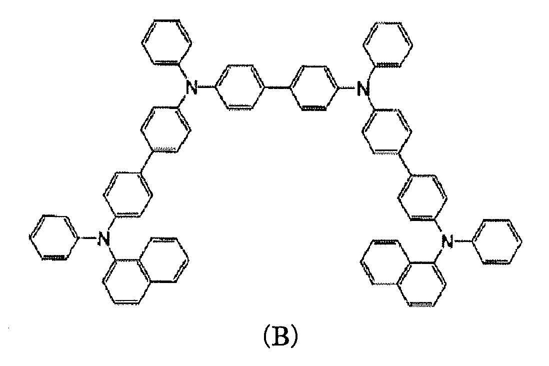

Patent Document 4 discloses a compound represented by the following general formula (C): 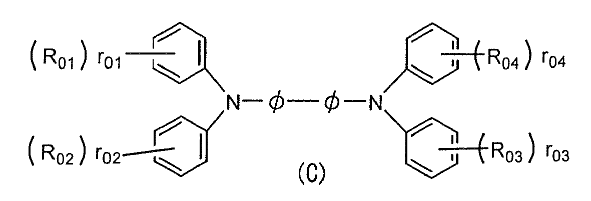 wherein R₀₁ to R₀₄ each represent any one of 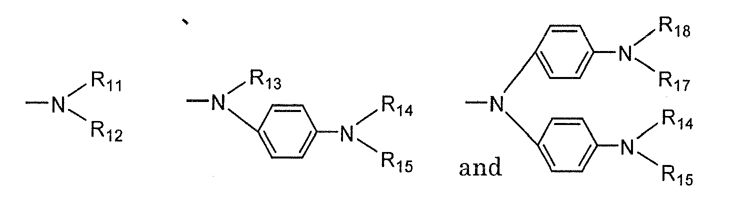 ; and
Φ represents a phenylene group; R₀₁, R₀₂, R₀₃, and R₀₄ each represent a diarylaminophenylene group; r₀₁, r₀₂, r₀₃, and r₀₄ each represent an integer of 0 to 5, while r₀₁ + r₀₂ + r₀₃ + r₀₄ make 1 or more and further; R₁₁, R₁₂, R₁₃, R₁₄, R₁₅, R₁₆, and R₁₇ each represent a substituted or unsubstituted aryl group.

Patent Document 5 discloses a compound represented by the following general formula (D): 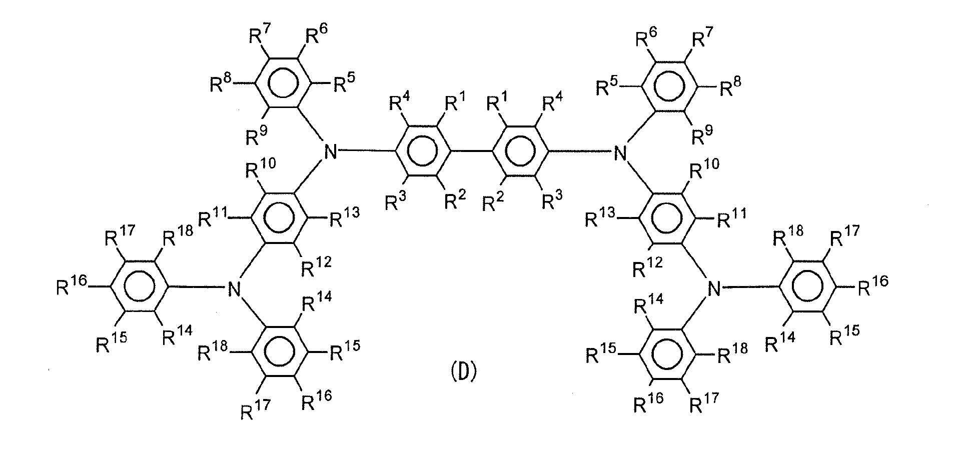 wherein R¹ to R¹⁸ each independently represent a group selected from the group consisting of a hydrogen atom, a lower alkyl group, and a lower alkoxy group.

Further, Patent Document 6 discloses a compound represented by the following general formula (E): 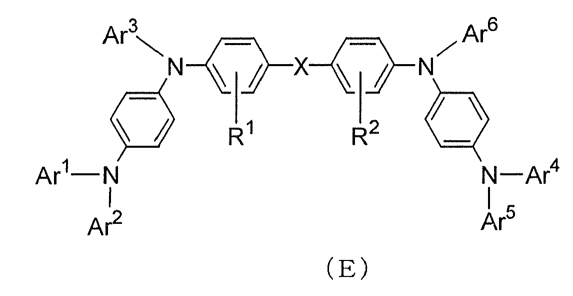 wherein Ar¹ to Ar⁶ each represent a hydrogen atom, an alkyl or alkoxy group having 1 to 6 carbon atoms, an aryl group having 6 to 24 ring carbon atoms, or an aryl group which has 6 to 24 ring carbon atoms and which may be substituted by a styryl group; **X** represents a linking group selected from a single bond, an arylene group having 6 to 24 ring carbon atoms, an alkylene group having 1 to 6 carbon atoms, a diphenylmethylene group, an ether bond, a thioether bond, a substituted or unsubstituted vinyl bond, and an aromatic heterocyclic ring; and further, R¹ and R² each represent an alkyl or alkoxy group having 1 to 6 carbon atoms, or a hydrogen atom, and may bond each other to form a substituted or unsubstituted, five-membered or six-membered ring.
However, none of the compounds described in Patent Documents 2 to 6 can provide sufficient hole injecting property.

In addition, a compound into which a fluorenyl group is introduced has been used as means for improving hole injecting property. For example, Patent Document 7 discloses a light emitting device using a compound represented by the following general formula (F) as a hole transporting material: 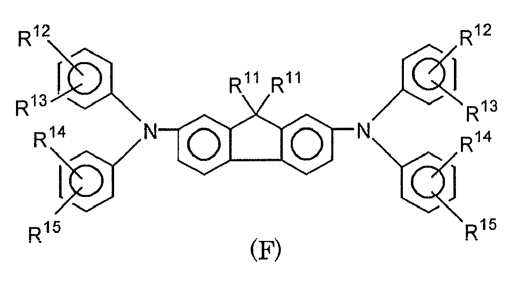 wherein R¹¹ represents an alkyl group or an aralkyl group; R¹², R¹³, R¹⁴, and R¹⁵ each represent a hydrogen atom, an alkyl group, an alkoxy group, or a halogen atom.
The compound has a Tg of 100°C or lower. A device using the compound cannot be put into practical use owing to its short lifetime and the absence of heat resistance.

Patent Document 8 discloses a light emitting device using a compound represented by the following compound (G): 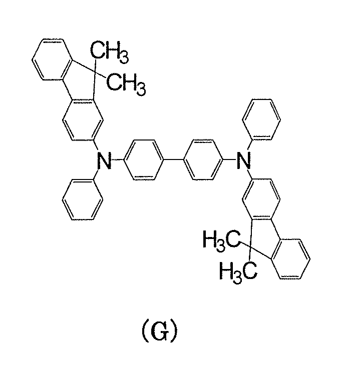 obtained by modifying the above general formula (F) as a hole transporting material.
Improvements in glass transition temperature and hole injecting property are observed in the compound, but the compound involves a problem in that the lifetime of a device using the compound is still short.

Further, Patent Document 9 discloses a compound represented by the following general formula (H): 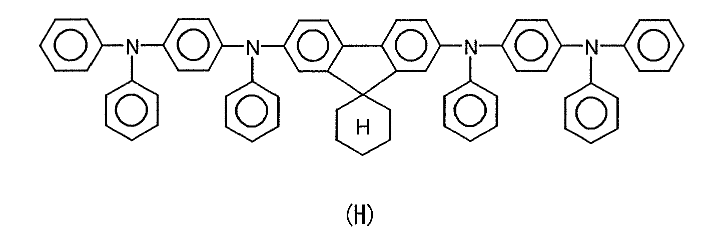 An improvement in hole injecting property is observed in the compound as well, but additional increases in glass transition temperature and lifetime have been demanded of the compound.

Patent Document 1: US 4356429
Patent Document 2: Japanese registered patent No.3220950
Patent Document 3: JP 2000-86595 A
Patent Document 4: JP 2000-156290 A
Patent Document 5: JP 09-301934 A
Patent Document 6: JP 2000-309566 A
Patent Document 7: JP 05-254723 A
Patent Document 8: JP 11-35532 A
Patent Document 9: JP 2000-80433 A
Non Patent Document 1: J. Chem. Phys. 38(1963)2042
Non Patent Document 2: Phys. Rev. Lett. 14(1965)229
Non Patent Document 3: Thin Solid Films 94(1982)171
Non Patent Document 4: Appl. Phys. Lett. 51(1987)913

### DISCLOSURE OF THE INVENTION

### PROBLEMS TO BE SOLVED BY THE INVENTION

The present invention has been made with a view to solving the above-mentioned problems, and an object of the present invention is to provide an organic EL device showing various hues of light emission and having high heat resistance, a long lifetime, high emission luminance, and high emission efficiency, and to provide a novel aromatic amine compound for realizing the organic EL device.

### MEANS FOR SOLVING THE PROBLEMS

The inventors of the present invention have made extensive studies with a view to achieving the above object. As a result, they have found that an aromatic amine compound having at least one fluorene structure and represented by the following general formula (1) can achieve the above obj ect. Thus, the inventors have completed the present invention.
That is, according to the present invention, there is provided an aromatic amine compound represented by the following general formula (1):

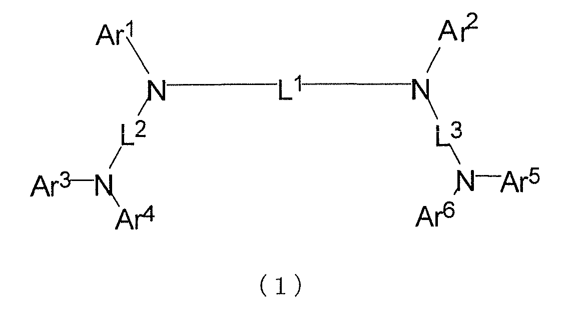

wherein Ar¹ to Ar⁶ each independently represent a substituted or unsubstituted aryl group having 5 to 60 ring carbon atoms, or a substituted or unsubstituted heteroaryl group having 3 to 50 ring carbon atoms; and
L¹ to L³ each independently represent a substituted or unsubstituted arylene group having 5 to 60 ring carbon atoms, or a substituted or unsubstituted heteroarylene group having 3 to 50 ring carbon atoms;
provided that the general formula (1) satisfies at least one of the following conditions (i) and (ii):
(i) at least one of Ar¹ to Ar⁶ represents a substituted or unsubstituted fluorenyl-containing group; and
(ii) at least one of L² and L³ represents a substituted or unsubstituted fluorenylene-containing group.

According to the present invention, there is provided an organic EL device comprising one or more organic thin film layers which comprise at least a light emitting layer sandwiched between a cathode and an anode, wherein at least one layer among the organic thin film layers comprises the aromatic amine compound singly or as its mixture component.

### EFFECT OF THE INVENTION

An organic EL device using the aromatic amine compound of the present invention shows various hues of light emission, and has high heat resistance. In particular, when the aromatic amine compound of the present invention is used as a hole injecting/transporting material, the organic EL device has a long lifetime, high emission luminance, and high emission efficiency.

### PREFERRED EMBODIMENTS FOR CARRYING OUT THE INVENTION

According to the present invention, there is provided an aromatic amine compound represented by the following general formula (1):

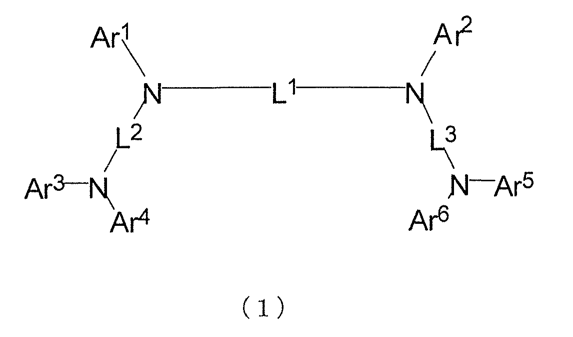

In the general formula (1), Ar¹ to Ar⁶ each independently represent a substituted or unsubstituted aryl group having 5 to 60 ring carbon atoms, or a substituted or unsubstituted heteroaryl group having 3 to 50 ring carbon atoms.
An aryl group represented by any one of Ar¹ to Ar⁶ preferably has 6 to 20 ring carbon atoms, and examples of such group include a fluorenyl group, a fluorenyl-containing group, a phenyl group, a biphenyl group, a terphenyl group, a naphthyl group, an anthryl group, and a fluoranthenyl group.
A heteroaryl group represented by any one of Ar¹ to Ar⁶ preferably has 6 to 20 ring carbon atoms, and examples of such group include a furanyl group, a thiophenyl group, a pyrrolyl group, an imidazolyl group, a pyrazolyl group, a triazolyl group, an oxadiazolyl group, a pyridinyl group, a pyrazinyl group, a triazinyl group, a pyrimidinyl group, a benzofuranyl group, a dibenzofuranyl group, a benzothiophenyl group, a dibenzothiophenyl group, a carbazolyl group, a quinoxalinyl group, a quinolinyl group, a benzimidazolyl group, and an imidazopyridinyl group.

In the general formula (1), L¹ to L³ each independently represent a substituted or unsubstituted arylene group having 5 to 60 ring carbon atoms, or a substituted or unsubstituted heteroarylene group having 3 to 50 ring carbon atoms.
An arylene group represented by any one of L¹ to L³ preferably has 6 to 20 ring carbon atoms, and examples of such group include a fluorenylene group, a fluorenylene-containing group, a phenylene group, a phenylene group, a biphenylene group, a naphthylene group, an anthrylene group, a chrysenylene group, a phenanthrylene group, a binaphthylene group, a terphenylene group, a quarter-phenylene group, a diphenylnaphthylene group, a phenylnaphthylene group, a benzofluorenylene group, and a dibenzofluorenylene group.
A heteroarylene group represented by any one of L¹ to L³ preferably has 6 to 20 ring carbon atoms, and examples of such group include a furanylene group, a thiophenylene group, a pyrrolylene group, an imidazolylene group, a pyrazolylene group, a triazolylene group, an oxadiazolylene group, a pyridinylene group, a pyrazinylene group, a triazinylene group, a pyrimidinylene group, a benzofuranylene group, a dibenzofuranylene group, a benzothiophenylene group, a dibenzothiophenylene group, a carbazolylene group, a quinoxalinylene group, a quinolinylene group, a benzimidazolylene group, and an imidazopyridinylene group.

Examples of a substituent for any one of Ar¹ to Ar⁶ and L¹ to L³ described above include: an alkyl group (having preferably 1 to 20, more preferably 1 to 12, or particularly preferably 1 to 8 carbon atoms such as a methyl, ethyl, iso-propyl, tert-butyl, n-octyl, n-decyl, n-hexadecyl, cyclopropyl, cyclopentyl, or cyclohexyl group); an alkenyl group (having preferably 2 to 20, more preferably 2 to 12, or particularly preferably 2 to 8 carbon atoms such as a vinyl, allyl, 2-butenyl, or 3-pentenyl group); an alkynyl group (having preferably 2 to 20, more preferably 2 to 12, or particularly preferably 2 to 8 carbon atoms such as a propargyl or 3-pentinyl group); an amino group (having preferably 0 to 20, more preferably 0 to 12, or particularly preferably 0 to 6 carbon atoms such as an amino, methylamino, dimethylamino, diethylamino, diphenylamino, or dibenzylamino group); an alkoxy group (having preferably 1 to 20, more preferably 1 to 12, or particularly preferably 1 to 8 carbon atoms such as a methoxy, ethoxy, or butoxy group); an aryloxy group (having preferably 6 to 20, more preferably 6 to 16, or particularly preferably 6 to 12 carbon atoms such as a phenyloxy or 2-naphthyloxy group); an acyl group (having preferably 1 to 20, more preferably 1 to 16, or particularly preferably 1 to 12 carbon atoms such as an acetyl, benzoyl, formyl, or pivaloyl group); an alkoxycarbonyl group (having preferably 2 to 20, more preferably 2 to 16, or particularly preferably 2 to 12 carbon atoms such as a methoxycarbonyl or ethoxycarbonyl group) ;an aryloxycarbonyl group (having preferably 7 to 20, more preferably 7 to 16, or particularly preferably 7 to 10 carbon atoms such as a phenyloxycarbonyl group) ; an acyloxy group (having preferably 2 to 20, more preferably 2 to 16, or particularly preferably 2 to 10 carbon atoms such as an acetoxy or benzoyloxy group); an acylamino group (having preferably 2 to 20, more preferably 2 to 16, or particularly preferably 2 to 10 carbon atoms such as an acetylamino or benzoylamino group); an alkoxycarbonylamino group (having preferably 2 to 20, more preferably 2 to 16, or particularly preferably 2 to 12 carbon atoms such as a methoxycarbonylamino group);an aryloxycarbonylamino group (having preferably 7 to 20, more preferably 7 to 16, or particularly preferably 7 to 12 carbon atoms such as a phenyloxycarbonylamino group); a sulfonylamino group (having preferably 1 to 20, more preferably 1 to 16, or particularly preferably 1 to 12 carbon atoms such as a methanesulfonylamino or benzenesulfonylamino group); a sulfamoyl group (having preferably 0 to 20, more preferably 0 to 16, or particularly preferably 0 to 12 carbon atoms such as a sulfamoyl, methylsulfamoyl, dimethylsulfamoyl, or phenylsulfamoyl group); a carbamoyl group (having preferably 1 to 20, more preferably 1 to 16, or particularly preferably 1 to 12 carbon atoms such as a carbamoyl, methylcarbamoyl, diethylcarbamoyl, or phenylcarbamoyl group); an alkylthio group (having preferably 1 to 20, more preferably 1 to 16, or particularly preferably 1 to 12 carbon atoms such as a methylthio or ethylthio group) ; an arylthio group (having preferably 6 to 20, more preferably 6 to 16, or particularly preferably 6 to 12 carbon atoms such as a phenylthio group) ; a sulfonyl group (having preferably 1 to 20, more preferably 1 to 16, or particularly preferably 1 to 12 carbon atoms such as a mesyl or tosyl group); a sulfinyl group (having preferably 1 to 20, more preferably 1 to 16, or particularly preferably 1 to 12 carbon atoms such as a methanesulfinyl or benzenesulfinyl group) ; a ureido group (having preferably 1 to 20, more preferably 1 to 16, or particularly preferably 1 to 12 carbon atoms such as a ureido, methylureido, or phenylureido group) ; a phosphoric amide group (having preferably 1 to 20, more preferably 1 to 16, or particularly preferably 1 to 12 carbon atoms such as a diethylphosphoric amide or phenylphosphoric amide group); a hydroxyl group; a mercapto group; a halogen atom (such as a fluorine atom, a chlorine atom, a bromine atom, or an iodine atom); a cyano group; a sulfo group; a carboxyl group; a nitro group; a hydroxamic acid group; a sulfino group; a hydrazino group; an imino group; a heterocyclic group (having preferably 1 to 30, or more preferably 1 to 12 carbon atoms, and containing, for example, a nitrogen atom, an oxygen atom, or a sulfur atom as a hetero atom such as an imidazolyl, pyridyl, quinolyl, furyl, thienyl, piperidyl, morpholino, benzoxazolyl, benzoimidazolyl, benzothiazolyl, or carbazolyl group) ; and a silyl group (having preferably 3 to 40, more preferably 3 to 30, or particularly preferably 3 to 24 carbon atoms such as a trimethylsilyl or triphenylsilyl group).

Each of those substituents may be additionally substituted. In addition, when two or more substituents are present, the substituents may be identical to or different from each other. In addition, if possible, the substituents may be linked to each other to form a cyclic structure. Examples of the cyclic structure include: a cycloalkane having 4 to 12 carbon atoms such as cyclobutane, cyclopentane, cyclohexane, adamantane, or norbornane; a cycloalkene having 4 to 12 carbon atoms such as cyclobutene, cyclopentene, cyclohexene, cycloheptene, or cyclooctene; a cycloalkadiene having 6 to 12 carbon atoms such as cyclohexadiene, cycloheptadiene, or cyclooctadiene; and an aromatic ring having 6 to 50 carbon atoms such as benzene, naphthalene, phenanthrene, anthracene, pyrene, chrysene, or acenaphthylene.

The aromatic amine compound represented by the general formula (1) of the present invention satisfies at least one of the following conditions (i) and (ii):
(i) at least one of Ar¹ to Ar⁶ represents a substituted or unsubstituted fluorenyl-containing group; and
(ii) at least one of L² and L³ represents a substituted or unsubstituted fluorenylene-containing group.

In the general formula (1), at least one of Ar¹ to Ar⁶ preferably represents a fluorenyl-containing group represented by the following general formula (1-a):

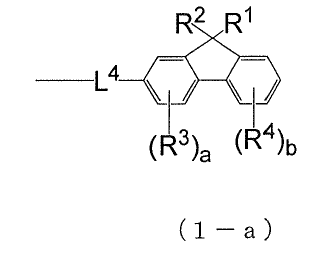

wherein R¹ and R² each independently represent a hydrogen atom or a substituent, and R¹ and R² may bond each other to form a cyclic structure;
R³ and R⁴ each independently represent a substituent; **a** represents an integer of 0 to 3, **b** represents an integer of 0 to 4, and further, when multiple R³'s are present, R³' s may bond each other to form a cyclic structure, or, when multiple R⁴'s are present, R⁴'s may bond each other to form a cyclic structure; and
L⁴ represents a single bond, a substituted or unsubstituted arylene group having 5 to 60 ring carbon atoms, or a substituted or unsubstituted heteroarylene group having 3 to 50 ring carbon atoms.
Examples of the substituent represented by any one of R¹ to R⁴; and the cyclic structure that may be formed include examples similar to those described for the substituent represented by any one of Ar¹ to Ar⁶ and L¹ to L³ in the general formula (1).
Examples of the arylene or heteroarylene group represented by L⁴ include examples similar to those described for L¹ to L³ in the general formula (1), and examples of a substituent for the arylene or heteroarylene group include examples similar to those described above.

The fluorenyl-containing group represented by the general formula (1-a) is preferably a fluorenyl-containing group represented by the following general formula (1-b):

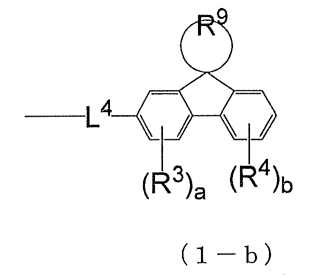

wherein R⁹ represents an atomic group forming a cyclic structure; R³, R⁴, **a, b,** and L⁴ each are the same as described above.
Examples of the atomic group represented by R⁹ of which a cyclic structure is formed include an alkylene group such as an ethylene group, a propylene group, an n-butylene group, an n-pentylene group, or an n-hexylene group; and a group obtained by substituting at least one carbon atom of any one of these alkylene groups by, for example, a nitrogen atom or an oxygen atom so that a heterocyclic ring is formed. The atomic group may have a substituent, and, furthermore, substituents may bond each other to form a saturated or unsaturated cyclic structure. Examples of the substituents and of the cyclic structure include examples similar to those described above.

In addition, examples of the fluorenyl-containing group represented by the general formula (1-b) are shown below.

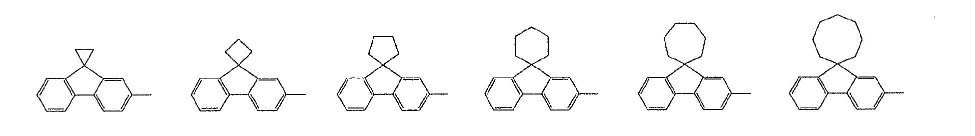

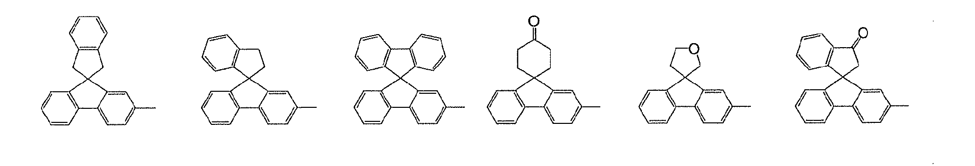

At least one of L² and L³ in the general formula (1) preferably represents a fluorenylene-containing group represented by the following general formula (2-a):

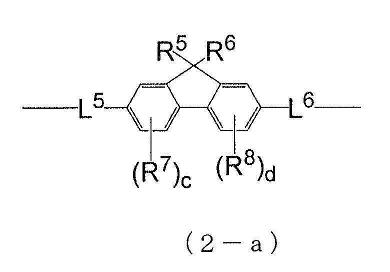

wherein R⁵ and R⁶ each independently represent a hydrogen atom or a substituent, and R⁵ and R⁶ may bond each other to form a cyclic structure;
R⁷ and R⁸ each independently represent a substituent; **c** and **d** each represent an integer of 0 to 3, and further, when multiple R⁷'s are present, R⁷'s may bond each other to form a cyclic structure, or, when multiple R⁸'s are present, R⁸'s may bond each other to form a cyclic structure; and
L⁵ and L⁶ each independently represent a single bond, a substituted or unsubstituted arylene group having 5 to 60 ring carbon atoms, or a substituted or unsubstituted heteroarylene group having 3 to 50 ring carbon atoms.
Examples of the substituent represented by any one of R⁵ and R⁶; and the cyclic structure which may be formed include examples similar to those described for the substituent represented by any one of Ar¹ to Ar⁶ and L¹ to L³ in the general formula (1).
Examples of an arylene or heteroarylene group represented by any one of L⁵ and L⁶ include examples similar to those described for L¹ to L³ in the general formula (1), and examples of a substituent for the arylene or heteroarylene group include examples similar to those described above.

The fluorenylene-containing group represented by the general formula (2-a) is preferably afluorenylene-containing group represented by the following general formula (2-b): 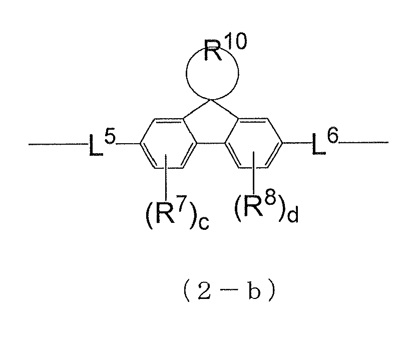 wherein R¹⁰ represents an atomic group forming a cyclic structure and further, R⁷, R⁸, **c, d,** L⁵, and L⁶ each are the same as described above.
Examples of the atomic group represented by R¹⁰ forming a cyclic structure include examples similar to those described for R⁹ in the above general formula (1-b).

In addition, examples of the fluorenylene-containing group represented by the general formula (2-b) are shown below.

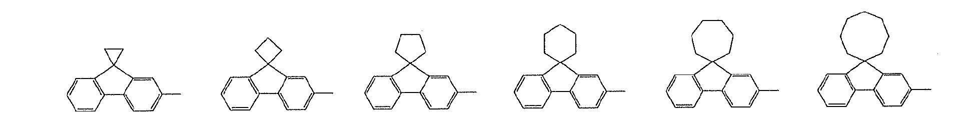

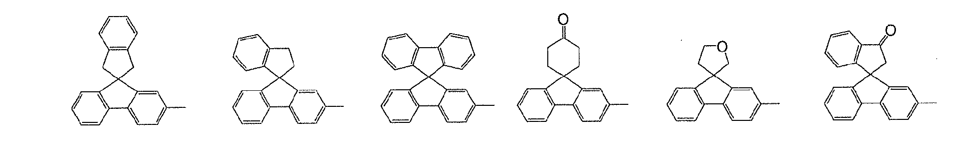

Specific examples of the aromatic amine compound represented by the general formula (1) of the present invention are shown below. However, the present invention is not limited to these exemplified compounds.

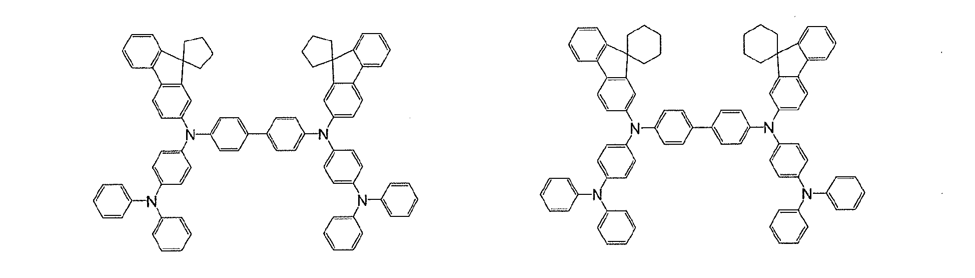

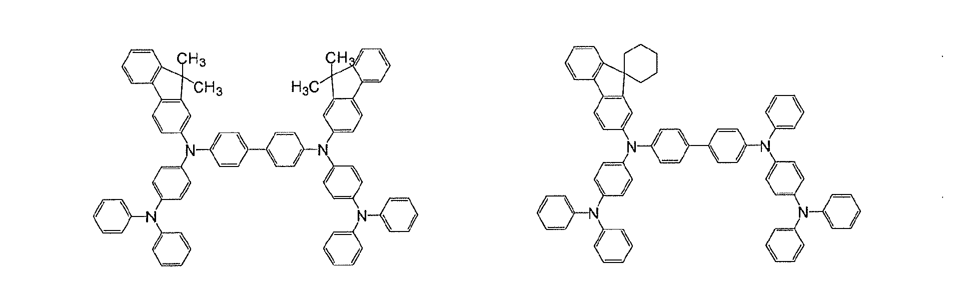

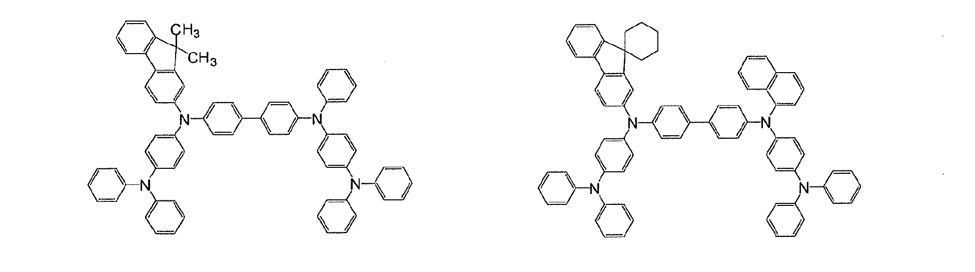

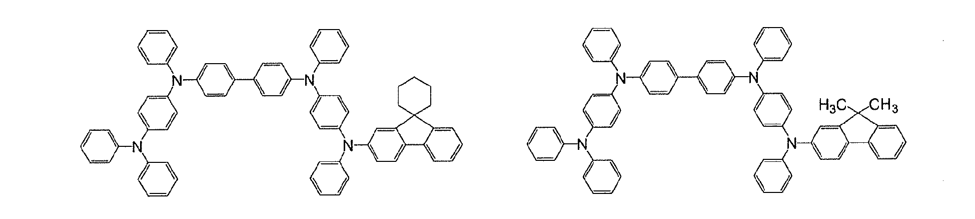

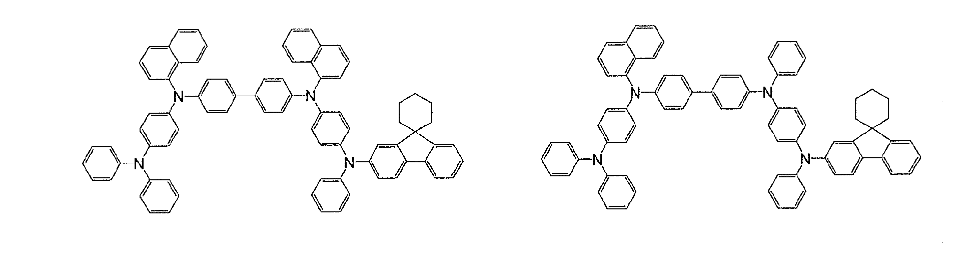

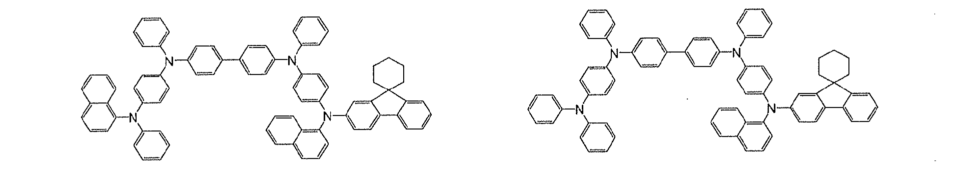

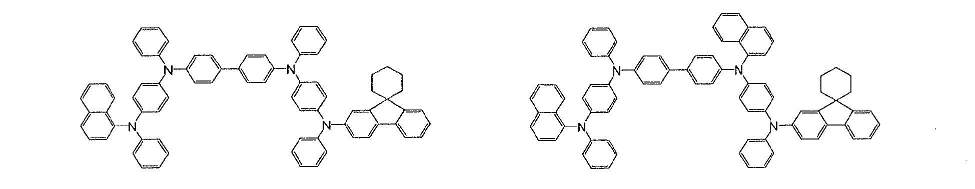

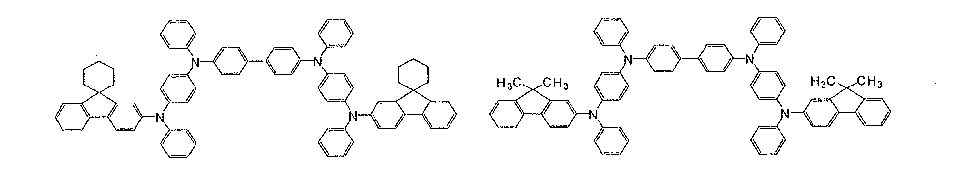

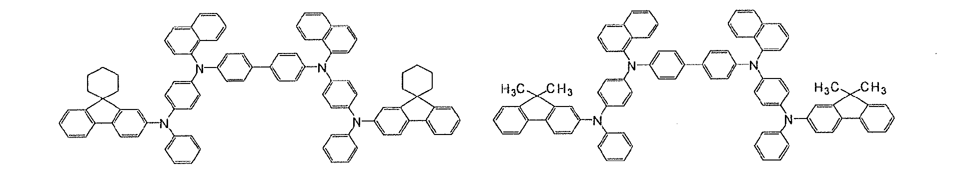

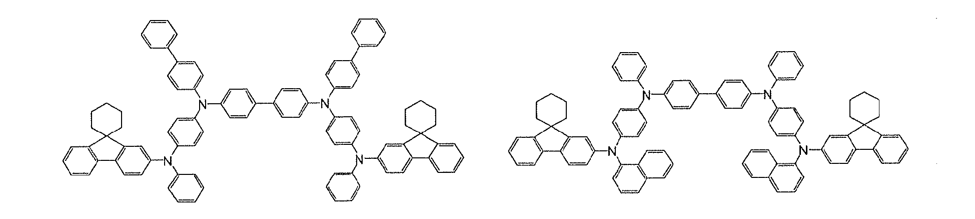

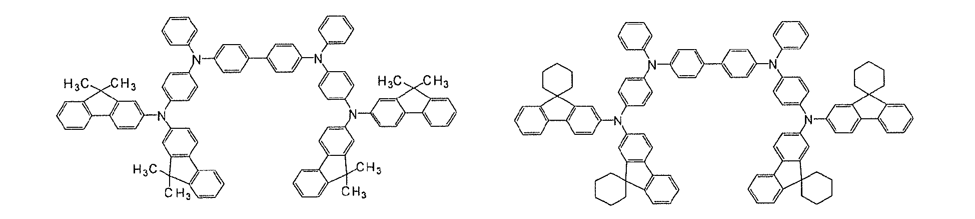

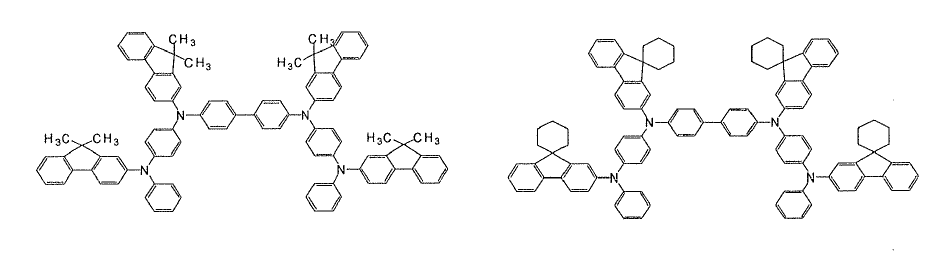

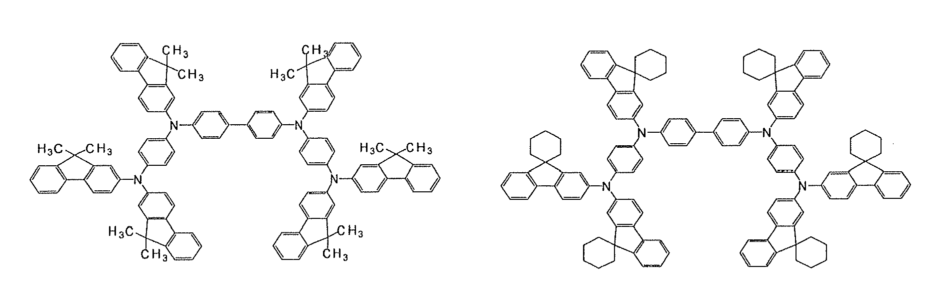

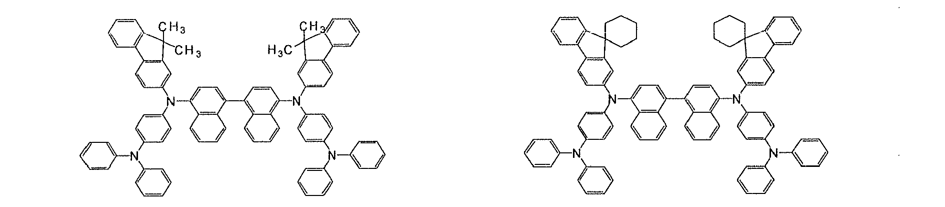

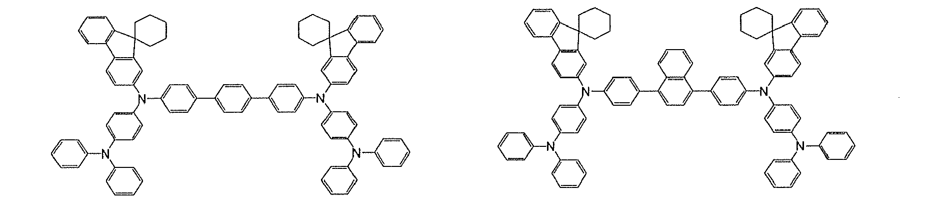

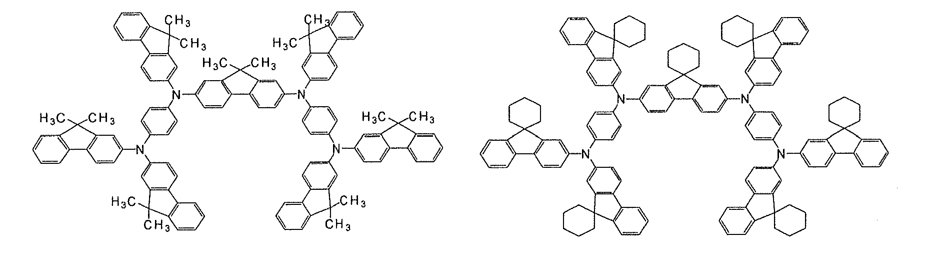

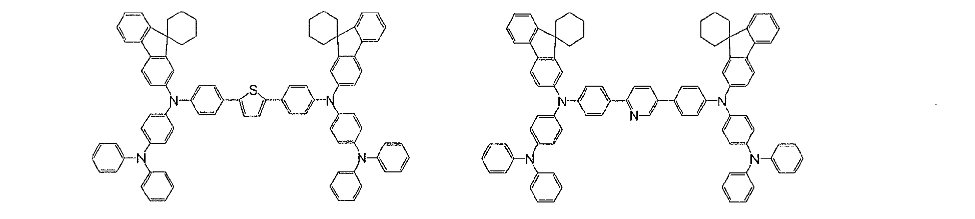

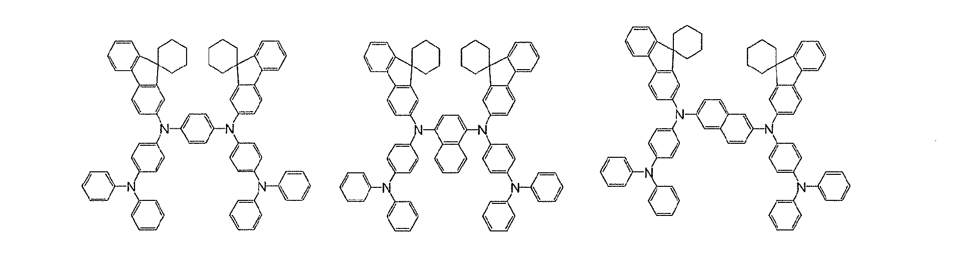

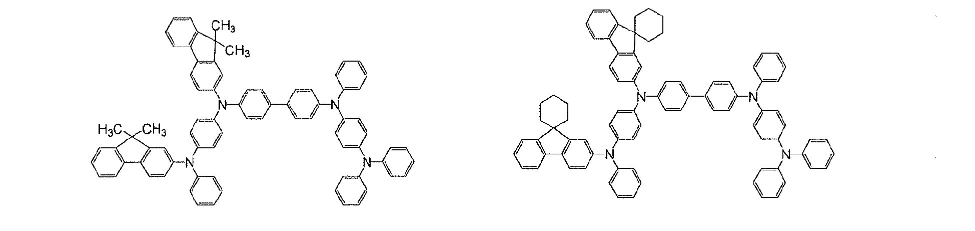

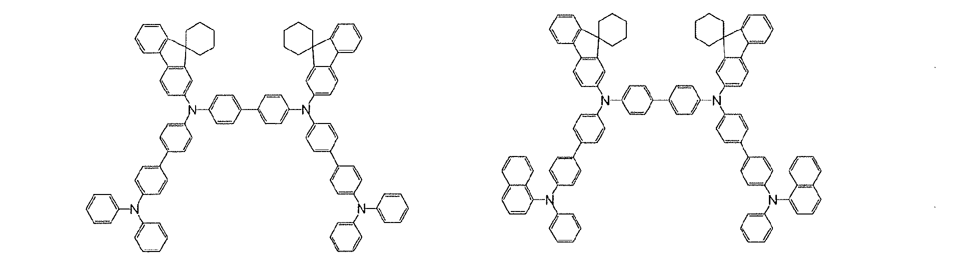

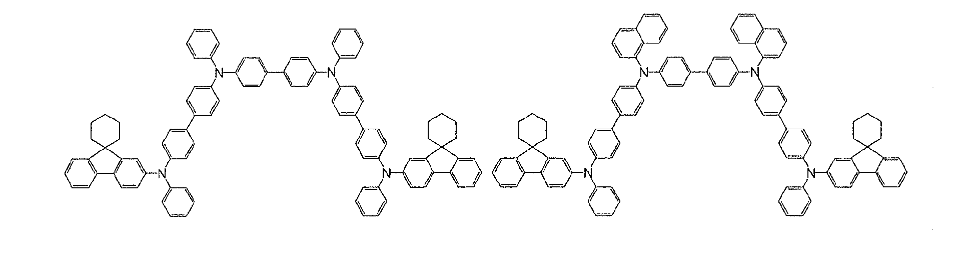

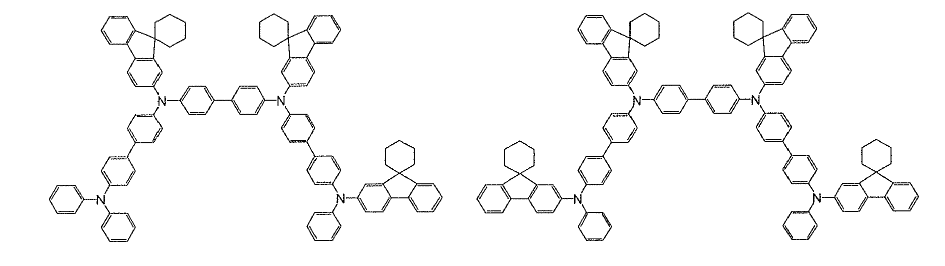

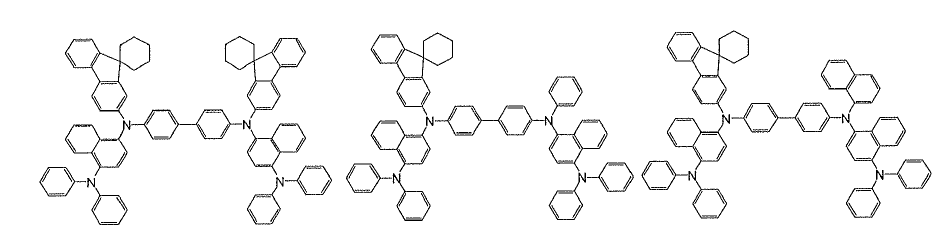

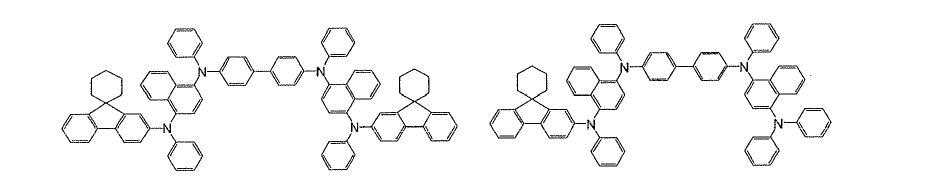

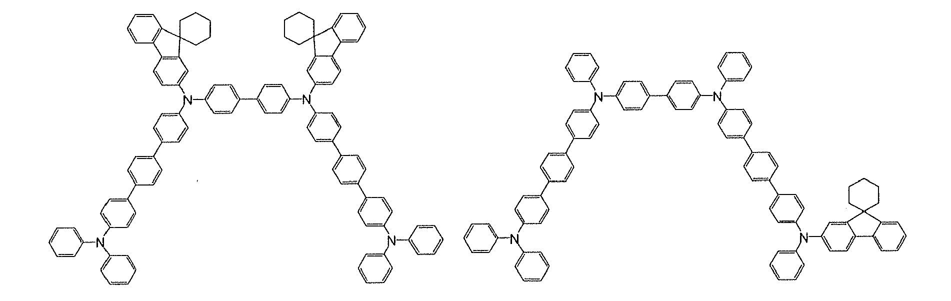

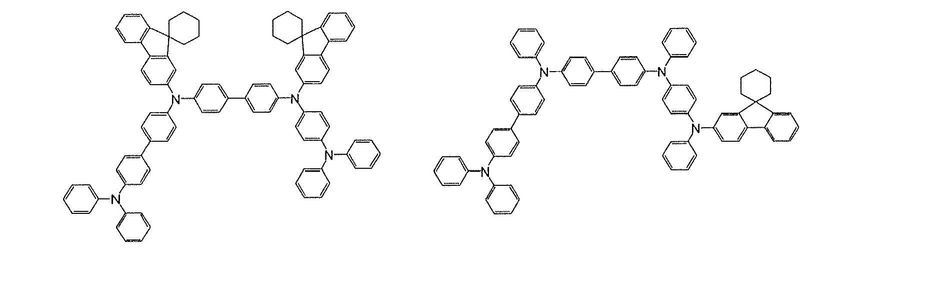

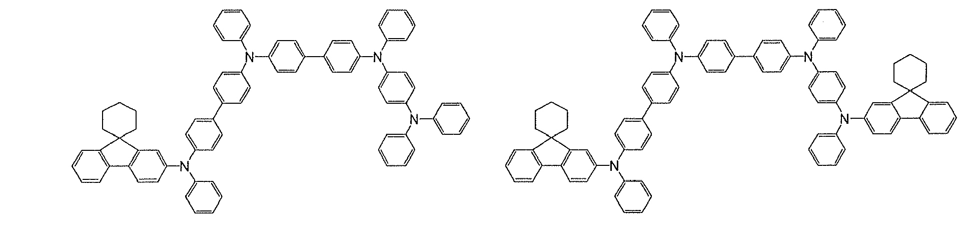

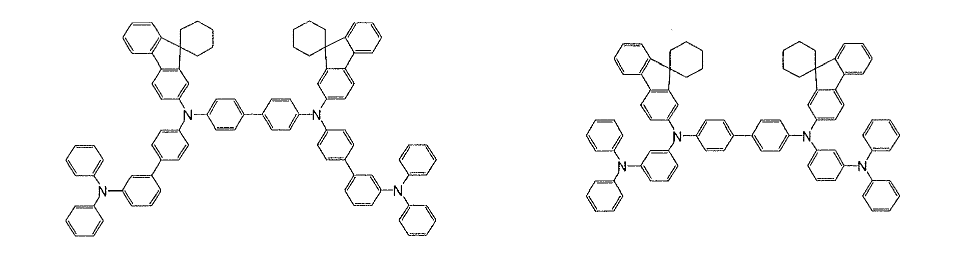

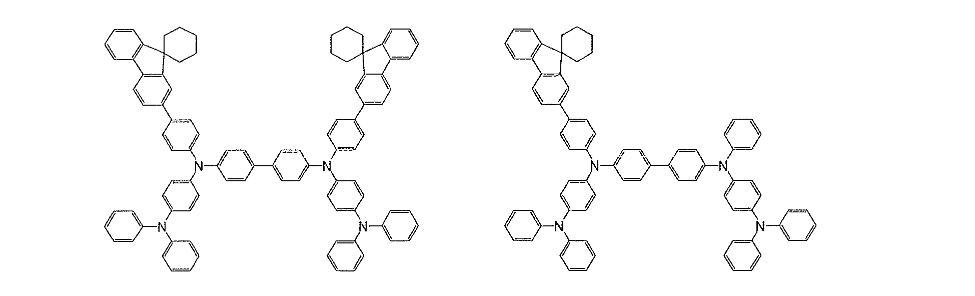

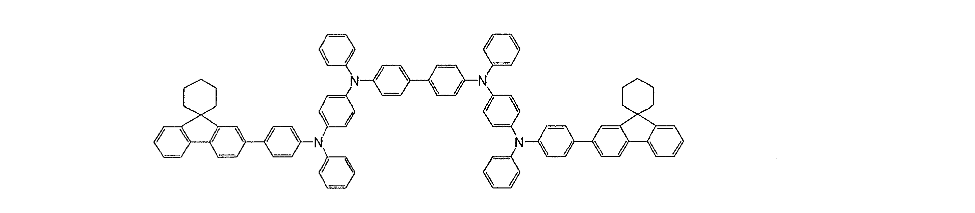

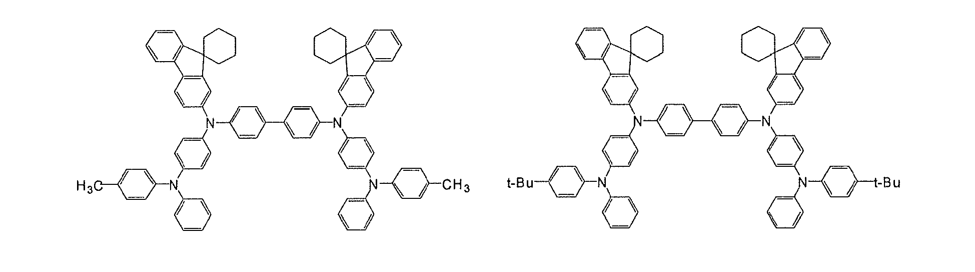

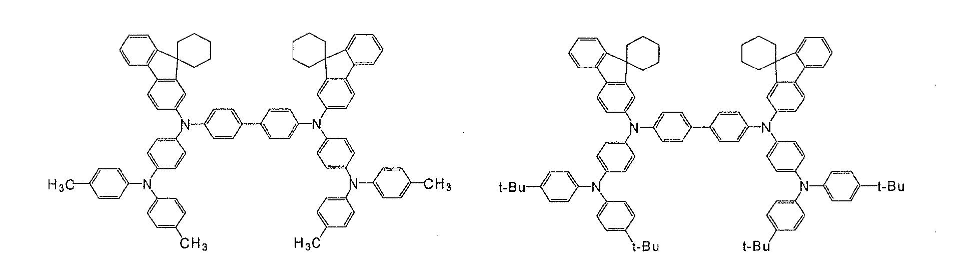

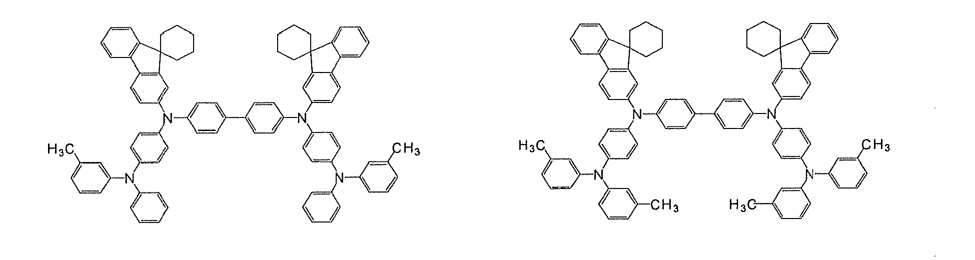

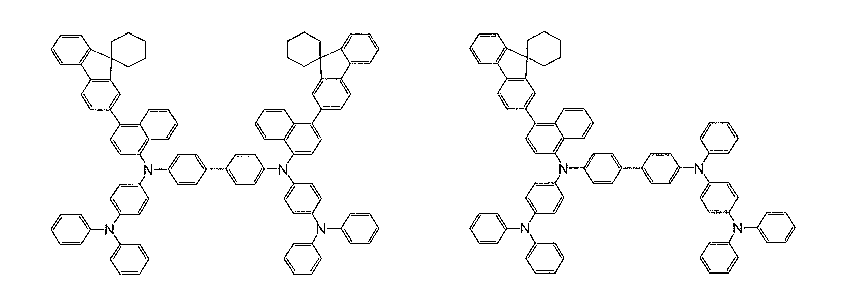

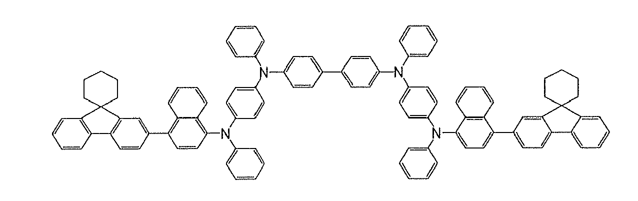

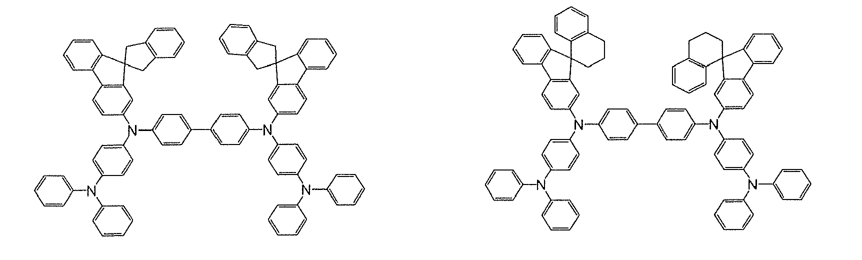

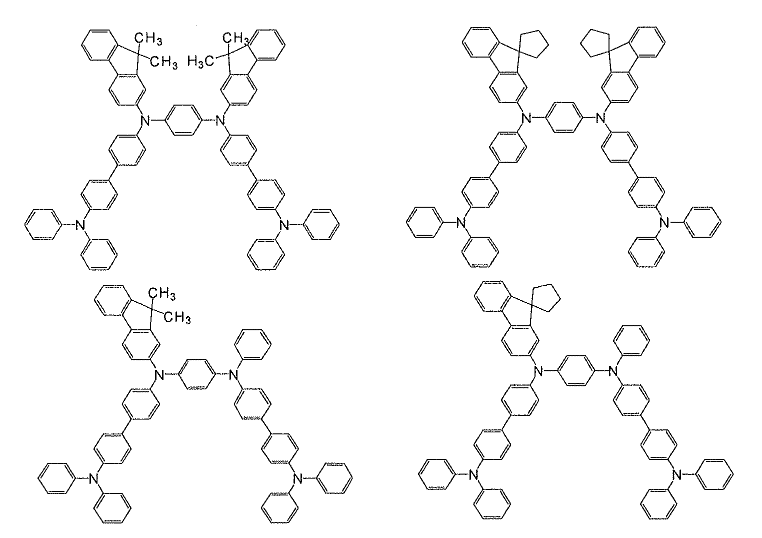

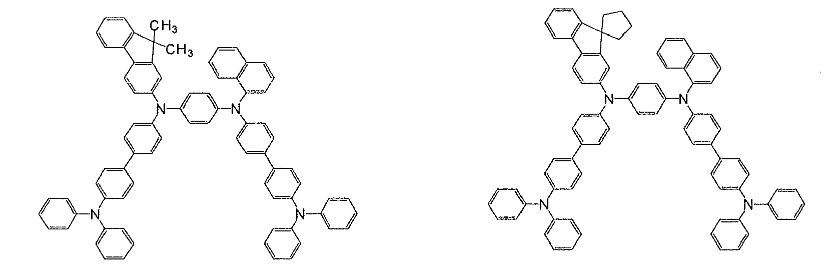

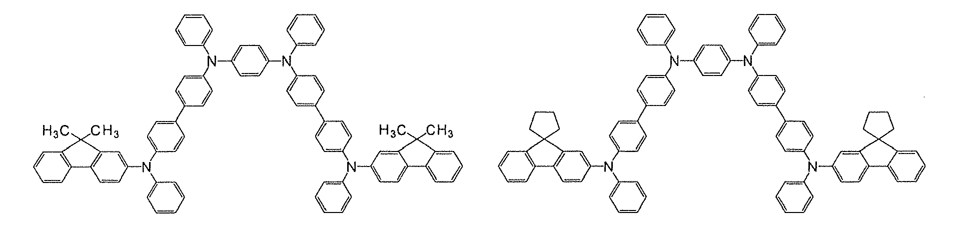

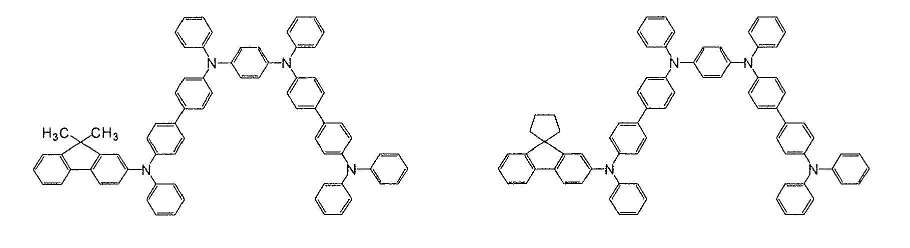

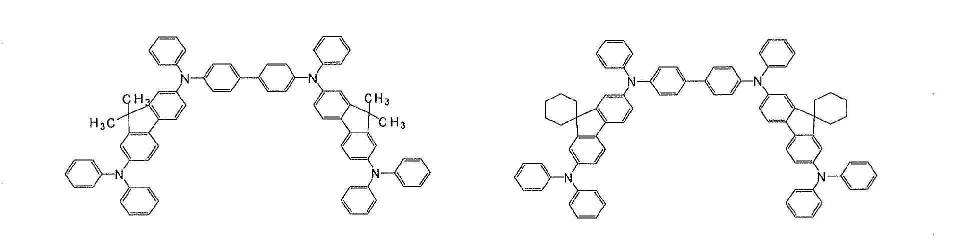

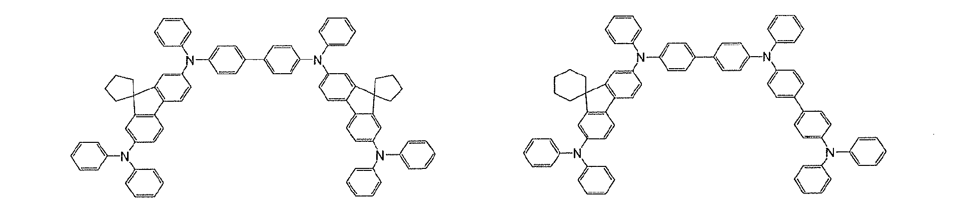

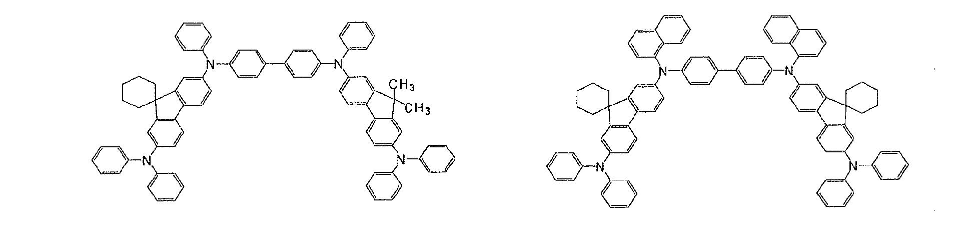

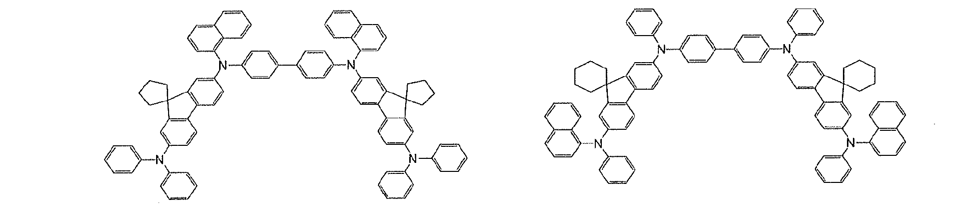

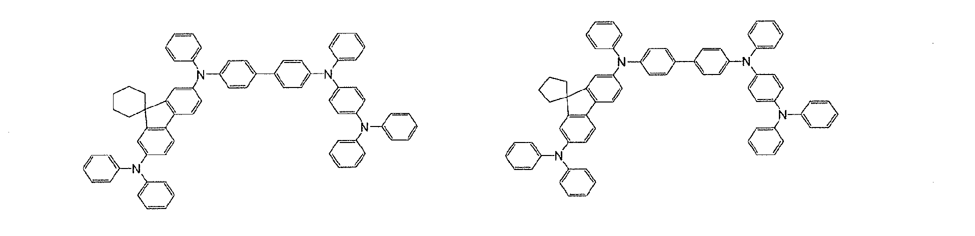

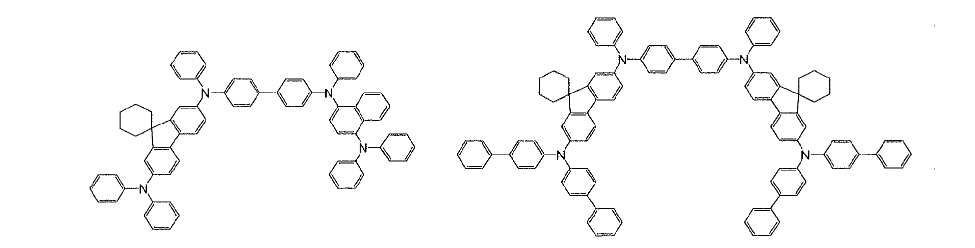

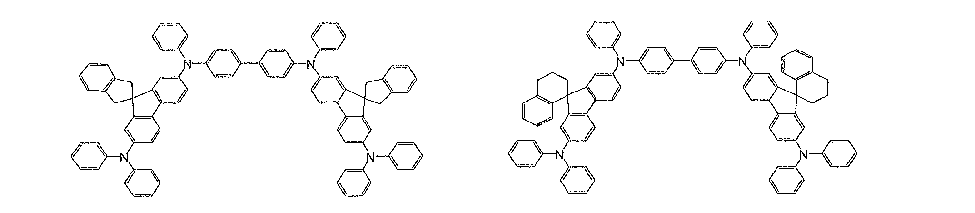

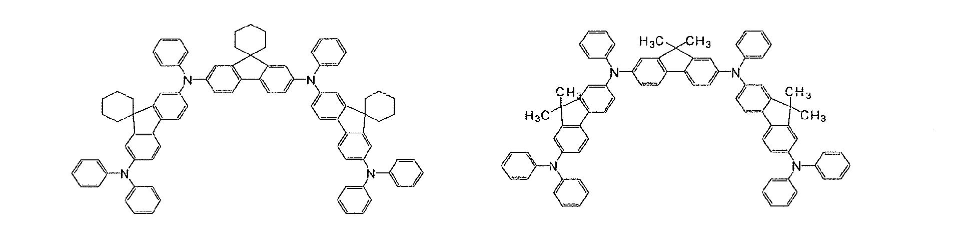

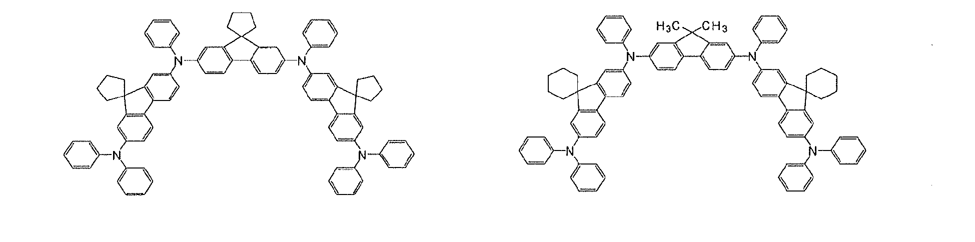

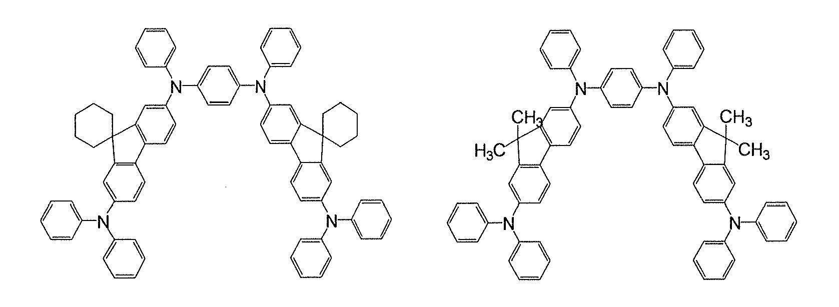

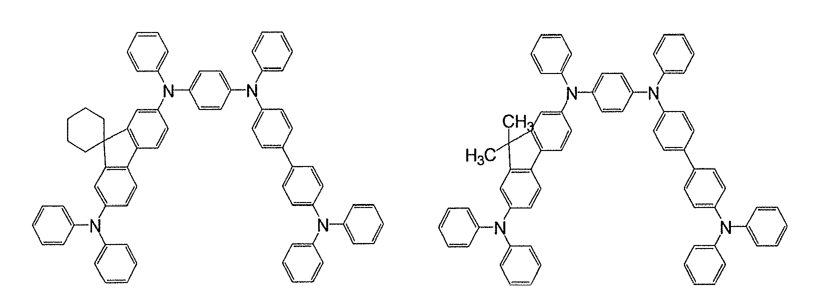

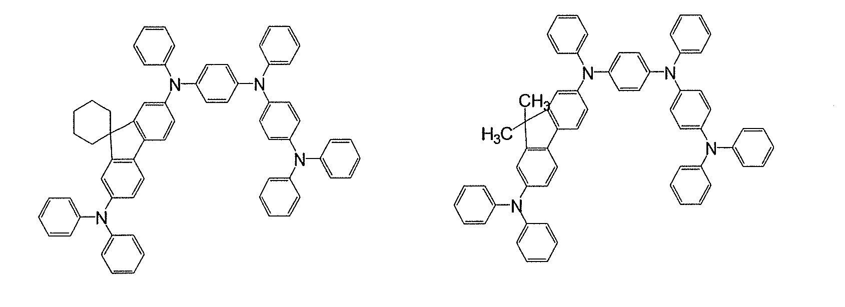

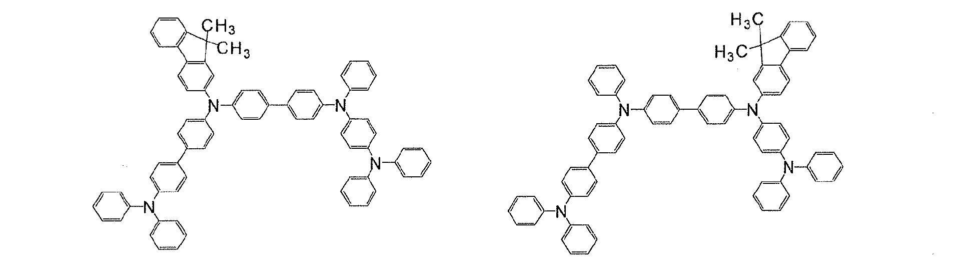

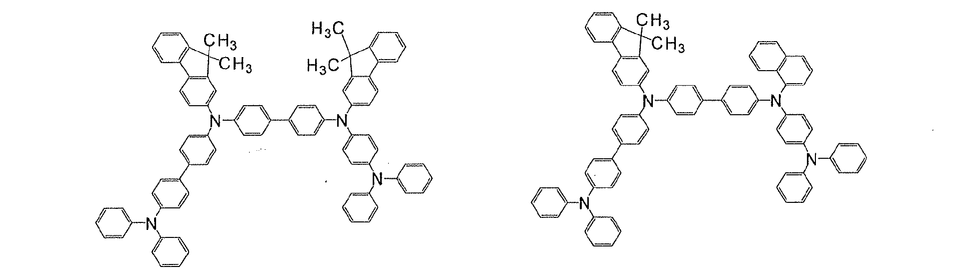

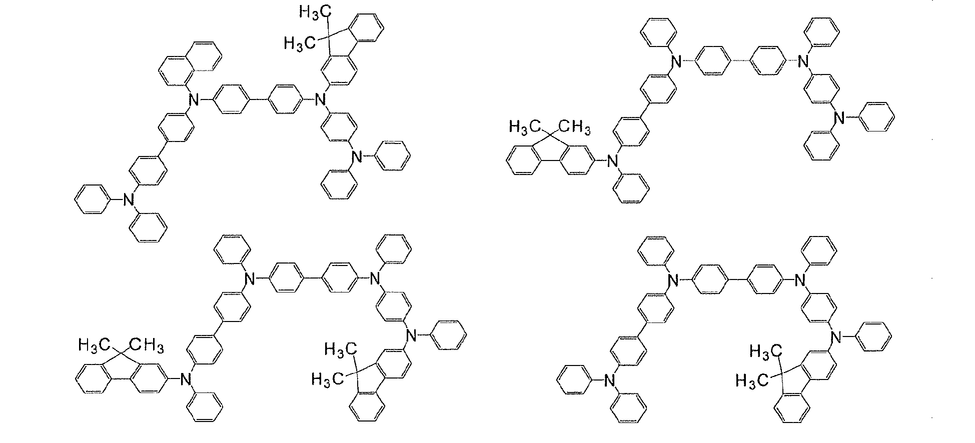

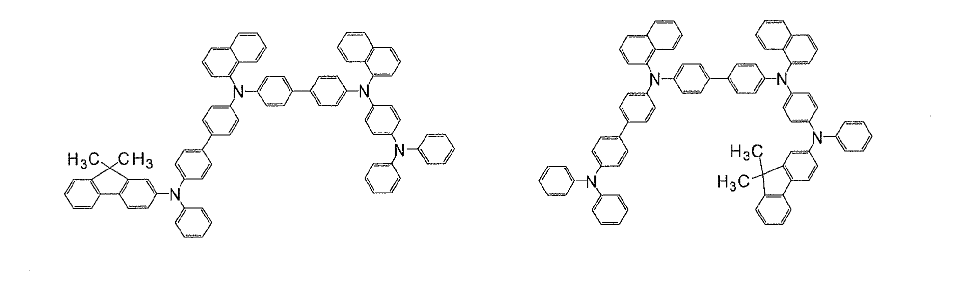

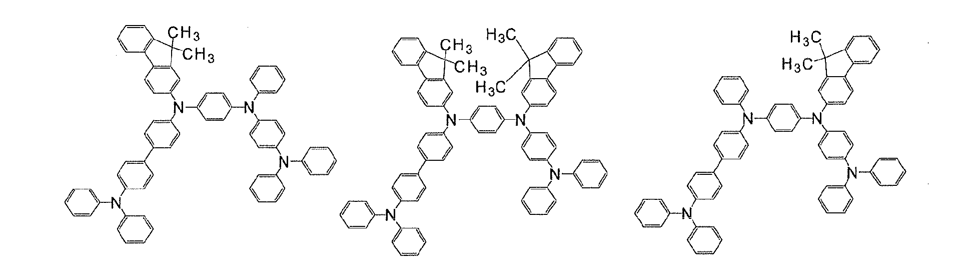

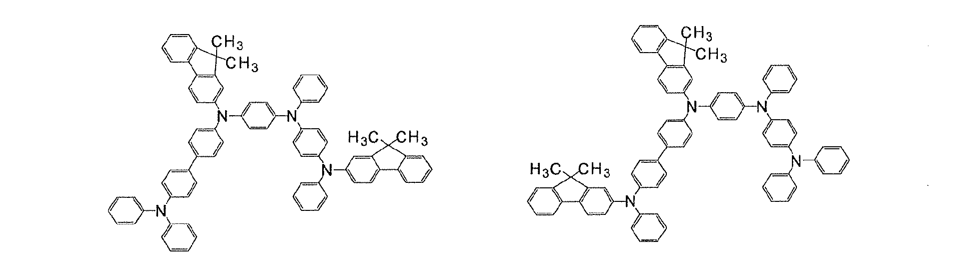

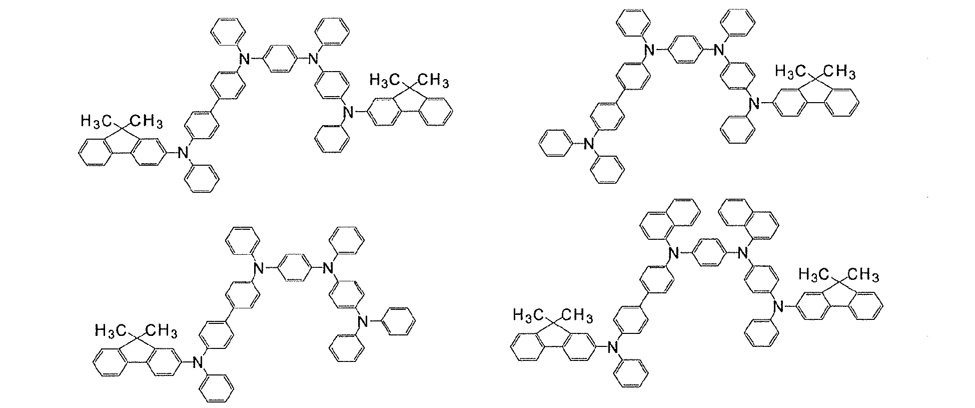

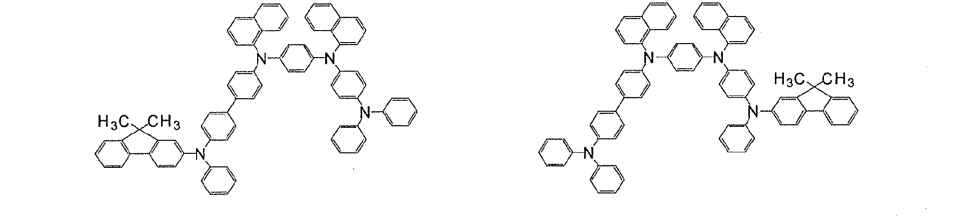

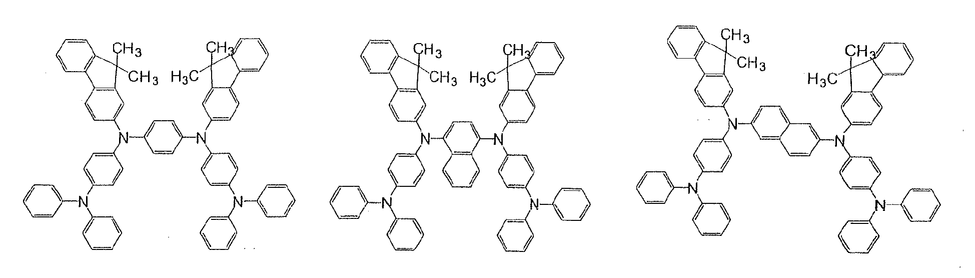

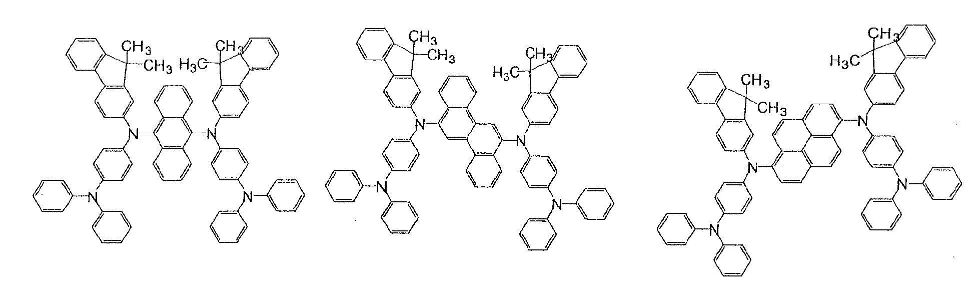

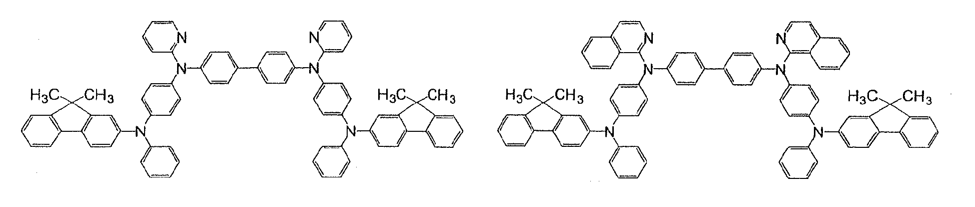

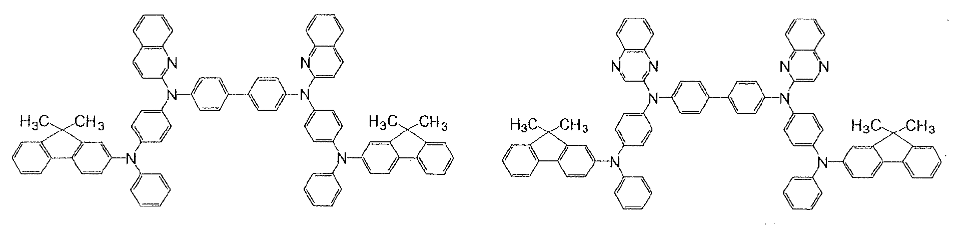

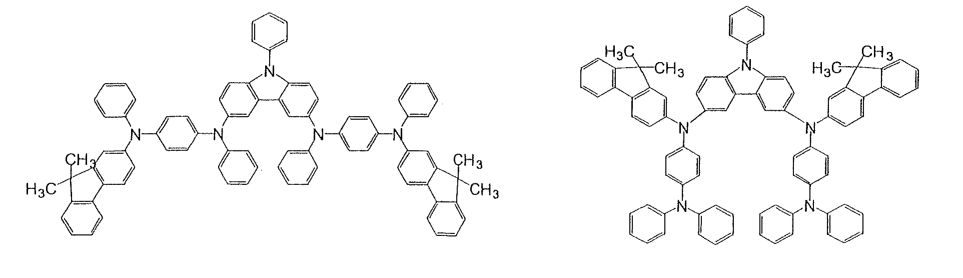

The aromatic amine compound of the present invention is preferably a material for an organic EL device, and is particularly suitable as a hole transporting material for an organic EL device, a hole injecting material for an organic EL device, or a light emitting material for an organic EL device.
In addition, the aromatic amine compound of the present invention, which can be used as a hole injecting material or a hole transporting material, is preferably used as a hole injecting material when the compound has a phenylenediamine skeleton, or as a hole transporting material when the compound has a diphenylenediamine skeleton.
Next, an organic EL device of the present invention will be described.
The present invention provides an organic EL device comprising one or more organic thin film layers which comprise at least a light emitting layer sandwiched between a cathode and an anode, wherein at least one layer among the organic thin film layers comprises the aromatic amine compound singly or as its mixture component.

Constructions of the organic EL device of the present invention will be described in the following.

### (1) Organic EL device construction

Typical examples of the construction of the organic EL device of the present invention include the following:
(1) an anode/light emitting layer/cathode;
(2) an anode/hole injecting layer/light emitting layer/cathode;
(3) an anode/light emitting layer/electron injecting layer/cathode;
(4) an anode/hole injecting layer/light emitting layer/electron injecting layer/cathode;
(5) an anode/organic semiconductor layer/light emitting layer/cathode;
(6) an anode/organic semiconductor layer/electron barrier layer/light emitting layer/cathode;
(7) an anode/organic semiconductor layer/light emitting layer/adhesion improving layer/cathode;
(8) an anode/hole injecting layer/hole transporting layer/light emitting layer/electron injecting layer/cathode;
(9) an anode/electrically insulating layer/light emitting layer/electrically insulating layer/cathode;
(10) an anode/inorganic semiconductor layer/electrically insulating layer/light emitting layer/electrically insulating layer/cathode;
(11) an anode/organic semiconductor layer/electrically insulating layer/light emitting layer/electrically insulating layer/cathode;
(12) an anode/electrically insulating layer/hole injecting layer/hole transporting layer/light emitting layer/ electrically insulating layer/cathode; and
(13) an anode/electrically insulating layer/hole injecting layer/hole transporting layer/light emitting layer/electron injecting layer/cathode.
   Of those, the construction (8) is preferably used in ordinary cases. However, the construction is not limited to the foregoing.

The aromatic amine compound of the present invention, which may be used in any part of the organic thin film layer of the organic EL device, is preferably incorporated into at least one of a hole transporting region and a hole injecting region, or more preferably incorporated into at least one of a hole transporting layer and a hole injecting layer. The content of the compound is typically selected from the range of 30 to 100 mol%. It is particularly preferable that at least one of the hole transporting layer and the hole injecting layer contain the aromatic amine compound as its main component.
In the organic EL device of the present invention, the layer containing the aromatic amine compound is preferably in contact with the anode, and the main component of the layer in contact with the anode is more preferably the aromatic amine compound.
In the organic EL device of the present invention, the organic thin film layer preferably has a layer containing the aromatic amine compound and a light emitting material. It is also preferable that the organic thin film layer have a laminate formed of a hole transporting layer containing the aromatic amine compound and/or a hole injecting layer containing the aromatic amine compound, and of a light emitting layer composed of a phosphorescent metal complex and a host material. The metal complex and/or the host material will be described below in the section titled "Light emitting layer".

### (2) A substrate which transmits light

The organic EL device of the present invention is prepared on a substrate which transmits light. The substrate which transmits light in this case is the substrate which supports the organic EL device. It is preferable that the substrate which transmits light have a transmittance of light of 50% or greater in the visible region of 400 to 700 nm and be a smooth substrate.
Examples of the substrate which transmits light include glass plates and polymer plates. Specific examples of the glass plate include plates made of soda-lime glass, glass containing barium and strontium, lead glass, aluminosilicate glass, borosilicate glass, barium borosilicate glass, and quartz. Specific examples of the polymer plate include plates made of polycarbonate, acrylic, polyethylene terephthalate, polyether sulfide, and polysulfone.

### (3) Anode

The anode in the organic EL device of the present invention has the function of injecting holes into the hole transporting layer or the light emitting layer. It is effective that the anode has a work function of 4.5 eV or greater. Examples of the material for the anode used in the present invention include indium tin oxide alloys (ITO), indium zinc oxide alloys (IZO), tin oxide (NESA), gold, silver, platinum, and copper.
The anode can be prepared by forming a thin film of the electrode materials by a process such as the vapor deposition process and the sputtering process.
When the light emitted from the light emitting layer is obtained through the anode, it is preferable that the anode have a transmittance of the emitted light greater than 10%. It is also preferable that the sheet resistance of the anode be several hundred Ω/□ or smaller. The thickness of the anode is, in general, selected in the range of 10 nm to 1 µm and preferably in the range of 10 to 200 nm although the preferable range may be different depending on the material to be used.

### (4) Light emitting layer

The light emitting layer in the organic EL device has a combination of the following functions (1) to (3):
(1) The injecting function: the function of injecting holes from the anode or the hole injecting layer and injecting electrons from the cathode or the electron-injecting layer when an electric field is applied;
(2) The transporting function: the function of transporting injected charges (electrons and holes) by the force of the electric field; and
(3) The light emitting function: the function of providing the field for recombination of electrons and holes and leading the emission of light.
   However, the easiness of injection may be different between holes and electrons and the ability of transportation expressed by the mobility may be different between holes and electrons. It is preferable that either one of the charges be transferred.
   When the compound of the present invention is used in the light emitting layer, the light emitting layer may be formed of the compound of the present invention alone, or the compound may be mixed with any other material before use.

A material to be mixed with the compound of the present invention to form the light emitting layer is not particularly limited as long as the material has the above preferable properties, and an arbitrary material can be selected from the known materials to be used in the light emitting layer of an organic EL device.
Examples of a light emitting material or a doping material which can be used in the light emitting layer together with the compound of the present invention include, but are not limited to, anthracene, naphthalene, phenanthrene, pyrene, tetracene, coronene, chrysene, fluoresceine, perylene, phthaloperylene, naphthaloperylene, perynone, phthaloperynone, naphthaloperynone, diphenylbutadiene, tetraphenylbutadiene, coumarin, oxadiazole, aldazine, bisbenzoxazoline, bisstyryl, pyrazine, cyclopentadiene, quinoline metal complexes, aminoquinoline metal complexes, benzoquinoline metal complexes, imine, diphenylethylene, vinylanthracene, diaminocarbazole, pyrane, thiopyrane, polymethine, merocyanine, imidazole-chelated oxynoid compounds, quinacridone, rubrene, and fluorescent dyes.
It is preferable that the compound of the present invention be mainly used at that time. The content of the compound in the light emitting layer is typically 30 to 100 mol%, or more preferably 50 to 99 mol%.
The light emitting material to be used in combination with the compound of the present invention is mainly an organic compound, and specific examples of the material include the following organic compounds depending on a desired color tone.

In order that light having a color ranging from a color in an ultraviolet region to a violet color may be emitted, a first example is a compound represented by the following general formula. 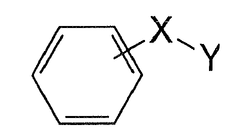 In the general formula, **X** represents the following group: 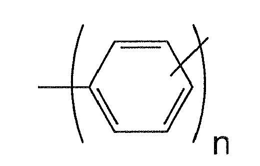 wherein **n** represents an integer of 2 to 5, and **Y** represents 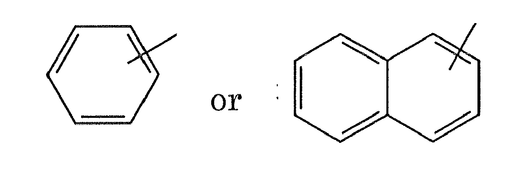 The phenyl group, phenylene group, or naphthyl group in the compound may be substituted by an alkyl or alkoxy group having 1 to 4 carbon atoms, a hydroxyl group, a sulfonyl group, a carbonyl group, an amino group, a dimethylamino group, a diphenylamino group, or the like; each group may be substituted by one or multiple substituents. In addition, those substituents may bond each other to form asaturated,five-membered orsix-membered ring. In addition, such substituent is preferably bonded at the para position of each of the phenyl, phenylene, and naphthyl groups for the formation of a smooth evaporated film because good bonding property can be obtained. Specific examples of the compound include the following compounds. Of those, a p-quarter-phenyl derivative or a p-quinquephenyl derivative is particularly preferable.

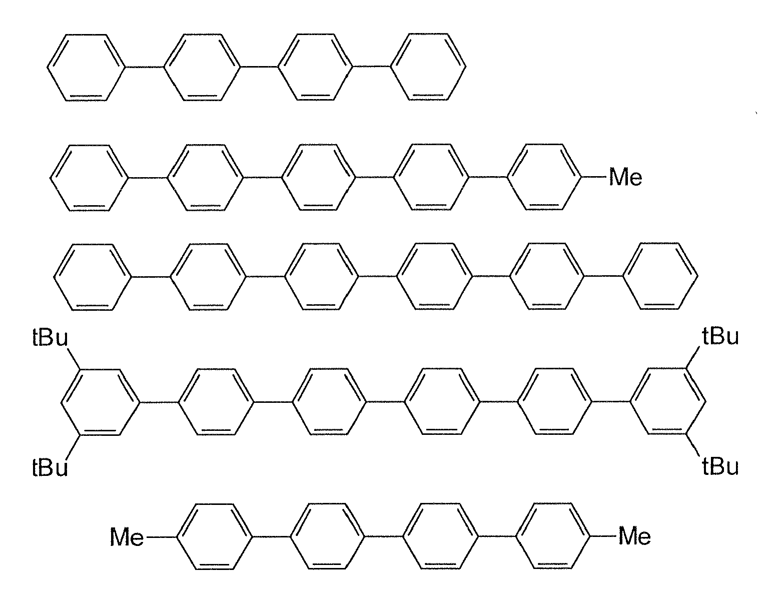

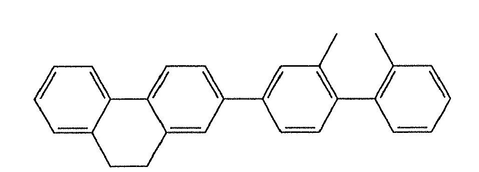

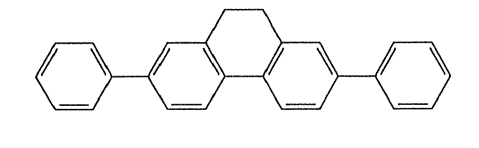

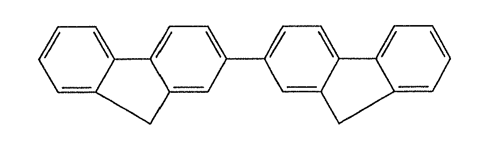

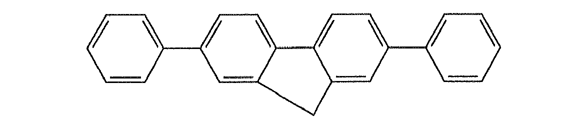

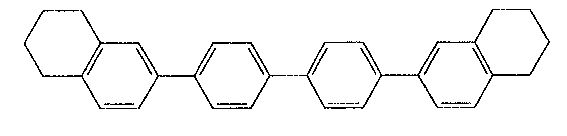

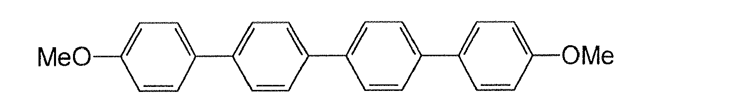

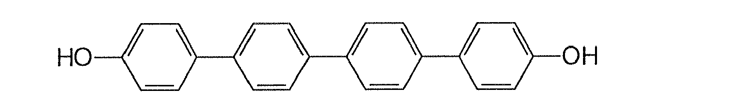

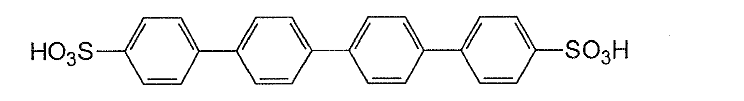

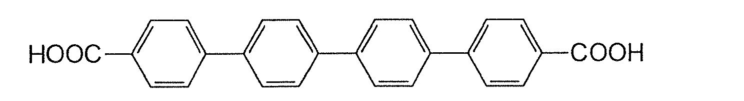

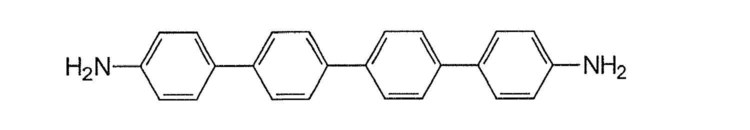

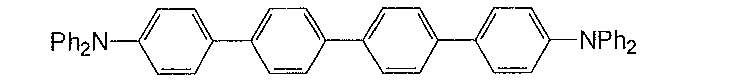

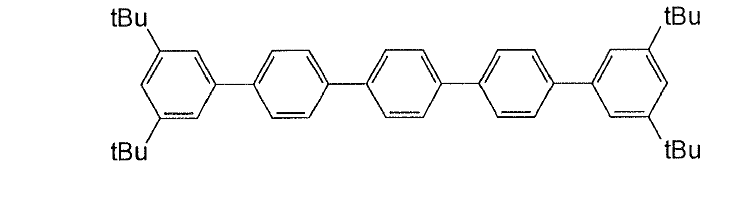

In addition, in order that light having a color ranging from a blue color to a green color may be emitted, for example, a benzothiazole-based, benzimidazole-based, or benzoxazole-based fluorescent bleach, a metal chelated oxynoid compound, a styrylbenzene-based compound, or a fused aromatic ring-based compound can be exemplified.
Specific examples of the compound names for the foregoing include those disclosed in JP 59-194393 A and Chemistry of Synthetic Dyes, 1971, p. 628 to 637 and 640.
Examples of the metal chelated oxynoid compound include those disclosed in JP 63-295695A. Representative examples of the compound include an 8-hydroxyquinoline-based metal complex such as tris (8-quinolinol) aluminum (hereinafter abbreviated as "Alq") and dilithium epintridione.
Examples of the styrylbenzene-based compound include those disclosed in EPC Publication Nos. EP 0319881 A and EP 0373582 A. A distyrylpyrazine derivative disclosed in JP 02-252793 A can also be used.
Examples of the fused aromatic ring-based compound include those disclosed in JP 2004-59535 A, JP 2004-75567 A, JP 2004-83481 A, and JP 2004-107326 A.

In addition, for example, a polyphenyl-based compound disclosed in EPC Publication No. EP 0387715 A can also be used as a material for the light emitting layer.
Further, in addition to the fluorescent bleach, the metal chelated oxynoid compound, and the styrylbenzene-based compound described above, for example, 12-phthaloperinone (J. Appl. Phys., vol. 27, L 713 (1988)), 1,4-diphenyl-1,3-butadine or 1,1,4,4-tetraphenyl-1,3-butadine (Appl. Phys. Lett., vol. 56, L 799 (1990)), a naphthalimide derivative (JP 02-305886 A), a perylene derivative (JP 02-189890 A), an oxadiazole derivative (JP 02-216791 A, or an oxadiazole derivative disclosed by Hamada et al. in the thirty-eighth joint conference on applied physics), an aldazine derivative (JP 02-220393 A), a pyraziline derivative (JP 02-220394 A), a cyclopentadiene derivative (JP 02-289675 A), a pyrrolopyrrol derivative (JP 02-296891 A), a styrylamine derivative (Appl. Phys. Lett. , vol. 56, L799 (1990), a coumarin-based compound (JP 02-191694), or a polymer compound described in PCT International Publication No. WO 90/13148 A or in Appl. Phys. Lett., vol. 58, 18, P 1982 (1991) can also be used as a material for the light emitting layer.

Of those, in the present invention, an aromatic dimethylidene-based compound (disclosed in EPC Publication No. EP 0388768 A or in JP 03-231970 A), or a fused aromatic ring compound is particularly preferably used. Specific examples of such compounds include 4,4'-bis(2,2-di-t-butylphenylvinyl)biphenyl (hereinafter abbreviated as "DTBPBBi"), 4,4'-bis(2,2-diphenylvinyl)biphenyl (hereinafter abbreviated as "DPVBi"), and derivatives of them.
Further, a compound described in, for example, JP 05-258862 A and represented by a general formula (Rs-**Q**)₂-Al-O-**L** (wherein **L** represents a hydrocarbon containing a phenyl portion and having 6 to 24 carbon atoms, O-L represents a phenolate ligand, **Q** represents a substituted 8-quinolinolato ligand, and Rs represents an 8-quinolinolato ring substituent selected so as to prevent sterically more than two substituted 8-quinolinolato ligands from bonding to an aluminum atom) can also be exemplified. Specific examples of the compound include bis(2-methyl-8-quinolinolato)(para-phenylphenolate)aluminum(II I) and bis(2-methyl-8-quinolinolato)(1-naphtholate)aluminum(III).
In addition, a method of obtaining the emission of the mixture of blue light and green light with high efficiency by employing doping according to, for example, JP 06-9953 A can be exemplified. In this case, an example of a host material is any one of the above-mentioned light emitting materials, and an example of a dopant is a fluorescent dye capable of strongly emitting light having a color ranging from a blue color to a green color such as a coumarin-based fluorescent dye or a fluorescent dye similar to that used as the above-mentioned host material. In this case, a preferable example of the host material is a light emitting material having a distyrylarylene skeleton, and a particularly preferable example of the host material is DPVBi; a preferable example of the dopant is diphenylaminovinylarylene, and a particularly preferable example of the dopant is N,N-diphenylaminovinylbenzene (DPAVB).

Further, examples of a the light emitting layer capable of emitting white light include, but not particularly limited to, the following:
(i) a layer in which the energy level of each layer of an organic EL laminate structure is specified, and light is emitted by utilizing tunnel injection (EPC Publication No. EP 0390551 A);
(ii) a layer utilizing tunnel injection as in the case of the above item (i) and, as an example, a white light emitting device (JP 03-230584 A);
(iii) a light emitting layer having a two-layered structure (each of JP 02-220390 A and JP 02-216790 A);
(iv) a construction having a light emitting layer separated into multiple parts constituted of materials different from each other in luminous wavelength (JP 04-51491 A);
(v) a construction in which a blue light emitter (having a fluorescence peak at 380 to 480 nm) and a green light emitter (having a fluorescence peak at 480 to 580 nm) are laminated, and, furthermore, a red phosphor is incorporated (JP 06-207170 A); and
(vi) a construction in which a blue light emitting layer contains a blue fluorescent dye, and a green light emitting layer has a region containing a red fluorescent dye and contains a green phosphor (JP 07-142169 A).
   Of those, the construction described in the above item (v) is preferably used.
   Examples of the red phosphor are shown below:

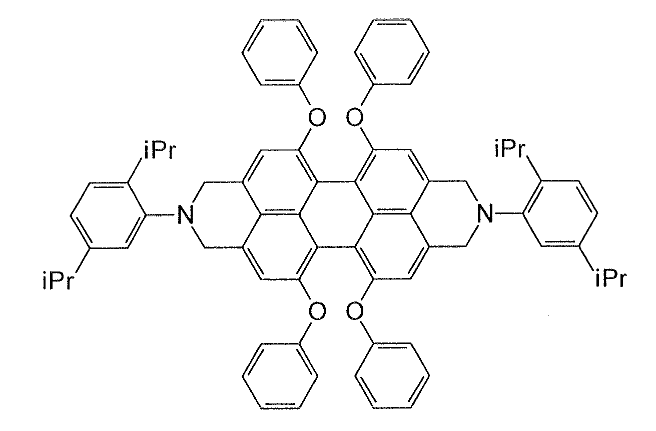

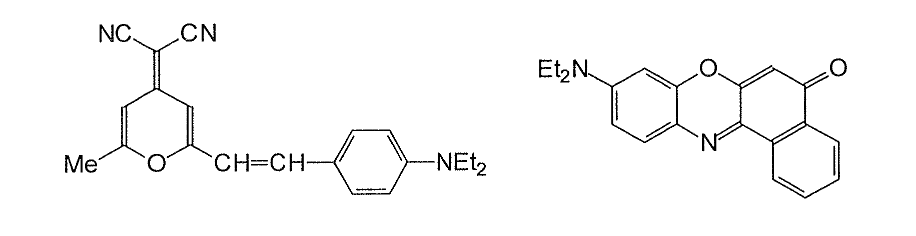

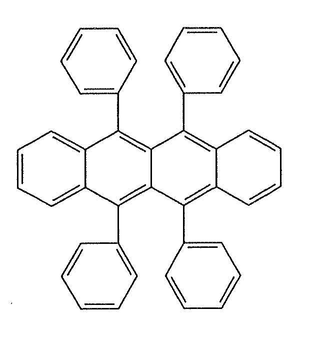

wherein iPr represents an isopropyl group and Et represents an ethyl group.

A host material that can be used in a light emitting layer together with the aromatic amine compound of the present invention is preferably a compound represented by any one of the following general formulae (i) to (ix).
An asymmetric anthracene represented by the following general formula (i): 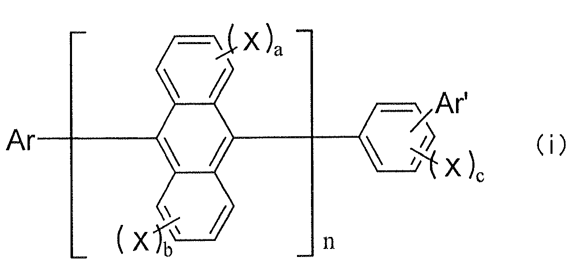 wherein **Ar** represents a substituted or unsubstituted fused aromatic group having 10 to 50 ring carbon atoms; **Ar'** represents a substituted or unsubstituted aromatic group having 6 to 50 ring carbon atoms; **X** represents a substituted or unsubstituted aromatic group having 6 to 50 ring carbon atoms, a substituted or unsubstituted aromatic heterocyclic group having 5 to 50 ring atoms, a substituted or unsubstituted alkyl group having 1 to 50 carbon atoms, a substituted or unsubstituted alkoxy group having 1 to 50 carbon atoms, a substituted or unsubstituted aralkyl group having 6 to 50 carbon atoms, a substituted or unsubstituted aryloxy group having 5 to 50 ring atoms, a substituted or unsubstituted arylthio group having 5 to 50 ring atoms, a substituted or unsubstituted alkoxycarbonyl group having 1 to 50 carbon atoms, a carboxyl group, a halogen atom, a cyano group, a nitro group, or a hydroxyl group. **a, b,** and **c** each represent an integer of 0 to 4.
**n** represents an integer of 1 to 3. In addition, when n represents 2 or more, components provided in [ ] maybe identical to or different from each other.

An asymmetric monoanthracene derivative represented by the following formula (ii): 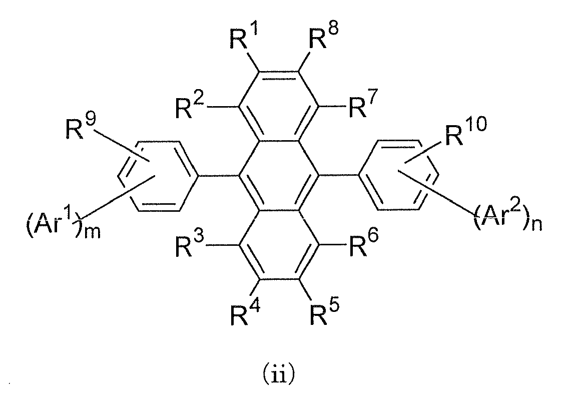 wherein Ar¹ and Ar² each independently represent a substituted or unsubstituted aromatic ring group having 6 to 50 ring carbon atoms. **m** and **n** each represent an integer of 1 to 4; provided that Ar¹ and Ar² are not identical to each other when **m** = **n** = 1 and positions at which Ar¹ and Ar² are bound to a benzene ring are bilaterally symmetric, and **m** and **n** represent different integers when **m** or **n** represents an integer of 2 to 4.
R¹ to R¹⁰ each independently represent a hydrogen atom, a substituted or unsubstituted aromatic ring group having 6 to 50 ring carbon atoms, a substituted or unsubstituted aromatic heterocyclic group having 5 to 50 ring atoms, a substituted or unsubstituted alkyl group having 1 to 50 carbon atoms, a substituted or unsubstituted cycloalkyl group, a substituted or unsubstituted alkoxy group having 1 to 50 carbon atoms, a substituted or unsubstituted aralkyl group having 6 to 50 carbon atoms, a substituted or unsubstituted aryloxy group having 5 to 50 ring atoms, a substituted or unsubstituted arylthio group having 5 to 50 ring atoms, a substituted or unsubstituted alkoxycarbonyl group having 1 to 50 carbon atoms, a substituted or unsubstituted silyl group, a carboxyl group, a halogen atom, a cyano group, a nitro group, or a hydroxyl group.

An asymmetric pyrene derivative represented by the following formula (iii): 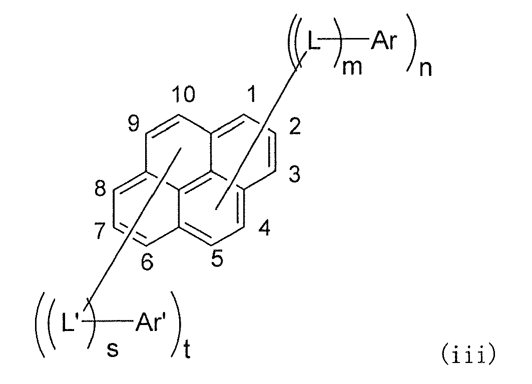 In the formula, **Ar** and **Ar'** each represent a substituted or unsubstituted aromatic group having 6 to 50 ring carbon atoms; **L** and L' each represent a substituted or unsubstituted phenylene group, a substituted or unsubstituted naphthalenylene group, a substituted or unsubstituted fluorenylene group, or a substituted or unsubstituted dibenzosilolylene group; **m** represents an integer of 0 to 2. **n** represents an integer of 1 to 4. **s** represents an integer of 0 to 2. **t** represents an integer of 0 to 4. In addition, **L** or **Ar** binds to any one of 1- to 5-positions of pyrene, and **L'** or **Ar'** binds to any one of 6- to 10-positions of pyrene; provided that **Ar, Ar', L,** and **L'** satisfy the following item (1) or (2) when **n** + t represents an even number.
(1) **Ar ≠ Ar'** and/or **L ≠ L'** (wherein the symbol "≠" means that groups connected with the symbol have different structures)
(2) When **Ar** = **Ar'** and **L** = **L'**,
   (2-1) **m ≠ s** and /or **n** ≠ **t,** or
   (2-2) when **m** = **s** and **n** = *t*,
      (2-2-1) both **L** and **L'** or pyrene bind(s) to different binding positions on **Ar** and **Ar',** or
      (2-2-2) both **L** and **L'** or pyrene bind (s) to the same binding positions on Ar and Ar', excluding the case where the substituting positions of **L** and **L',** or of **Ar** and **Ar'** in pyrene are 1-and 6-positions, or 2- and 7-positions.

An asymmetric anthracene derivative represented by the following the formula (iv): 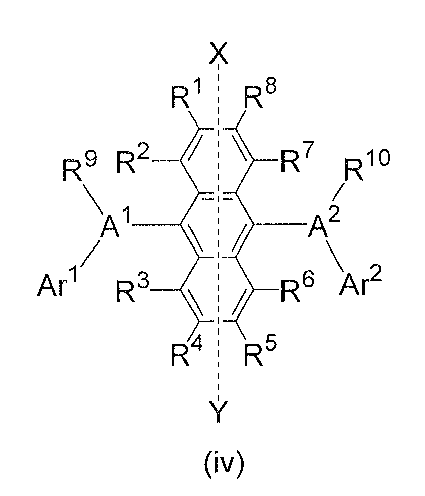 wherein A¹ and A² each independently represent a substituted or unsubstituted fused aromatic ring group having 10 to 20 ring carbon atoms.
Ar¹ and Ar² each independently represent a hydrogen atom, or a substituted or unsubstituted aromatic ring group having 6 to 50 ring carbon atoms.
R¹ to R¹⁰ each independently represent a hydrogen atom, a substituted or unsubstituted aromatic ring group having 6 to 50 ring carbon atoms, a substituted or unsubstituted aromatic heterocyclic group having 5 to 50 ring atoms, a substituted or unsubstituted alkyl group having 1 to 50 carbon atoms, a substituted or unsubstituted cycloalkyl group, a substituted or unsubstituted alkoxy group having 1 to 50 carbon atoms, a substituted or unsubstituted aralkyl group having 6 to 50 carbon atoms, a substituted or unsubstituted aryloxy group having 5 to 50 ring atoms, a substituted or unsubstituted arylthio group having 5 to 50 ring atoms, a substituted or unsubstituted alkoxycarbonyl group having 1 to 50 carbon atoms, a substituted or unsubstituted silyl group, a carboxyl group, a halogen atom, a cyano group, a nitro group, or a hydroxyl group.
The number of each of Ar¹, Ar², R⁹, and R¹⁰ may be two or more, and adjacent groups may form a saturated or unsaturated cyclic structure; provided that the case where groups symmetric with respect to the **X-Y** axis shown on central anthracene in the general formula (1) bind to 9- and 10-positions of the anthracene does not occur.

An anthracene derivative represented by the following general formula (v): 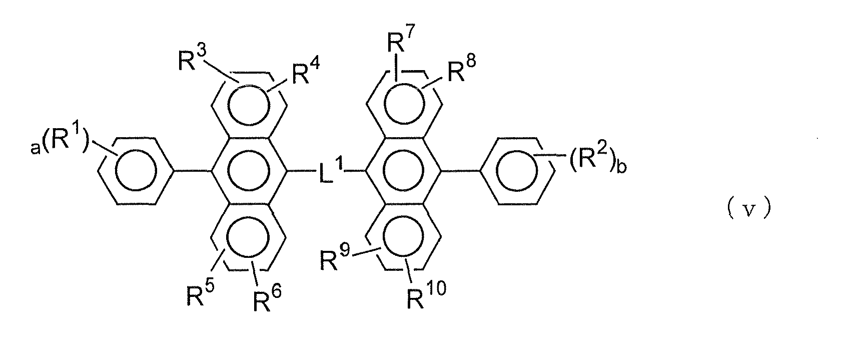 wherein R¹ to R¹⁰ each independently represent a hydrogen atom, an alkyl group, a cycloalkyl group, an aryl groupwhichmaybe substituted, an alkoxyl group, an aryloxy group, an alkylamino group, an alkenyl group, an arylamino group, or a heterocyclic group which may be substituted, **a** and **b** each represent an integer of 1 to 5, and, when **a** or **b** represents 2 or more, R¹'s or R²'s may be identical to or different from each other, and R¹'s or R²'s may bond each other to form a ring, R³ and R⁴, R⁵ and R⁶, R⁷ and R⁸, or R⁹ and R¹⁰ may bond each other to form a ring, and L¹ represents a single bond, -0-, -S-, -N(**R**)- wherein **R** represents an alkyl group or an aryl group which may be substituted, an alkylene group, or an arylene group.

An anthracene derivative represented by the following general formula (vi): 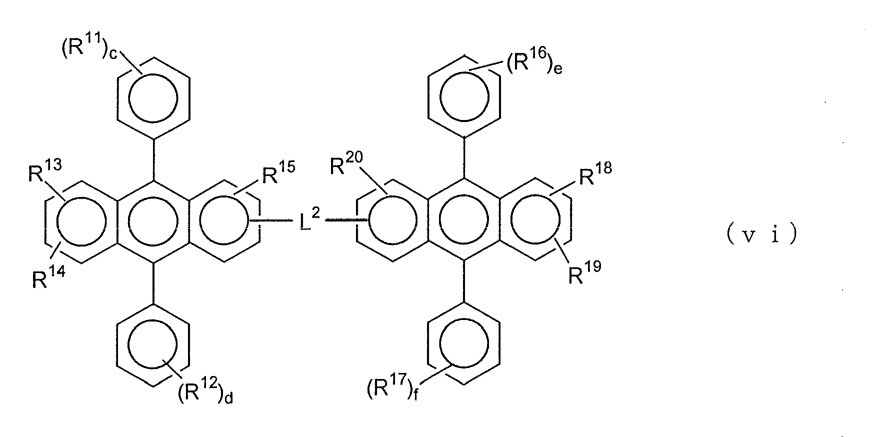 wherein R¹¹ to R²⁰ each independently represent a hydrogen atom, an alkyl group, a cycloalkyl group, an aryl group, an alkoxyl group, an aryloxy group, an alkylamino group, an arylamino group, or a heterocyclic group which may be substituted, **c, d, e,** and **f** each represent an integer of 1 to 5, and, when **c, d, e,** or **f** represents 2 or more, R¹¹' s, R¹²'s, R¹⁶' s, or R¹⁷'s may be identical to or different from each other, and R¹¹' s, R¹²'s, R¹⁶'s, or R¹⁷'s may bond each other to form a ring, R¹³ and R¹⁴, or R¹⁸ and R¹⁹ may bond each other to form a ring, and L² represents a single bond, -O-, -S-, -N (**R**) - wherein **R** represents an alkyl group or an aryl group which may be substituted, an alkylene group, or an arylene group.

A spirofluorene derivative represented by the following general formula (vii): 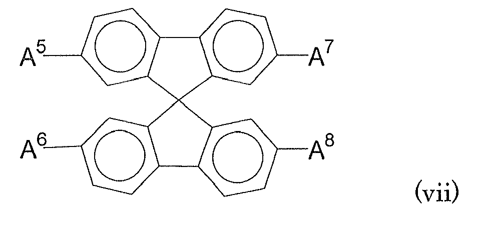 wherein A⁵ to A⁸ each independently represent a substituted or unsubstituted biphenyl group, or a substituted or unsubstituted naphthyl group.

A fused ring-containing compound represented by the following general formula (viii): 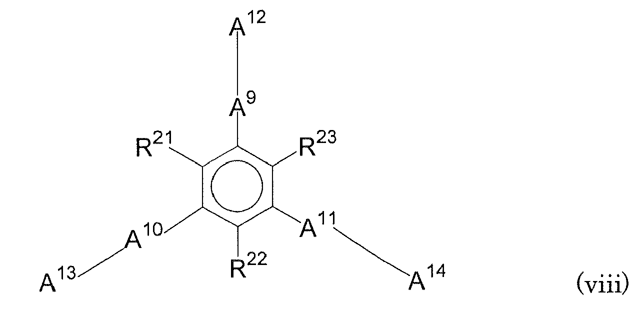 wherein A⁹ to A¹⁴ each are the same as described above, R²¹ to R²³ each independently represent a hydrogen atom, an alkyl group having 1 to 6 carbon atoms, a cycloalkyl group having 3 to 6 carbon atoms, an alkoxyl group having 1 to 6 carbon atoms, an aryloxy group having 5 to 18 carbon atoms, an aralkyloxy group having 7 to 18 carbon atoms, an arylamino group having 5 to 16 carbon atoms, a nitro group, a cyano group, an ester group having 1 to 6 carbon atoms, or a halogen atom, and at least one of A⁹ to A¹⁴ represents a group having three or more fused aromatic rings.

A fluorene compound represented by the following general formula (ix): 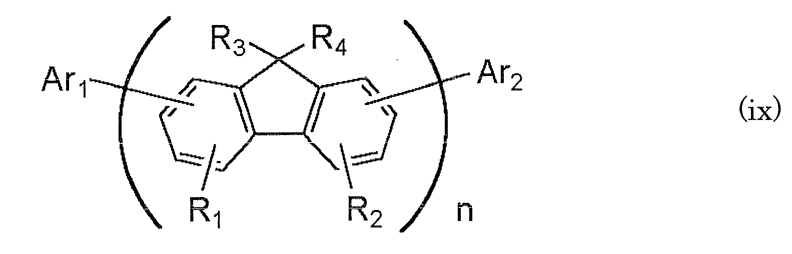 wherein R₁ and R₂ each represent a hydrogen atom, a substituted or unsubstituted alkyl group, a substituted or unsubstituted aralkyl group, a substituted or unsubstituted aryl group, a substituted or unsubstituted heterocyclic group, a substituted amino group, a cyano group, or a halogen atom, R₁'s or R₂'s bonded to different fluorene groups may be identical to or different from each other, and R₁ and R₂ bonded to the same fluorene group may be identical to or different from each other, R₃ and R₄ each represent a hydrogen atom, a substituted or unsubstituted alkyl group, a substituted or unsubstituted aralkyl group, a substituted or unsubstituted aryl group, or a substituted or unsubstituted heterocyclic group, R₃' s or R₄' s bonded to different fluorene groups may be identical to or different from each other, and R₃ and R₄ bonded to the same fluorene group may be identical to or different from each other, Ar₁ and Ar₂ each represent a substituted or unsubstituted fused polycyclic aromatic group having a total of 3 or more benzene rings, or a substituted or unsubstituted fused polycyclic heterocyclic group having a total of 3 or more benzene and heterocyclic rings and having carbon to be bonded to a fluorene group, and Ar₁ and Ar₂ may be identical to or different from each other; and **n** represents an integer of 1 to 10.

Of the foregoing host materials, an anthracene derivative is preferable, a monoanthracene derivative is more preferable, and an asymmetric anthracene is particularly preferable.
In addition, a phosphorescent compound can also be used as a light emitting material as a dopant. A compound obtained by incorporating a carbazole ring into a host material is a preferable phosphorescent compound. The dopant, which is a compound capable of emitting light from a triplet exciton, is not particularly limited as long as it emits light from a triplet exciton; the dopant is preferably a metal complex containing at least one metal selected from the group consisting of Ir, Ru, Pd, Pt, Os, and Re, and is preferably a porphyrin metal complex or an ortho-metalated metal complex.
A host composed of a compound containing a carbazole ring and suitable for phosphorescence is a compound having a function of causing a phosphorescent compound to emit light as a result of energy transfer from its excited state to the phosphorescent compound. A host compound is not particularly limited as long as it is a compound capable of transferring exciton energy to a phosphorescent compound, and can be appropriately selected depending on a purpose. The host compound may have an arbitrary heterocyclic ring or the like in addition to a carbazole ring.

Specific examples of such host compound include a carbazole derivative; a triazole derivative; an oxazole derivative; an oxadiazole derivative; an imidazole derivative; a polyarylalkane derivative; a pyrazoline derivative; a pyrazolone derivative; a phenylenediamine derivative; an arylamine derivative; an amino-substituted chalcone derivative; a styrylanthracene derivative; a fluorenone derivative; a hydrazone derivative; a stilbene derivative; a silazane derivative; an aromatic tertiary amine compound; a styrylamine compound; an aromatic dimethylidene-based compound; a porphyrin-based compound; an anthraquinodimethane derivative; an anthrone derivative; a diphenylquinone derivative; a thiopyran dioxide derivative; a carbodiimide derivative; a fluorenylidenemethane derivative; a distyrylpyrazine derivative; a heterocyclic tetracarboxylic anhydride such as naphthaleneperylene; a phthalocyanine derivative; various metal complex polysilane-based compounds typified by a metal complex of an 8-quinolinol derivative, a metal phthalocyanine, and a metal complex using benzoxazole or benzothiazole as a ligand; a electrically conductive polymeric oligomer such as a poly(N-vinylcarbazole) derivative, an aniline-based copolymer, a thiophene oligomer, or polythiophene; and a polymer compound such as a polythiophene derivative, a polyphenylene derivative, a polyphenylene vinylene derivative, or a polyfluorene derivative. One kind of a host compound may be used alone, or two or more kinds of host compounds may be used in combination.
Specific examples of the host compound include the following compounds.

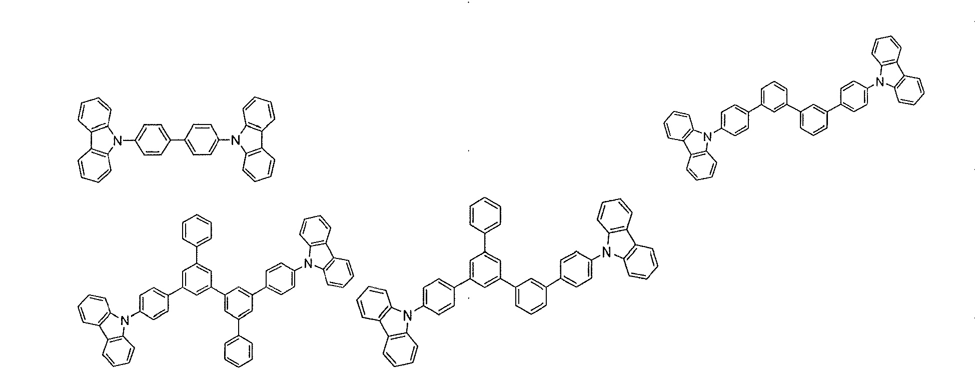

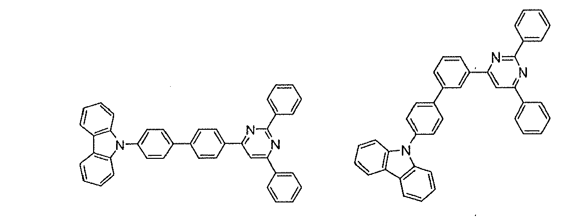

In addition, in the organic EL device of the present invention, the light emitting layer may contain a fluorescent or phosphorescent dopant in addition to the aromatic amine compound of the present invention and/or the light emitting material.
At least one of an arylamine compound and a styrylamine compound is additionally preferable as the fluorescent dopant.
The styrylamine compound is preferably one represented by the following general formula (I):

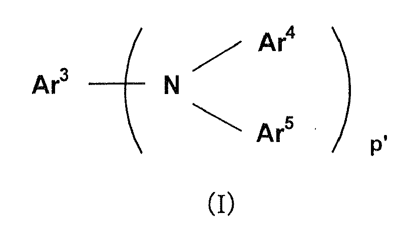

wherein Ar³ represents a group selected from a phenyl group, a biphenyl group, a terphenyl group, a stilbene group, and a distyrylaryl group, Ar⁴ and Ar⁵ each represent a hydrogen atom or an aromatic group having 6 to 20 carbon atoms, and each of Ar³ to Ar⁵ may be substituted, **P'** represents an integer of 1 to 4, and at least one of Ar⁹ and Ar⁵ is additionally preferably substituted by a styryl group.
Here, examples of the aromatic group having 6 to 20 carbon atoms include a phenyl group, a naphthyl group, an anthranyl group, a phenanthryl group, and a terphenyl group.

The arylamine compound is preferably one represented by the following general formula (II): 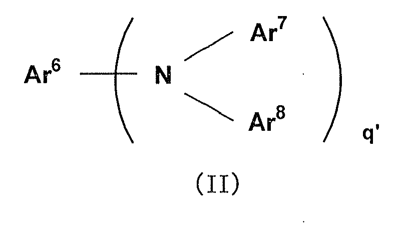 wherein Ar⁶ to Ar⁸ each represent a substituted or unsubstituted aryl group having 5 to 40 ring carbon atoms, and **q'** represents an integer of 1 to 4.

Here, examples of the aryl group having 5 to 40 ring carbon atoms include a phenyl group, a naphthyl group, a chrysenyl group, a naphthacenyl group, an anthranyl group, a phenanthryl group, a pyrenyl group, a coronyl group, a biphenyl group, a terphenyl group, a pyrrolyl group, a furanyl group, a thiophenyl group, a benzothiophenyl group, an oxadiazolyl group, a diphenylanthranyl group, an indolyl group, a carbazolyl group, a pyridyl group, a benzoquinolyl group, a fluoranthenyl group, an acenaphthofluoranthenyl group, and a stilbene group. It should be noted that examples of a preferable substituent for the aryl group include: an alkyl group having 1 to 6 carbon atoms (such as an ethyl group, a methyl group, an i-propyl group, an n-propyl group, an s-butyl group, a t-butyl group, a pentyl group, a hexyl group, a cyclopentyl group, or a cyclohexyl group) ; an alkoxy group having 1 to 6 carbon atoms (such as an ethoxy group, a methoxy group, an i-propoxy group, an n-propoxy group, an s-butoxy group, a t-butoxy group, a pentoxy group, a hexyloxy group, a cyclopentoxy group, or a cyclohexyloxy group); an aryl group having 5 to 40 ring atoms; an amino group substituted by an aryl group having 5 to 40 ring atoms; an ester group having an aryl group having 5 to 40 ring atoms; an ester group having an aryl group having 1 to 6 carbon atoms; a cyano group; a nitro group; and a halogen atom.

In addition, the phosphorescent dopant to be used in combination with the host material in the light emitting layer is preferably a metal complex; the dopant is preferably a metal complex compound containing at least one metal selected from Ir, Ru, Pd, Pt, Os, and Re, and a ligand of the complex preferably has at least one skeleton selected from a phenylpyridine skeleton, a bipyridyl skeleton, and a phenanthroline skeleton. Specific examples of such metal complex include, but not limited to, tris (2-phenylpyridine) iridium, tris (2-phenylpyridine) ruthenium, tris (2-phenylpyridine) palladium, bis (2-phenylpyridine) platinum, tris(2-phenylpyridine)osmium, tris(2-phenylpyridine)rhenium, octaethyl platinum porphyrin, octaphenyl platinum porphyrin, octaethyl palladium porphyrin, and octaphenyl palladium porphyrin. A proper complex is selected depending on a required luminescent color, device performance, and a relationship with the host material.

In the present invention, a known method such as a deposition method, a spin coatingmethod, oranLBmethodisapplicable to the formation of the light emitting layer by using the various light emitting materials. The light emitting layer is particularly preferably a molecular deposit film. The term "molecular deposit film" as used herein refers to a thin film formed as a result of the deposition of a material compound in a vapor phase state or a film formed as a result of the solidification of a material compound in a solution state or a liquid phase state. The molecular deposit film can be typically distinguished from a thin film (molecular accumulation film) formed by an LB method on the basis of a difference in agglomerated structure or higher-order structure and a functional difference resulting from the difference. In addition, as disclosed in JP 57-51781 A, the light emitting layer can be formed by: dissolving a binder such as a resin and a material compound in a solvent to prepare a solution; and turning the solution into a thin film by a spin coating method or the like.
The thickness of the light emitting layer to be formed as described above is not particularly limited, and can be appropriately selected as circumstances demand; the thickness is preferably in the range of 5 nm to 5 µm in ordinary cases. The light emitting layer may be constituted of one layer composed of one or two or more kinds of the above-mentioned materials. Alternatively, a light emitting layer composed of a compound different from that of the light emitting layer may be laminated on the light emitting layer.
When the aromatic amine compound of the present invention is used in a light emitting region or a light emitting layer, the light emitting region or the light emitting layer may be composed of a single layer using one or more kinds of the above-mentioned materials as long as the region or the layer comprises the compound of the present invention.

### (5) Hole injecting/transporting layer

The hole injecting/transporting layer is a layer which helps injection of holes into the light emitting layer and transports the holes to the light emitting region. The hole injecting/transporting layer exhibits a great mobility of holes and, in general, has an ionization energy as small as 5.5 eV or smaller. For such the hole injecting/transporting layer, a material which transports holes to the light emitting layer under an electric field of a smaller strength is preferable. A material which exhibits, for example, a mobility of holes of at least 10⁻⁴ cm²/V·sec under application of an electric field of 10⁴ to 10⁶ V/cm is preferable.
When the aromatic amine compound of the present invention is used in the hole transporting region, the compound of the present invention may be used alone or as a mixture with other materials for forming the hole injecting/transporting layer.
The material which can be used for forming the hole injecting/transporting layer as a mixture with the aromatic amine compound of the present invention is not particularly limited as long as the material has a preferable property described above. The material can be arbitrarily selected from materials which are conventionally used as the charge transporting material of holes in photoconductive materials and known materials which are used for the hole injecting/transporting layer in organic EL devices.

Specific examples include a triazole derivative (see, for example, US Patent No. 3,112,197); an oxadiazole derivative (see, for example, US Patent No. 3,189,447); an imidazole derivative (see, for example, JP 37-16096B); a polyarylalkane derivative (see, for example, US Patent Nos. 3,615,402; 3,820,989; 3,542,544; JP 45-555 B, JP 51-10983 B, JP 51-93224 A, JP 55-17105 A, JP 56-4148 A, JP 55-108667 A, JP 55-156953 A, and JP 56-36656 A) ; a pyrazoline derivative and a pyrazolone derivative (see, for example, US Patent Nos. 3,180,729; 278,746; JP 55-88064 A, JP 55-88065 A, JP 49-105537 A, JP 55-51086 A, JP 56-80051 A, JP 56-88141 A, JP 57-45545 A, JP 54-112637 A, and JP 55-74546 A) ; a phenylenediamine derivative (see, for example, US Patent No. 3,615,404, JP 51-10105 B, JP 46-3712 B, JP 47-25336 B, JP 54-53435 A, JP 54-110536 A, and JP 54-119925 A); an arylamine derivative (see, for example, US Patent Nos. 3,567,450; 3,180,703; 3,240,597; 3,658,520; 4,232,103; 4, 175, 961; 4,012,376; JP 49-35702 B, JP 39-27577 B, JP 55-144250 A, JP 56-119132 A, JP 56-22437 A, and DE 1, 110, 518) ; an amino-substituted chalcone derivative (see, for example, Patent No. 3,526,501); an oxazole derivative (those disclosed in US Patent No. 3,257,203); a styrylanthracene derivative (see, for example, JP 56-46234 A); a fluorenone derivative (see, for example, JP 54-110837 A) ; a hydrazone derivative (see, for example, US Patent No. 3, 717, 462, JP 54-59143 A, JP 55-52063 A, JP 55-52064 A, JP 55-46760 A, JP 55-85495 A, JP 57-11350 A, JP 57-148749 A, and JP 2-311591 A); a stilbene derivative (see, for example, JP 61-210363 A, JP 61-228451 A, JP 61-14642 A, JP61-72255A, JP62-47646A, JP62-36674A, JP62-10652A, JP62-30255 A, JP 60-93445 A, JP 60-94462 A, JP 60-174749 A, and JP 60-175052 A); a silazane derivative (US Patent No. 4,950,950); a polysilane-based copolymer (JP 02-204996 A); an aniline-based copolymer (JP 02-282263 A); and a electrically conductive high molecular weight oligomer (particularly a thiophene oligomer) disclosed in JP 01-211399 A.

In addition to the above-mentioned materials which can be used as the material for the hole injecting/transporting layer, a porphyrin compound (those disclosed in, for example, JP 63-2956965 A) ; an aromatic tertiary amine compound and a styrylamine compound (see, for example, US Patent No. 4, 127, 412, JP 53-27033 A, JP 54-58445 A, JP 54-149634 A, JP 54-64299 A, JP 55-79450 A, JP 55-144250 A, JP 56-119132 A, JP 61-295558 A, JP 61-98353 A, and JP 63-295695 A) are preferable, and the aromatic tertiary amine compound is particularly preferable.
Further examples of aromatic tertiary amine compounds include compounds having two fused aromatic rings in the molecule such as 4,4'-bis(N-(1-naphthyl)-N-phenylamino)-biphenyl (hereinafter referred to as NPD) as disclosed in US 5,061,569, and a compound in which three triphenylamine units are bonded together in a star-burst shape, such as 4,4',4"-tris(N-(3-methylphenyl)-N-phenylamino)-triphenylamine (hereinafter referred to as MTDATA) as disclosed in JP 04-308688 A.
Further, in addition to the aromatic dimethylidene-based compounds described above as the material for the light emitting layer, inorganic compounds such as Si of the p-type and SiC of the p-type can also be used as the material for the hole injecting layer.

The hole injecting/transporting layer can be formed by forming a thin layer from the aromatic amine compound of the present invention or the above-mentioned compounds by a known process such as the vacuum vapor deposition process, the spin coating process, the casting process and the LB process. The thickness of the hole injecting/transporting layer is not particularly limited. In general, the thickness is 5 nm to 5 µm. Preferably, the hole injecting/transporting layer contains the compound of the present invention incorporated in the hole injecting/transporting region, and may be constituted of a single layer containing one or more materials described above or may be a laminate constituted of hole injecting/transporting layers containing compounds different from those of the hole injecting/transporting layer described above.
Further, an organic semiconductor layer may be disposed as a layer for helping the injection of holes or electrons into the light emitting layer. As the organic semiconductor layer, a layer having a conductivity of 10⁻¹⁰ S/cm or greater is preferable. As the material for the organic semiconductor layer, oligomers containing thiophene, and electrically conductive oligomers such as oligomers containing arylamine and electrically conductive dendrimers such as dendrimers containing arylamine which are disclosed in JP 08-193191 A, can be used.

### (6) Electron injecting layer

The electron injecting/transporting layer is a layer which helps injection of electrons into the light emitting layer and exhibits a great mobility of electrons. An adhesion improving layer is an electron injecting/transporting layer comprising a material exhibiting particularly improved adhesion with the cathode. A metal complex of 8-hydroxyquinoline or of a derivative of 8-hydroxyquinoline, or an oxadiazole derivative is suitable as a material to be used in an electron injecting/transporting layer.
Specific examples of the metal complex of 8-hydroxyquinoline or of a derivative of 8-hydroxyquinoline include metal chelate oxynoid compounds each containing a chelate of oxine (generally 8-quinolinol or 8-hydroxyquinoline). For example, Alq exemplified as a light-emitting material can be used as a electron injecting material.
On the other hand, examples of the oxadiazole derivative include electron transfer compounds represented by the following general formulae:

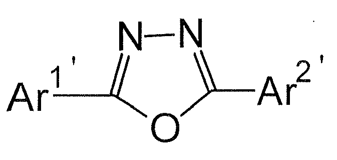

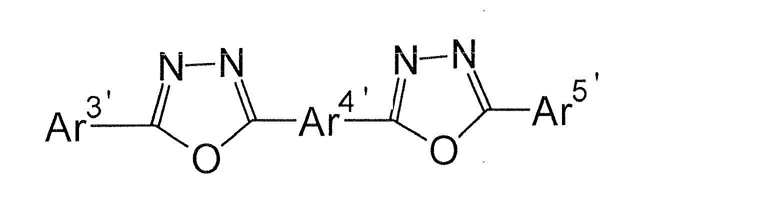

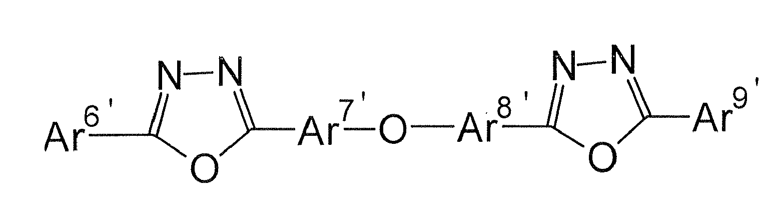

wherein Ar¹, Ar², Ar³, Ar⁵, Ar⁶ and Ar⁹ each represent a substituted or unsubstituted aryl group and may represent the same group or different groups. Ar⁴, Ar⁷ and Ar⁸ each represent a substituted or unsubstituted arylene group and may represent the same group or different groups.

Examples of the aryl group include a phenyl group, a biphenyl group, an anthranyl group, a perylenyl group, and a pyrenyl group. Examples of the arylene group include a phenylene group, a naphthylene group, a biphenylene group, an anthranylene group, a perylenylene group, and a pyrenylene group. Examples of the substituent include alkyl groups having 1 to 10 carbon atoms, alkoxyl groups having 1 to 10 carbon atoms, and a cyano group.
In addition, examples of a material for an electron injecting/transporting layer include following compounds.
A heterocyclic derivative having a nitrogen atom represented by a following general formula [1] described in JP 2004-002297 A:

**HAr**-L¹-Ar¹-Ar² [1]

wherein L¹ represents a single bond, an arylene group having 6 to 60 carbon atoms which may have a substituent, a heteroarylene group having 3 to 60 carbon atoms which may have a substituent, or a fluorenylene group which may have a substituent, Ar¹ represents a divalent aromatic hydrocarbon group having 6 to 60 carbon atoms which may have a substituent, Ar² represents an aryl group having 6 to 60 carbon atoms which may have a substituent, or a heteroaryl group having 3 to 60 carbon atoms which may have a substituent, and **HAr** represents any one of heterocyclic rings having a nitrogen atom that are shown below.

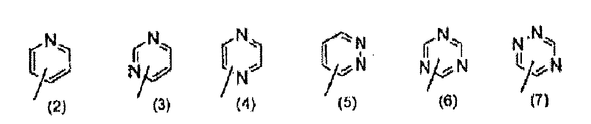

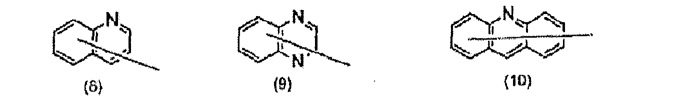

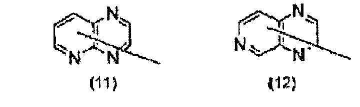

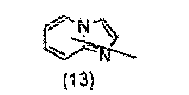

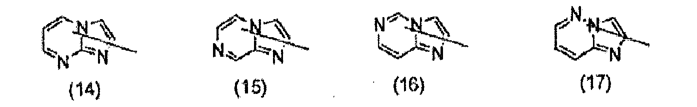

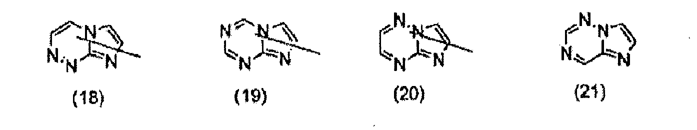

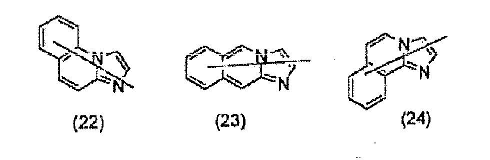

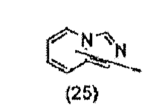

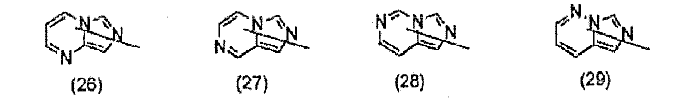

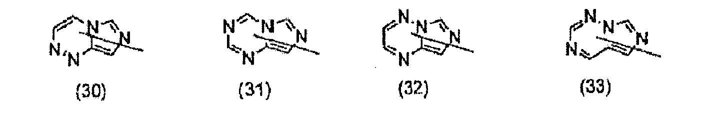

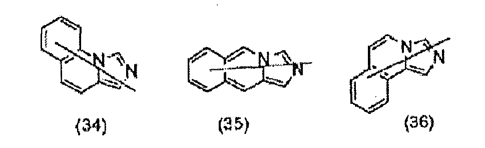

A heterocyclic derivative having a nitrogen atom represented by the following general formula [2] or [3]:

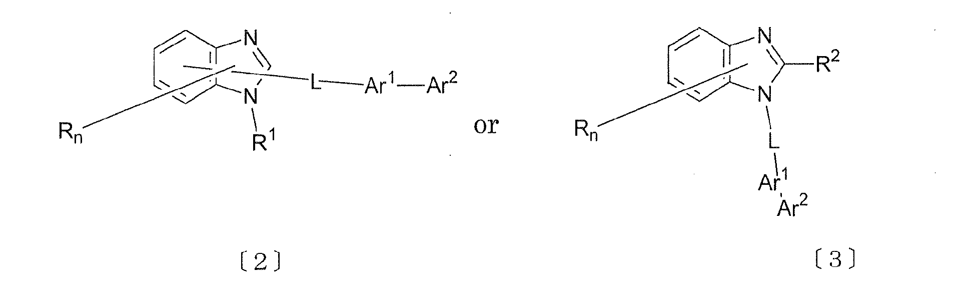

wherein **R** represents a hydrogen atom, an aryl group which has 6 to 60 carbon atoms and which may have a substituent, a pyridyl group which may have a substituent, a quinolyl group which may have a substituent, an alkyl group having 1 to 20 carbon atoms, a haloalkyl group having 1 to 20 carbon atoms, or an alkoxy group having 1 to 20 carbon atoms, n represents an integer of 0 to 4, R¹ represents an aryl group which has 6 to 60 carbon atoms and which may have a substituent, a pyridyl groupwhichmayhave a substituent, a quinolyl group which may have a substituent, an alkyl group having 1 to 20 carbon atoms, a haloalkyl group having 1 to 20 carbon atoms, or an alkoxy group having 1 to 20 carbon atoms, R² represents a hydrogen atom, an aryl group which has 6 to 60 carbon atoms and which may have a substituent, a pyridyl group which may have a substituent, a quinolyl group which may have a substituent, an alkyl group having 1 to 20 carbon atoms, a haloalkyl group having 1 to 20 carbon atoms, or an alkoxy group having 1 to 20 carbon atoms, L represents an arylene group which has 6 to 60 carbon atoms and which may have a substituent, a pyridinylene group which may have a substituent, a quinolinylene group which may have a substituent, or a fluorenylene group which may have a substituent, Ar¹ represents an arylene group which has 6 to 60 carbon atoms and which may have a substituent, a pyridinylene group which may have a substituent, or a quinolinylene group which may have a substituent, and Ar² represents an alkyl group having 1 to 20 carbon atoms, a haloalkyl group having 1 to 20 carbon atoms, an alkoxy group having 1 to 20 carbon atoms, an aryl group which has 6 to 60 carbon atoms and which may have a substituent, a pyridyl group which may have a substituent, or a quinolyl group which may have a substituent.

Aborane derivative represented by the following general formula [4] described in JP 2000-40586 A:

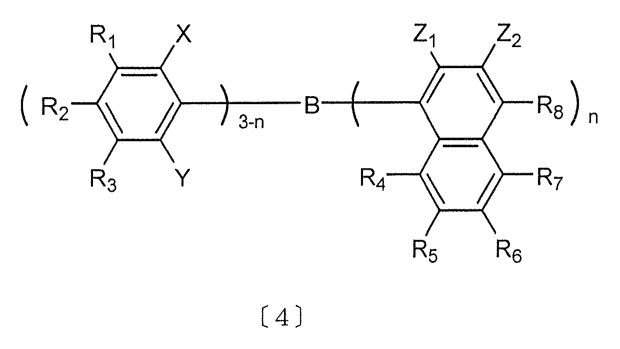

wherein R₁ to R₈ and **Z₂** each independently represent a hydrogen atom, a saturated or unsaturated hydrocarbon group, an aromatic group, a heterocyclic group, a substituted amino group, a substituted boryl group, an alkoxy group, or an aryloxy group; **X, Y,** and **Z₁** each independently represent a saturated or unsaturated hydrocarbon group, an aromatic group, a heterocyclic group, a substituted amino group, an alkoxy group, or an aryloxy group; substituents of **Z₁** and **Z₂** may bond each other to form a fused ring; and n represents an integer of 1 to 3, and, when **n** represents 2 or more, Z₁'s may be different from each other provided that the case wherein **n** represents 1, **X, Y**, and R₂ each represent a methyl group, R₈ represents a hydrogen atom or a substituted boryl group and the case wherein n represents 3 and **Z₁**'s each represent a methyl group are excluded.

A silacyclopentadiene derivative represented by the following general formula [5] described in JP 09-194487 A:

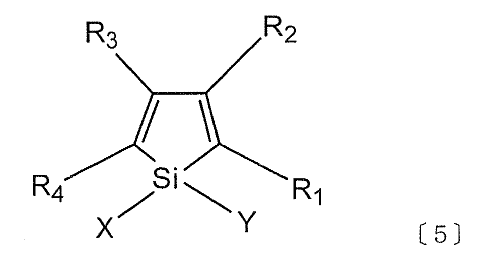

wherein **X** and **Y** each independently represent a saturated or unsaturated hydrocarbon group having 1 to 6 carbon atoms, an alkoxy group, an alkenyloxy group, an alkynyloxy group, a hydroxy group, a substituted or unsubstituted aryl group, or a substituted or unsubstituted heterocycle, or represent a structure made by bonding **X** and **Y** each other to form a saturated or unsaturated ring; and R₁ to R₄ each independently represent hydrogen, a halogen atom, a substituted or unsubstituted alkyl group having 1 to 6 carbon atoms, an alkoxy group, an aryloxy group, a perfluroalkyl group, a perfluoroalkoxy group, an amino group, an alkylcarbonyl group, an arylcarbonyl group, an alkoxycarbonyl group, an aryloxycarbonyl group, an azo group, an alkylcarbonyloxy group, an arylcarbonyloxy group, an alkoxycarbonyloxy group, an aryloxycarbonyloxy group, a sulfinyl group, a sulfonyl group, a sulfanyl group, a silyl group, carbamoyl group, an aryl group, a heterocyclic group, an alkenyl group, an alkynyl group, a nitro group, a formyl group, a nitroso group, a formyloxy group, an isocyano group, a cyanate group, an isocyanate group, a thiocyanate group, an isothiocyanate group, or a cyano group, or, when two or more of R₁ to R₄ are adjacent to each other, they form a structure in which a substituted or unsubstituted ring is fused.

A compound represented by the following general formula [6] described in JP 10-88121 A: 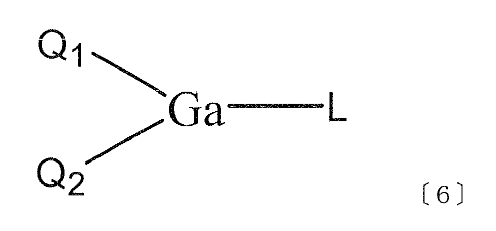 wherein L represents a halogen atom, a substituted or unsubstituted alkyl group, a substituted or unsubstituted cycloalkyl group, a substituted or unsubstituted aryl group, a substituted or unsubstituted heterocyclic group, -OR¹ wherein R¹ represents a halogen atom, a substituted or unsubstituted alkyl group, a substituted or unsubstituted cycloalkyl group, a substituted or unsubstituted aryl group, or a substituted or unsubstituted heterocyclic group, or a ligand represented by -O-Ga-Q³ (Q⁴) wherein Q³ and Q⁴ each have the same meaning as that of each of following Q¹ and Q²; and
Q¹ and Q² each independently represent a ligand represented by the following general formula [7]:

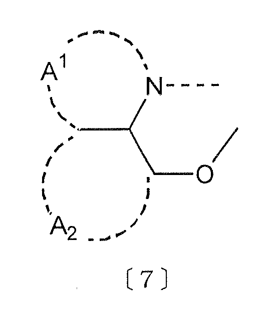

wherein rings A¹ and A² are six-membered aryl ring structures which may have substituents and which are fused with each other.

The metal complex has strong n-type semiconductor-like property, and has a large electron injecting ability. Further, energy to be generated at the time of the formation of the complex is low, so bonding property between the metal and ligand of the formed metal complex becomes strong, and hence the fluorescent quantum efficiency of the complex as a light emitting material becomes large.
Specific examples of a substituent for each of Rings A¹ and A² of which the ligand represented by the general formula [7] is formed include a halogen atom such as chlorine, bromine, iodine, or fluorine; a substituted or unsubstituted alkyl group such as a methyl group, an ethyl group, a propyl group, a butyl group, an sec-butyl group, a tert-butyl group, a pentyl group, a hexyl group, a heptyl group, an octyl group, a stearyl group, or a trichloromethyl group; a substituted or unsubstituted aryl group such as a phenyl group, a naphthyl group, a 3-methylphenyl group, a 3-methoxyphenyl group, a 3-fluorophenyl group, a 3-trichloromethylphenyl group, a 3-trifluoromethylphenyl group, or a 3-nitrophenyl group; a substituted or unsubstituted alkoxy group such as a methoxy group, an n-butoxy group, a tert-butoxy group, a trichloromethoxy group, a trifluoroethoxy group, a pentafluoropropoxy group, a 2,2,3,3-tetrafluoropropoxy group, a 1,1,1,3,3,3-hexafluoro-2-propoxy group, or a 6-(perfluoroethyl)hexyloxy group; a substituted or unsubstituted aryloxy group such as a phenoxy group, a p-nitrophenoxy group, a p-tert-butylphenoxy group, a 3-fluorophenoxy group, a pentafluorophenyl group, or a 3-trifluoromethylphenoxy group; a substituted or unsubstituted alkylthio group such as a methylthio group, an ethylthio group, a tert-butylthio group, a hexylthio group, an octylthio group, or a trifluoromethylthio group; a substituted or unsubstituted arylthio group such as a phenylthio group, a p-nitrophenylthio group, a ptert-butylphenylthio group, a 3-fluorophenylthio group, a pentafluorophenylthio group, or a 3-trifluoromethylphenylthio group; a cyano group; a nitro group; an amino group; a mono- or di-substituted amino group such as a methylamino group, a diethylamino group, an ethylamino group, a diethylamino group, a dipropylamino group, a dibutylamino group, or a diphenylamino group; an acylamino group such as a bis (acetoxymethyl) amino group, a bis(acetoxyethyl)amino group, a bisacetoxypropyl)amino group, or a bis(acetoxybutyl)amino group; a hydroxyl group; a siloxy group; an acyl group; a carbamoyl group such as a methylcarbamoyl group, a dimethylcarbamoyl group, an ethylcarbamoyl group, a diethylcarbamoyl group, a propylcarbamoyl group, a butylcarbamoyl group, or a phenylcarbamoyl group; a carboxylic group; a sulfonic group; an imide group; a cycloalkyl group such as a cyclopentane group or a cyclohexyl group; an aryl group such as a phenyl group, a naphthyl group, a biphenyl group, an anthranyl group, a phenanthryl group, a fluorenyl group, or a pyrenyl group; and a heterocyclic group such as a pyridinyl group, a pyrazinyl group, a pyrimidinyl group, a pyridazinyl group, a triazinyl group, an indolinyl group, a quinolinyl group, an acridinyl group, a pyrrolidinyl group, a dioxanyl group, a piperidinyl group, a morpholidinyl group, a piperazinyl group, a triathinyl group, a carbazolyl group, a furanyl group, a thiophenyl group, an oxazolyl group, an oxadiazolyl group, a benzoxazolyl group, a thiazolyl group, a thiadiazolyl group, a benzothiazolyl group, a triazolyl group, an imidazolyl group, a benzoimidazolyl group, or a pranyl group. In addition, two or more of the foregoing substituents may bond each other to form an additional six-membered aryl or heterocyclic ring.
Examples of the residue of the general formula [7] include, but not limited to, quinoline residues such as 8-hydroxyquinoline and 2-methyl-8-hydroxyquinoline.

A compound represented by any one of the following general formulae [8] to [11] described in JP 1993-3314579 A: 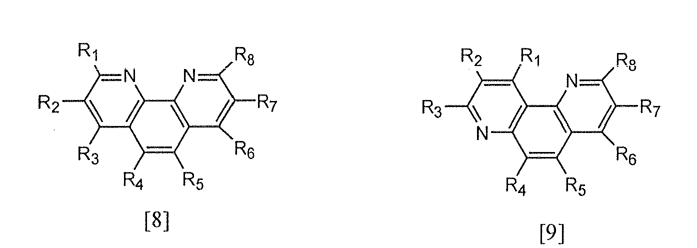 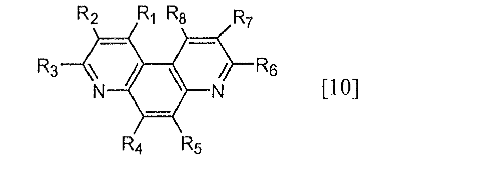 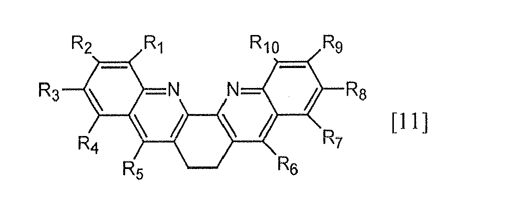 wherein R₁ to R₈ each independently represent a halogen atom, a substituted or unsubstituted alkyl group, a substituted or unsubstituted aryl group, a substituted or unsubstituted amino group, a halogen atom, a nitro group, a cyano group, or a hydroxyl group.

In addition to those described above, a hetero atom-containing compound disclosed in each of JP 2001-006877 A, JP 2002-038141 A, JP 10-106749 A, JP 2002-158093 A, and PCT international Publication No. WO 03/060956 A can also be used.
Each of those electron transferring compounds preferably has thin-film forming ability.
Specific examples of such electron transferring compound include the following.

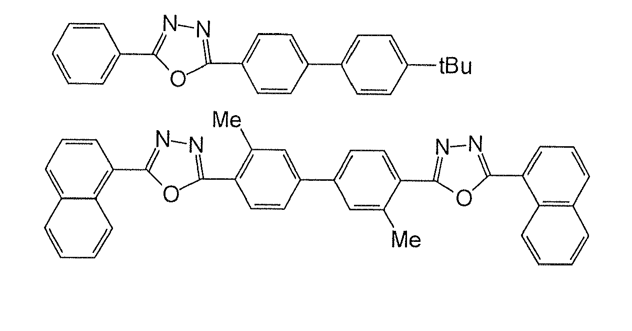

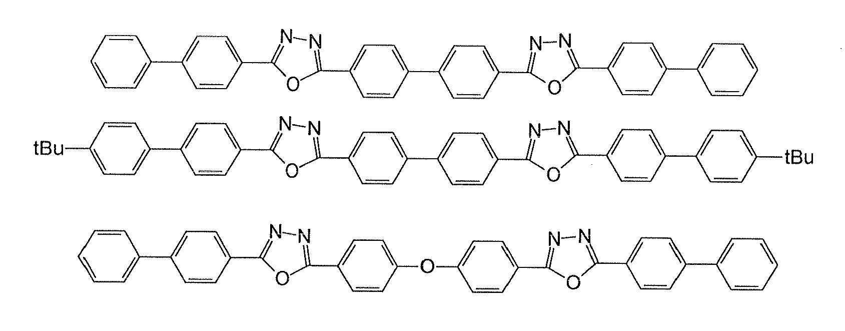

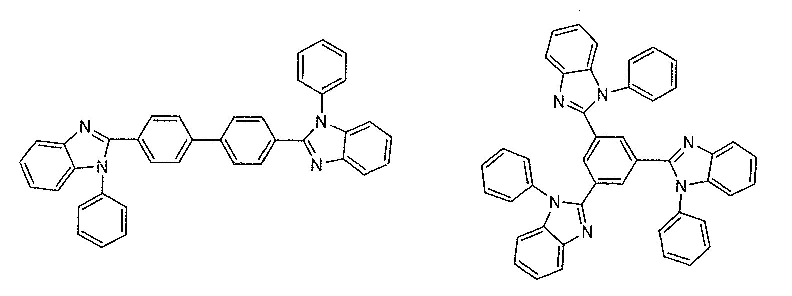

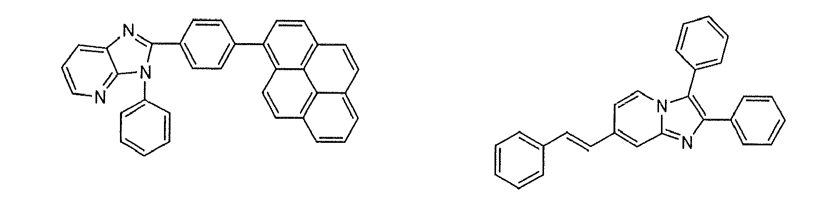

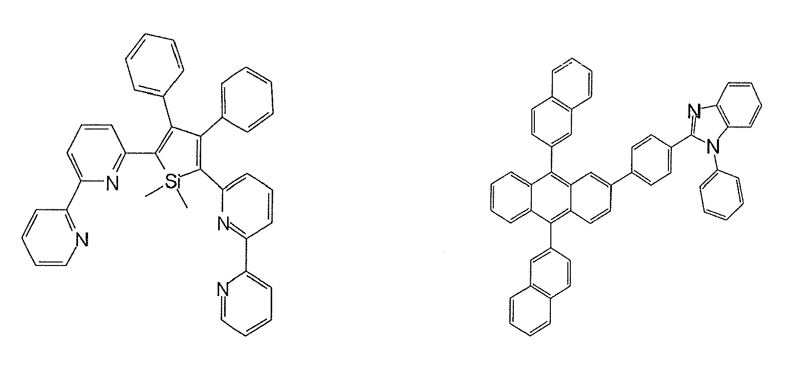

In addition, in the organic EL device of the present invention, the electron injecting/transporting layer preferably contains a reducing dopant. The reducing dopant may be incorporated into an interfacial region between a region wherein an electron is transported or the cathode and the organic thin film layer. The reducing dopant is defined as a substance which can reduce a compound having the electron transporting property. Various compounds can be used as the reducing dopant as long as the compounds have a uniform reductive property. For example, at least one substance selected from the group consisting of alkali metals, alkaline earth metals, rare earth metals, alkali metal oxides, alkali metal halides, alkaline earth metal oxides, alkaline earth metal halides, rare earth metal oxides, rare earth metal halides, organic complexes of alkali metals, organic complexes of alkaline earth metals, and organic complexes of rare earth metals can be preferably used.

Specific examples of the reducing dopant include substances having a work function of 2.9 eV or smaller, specific examples of which include at least one alkali metal selected from the group consisting of Na (the work function: 2.36 eV), K (the work function: 2.28 eV), Rb (the work function: 2.16 eV), and Cs (the work function: 1.95 eV) and at least one alkaline earth metal selected from the group consisting of Ca (the work function: 2.9 eV), Sr (the work function: 2.0 to 2.5 eV), and Ba (the work function: 2.52 eV). Among the above-mentioned substances, at least one alkali metal selected from the group consisting of K, Rb, and Cs is more preferable, Rb and Cs are still more preferable, and Cs is most preferable as the reducing dopant. Those alkali metals have great reducing ability, and the luminance of the emitted light and the lifetime of the organic EL device can be increased by addition of a relatively small amount of the alkali metal into the electron injecting region. As the reducing dopant having a work function of 2.9 eV or smaller, combinations of two or more alkali metals thereof are also preferable. Combinations having Cs such as the combinations of Cs and Na, Cs and K, Cs and Rb, and Cs, Na, and K are more preferable. The reducing ability can be efficiently exhibited by the combination having Cs. The luminance of emitted light and the lifetime of the organic EL device can be increased by adding the combination having Cs into the electron injecting region.

The organic EL device of the present invention may further include an electron injecting layer which is composed of an electrically insulating material or a semiconductor and disposed between the cathode and the organic layer. With the structure, leak of electric current can be effectively prevented by the electron injecting layer and the electron injecting property can be improved. As the electrically insulating material, at least one metal compound selected from the group consisting of alkali metal chalcogenides, alkaline earth metal chalcogenides, alkali metal halides, and alkaline earth metal halides is preferable. It is preferable that the electron injecting layer be composed of the above-mentioned substance such as the alkali metal chalcogenide since the electron injecting property can be further improved. Preferable examples of the alkali metal chalcogenide include Li₂O, LiO, Na₂S, Na₂Se, and NaO. Specifically, preferable examples of the alkaline earth metal chalcogenide include CaO, BaO, SrO, BeO, BaS, and CaSe. Preferable examples of the alkali metal halide include LiF, NaF, KF, LiCl, KCl, and NaCl. Preferable examples of the alkaline earth metal halide include fluorides such as CaF₂, BaF₂, SrF₂, MgF₂, and BeF₂ and halides other than the fluorides.

Examples of the semiconductor composing the electron transporting layer include oxides, nitrides, and oxide nitrides of at least one element selected from Ba, Ca, Sr, Yb, Al, Ga, In, Li, Na, Cd, Mg, Si, Ta, Sb, and Zn used alone or in combination of two or more. It is preferable that the inorganic compound composing the electron transporting layer form a crystallite or amorphous electrically insulating thin film. When the electron injecting layer is composed of the electrically insulating thin film described above, a more uniform thin film can be formed, and defects of pixels such as dark spots can be decreased. Examples of the inorganic compound include alkali metal chalcogenides, alkaline earth metal chalcogenides, alkali metal halides, and alkaline earth metal halides which are described above.

### (7) Cathode

As the cathode, a material such as a metal, an alloy, a electrically conductive compound, or a mixture of those materials which has a small work function (4 eV or smaller) is used because the cathode is used for injecting electrons to the electron injecting/transporting layer or the light emitting layer. Specific examples of the electrode material include sodium, sodium-potassium alloys, magnesium, lithium, magnesium-silver alloys, aluminum/aluminum oxide, aluminum-lithium alloys, indium, and rare earth metals.
The cathode can be prepared by forming a thin film of the electrode material described above by a process such as the vapor deposition process and the sputtering process.
When the light emitted from the light emitting layer is obtained through the cathode, it is preferable that the cathode have a transmittance of the emitted light greater than 10%.
It is also preferable that the sheet resistance of the cathode be several hundred Ω/□ or smaller. The thickness of the cathode is, in general, selected in the range of 10 nm to 1 µm and preferably in the range of 50 to 200 nm.

### (8) Electrically insulating layer

Defects in pixels tend to be formed in organic EL device due to leak and short circuit since an electric field is applied to ultra-thin films. To prevent the formation of the defects, a layer of a thin film having an electrically insulating property may be inserted between the pair of electrodes.
Examples of the material used for the electrically insulating layer include aluminum oxide, lithium fluoride, lithium oxide, cesium fluoride, cesium oxide, magnesium oxide, magnesium fluoride, calcium oxide, calcium fluoride, aluminum nitride, titanium oxide, silicon oxide, germanium oxide, silicon nitride, boron nitride, molybdenum oxide, ruthenium oxide, and vanadium oxide. Mixtures and laminates of the above-mentioned compounds may also be used.

### (9) Method of fablicating the organic EL device

To fabricate the organic EL device of the present invention, the anode and the light emitting layer, and, where necessary, the hole injecting/transporting layer and the electron injecting/transporting layer are formed by the illustrated process using the illustrated materials, and the cathode is further formed. The organic EL device may also be fabricated by forming the above-mentioned layers in the order reverse to that described above, i.e., the cathode being formed in the first step and the anode in the last step.
Hereinafter, an example of the process for preparing an organic EL device having a construction in which an anode, a hole injecting layer, a light emitting layer, an electron injecting layer, and a cathode are disposed successively on a substrate transmitting light will be described.
On a suitable substrate which transmits light, a thin film made of a material for the anode is formed by the vapor deposition process or the sputtering process so that the thickness of the formed thin film is 1µm or smaller and preferably in the range of 10 to 200 nm. The formed thin film is used as the anode. Then, a hole injecting layer is formed on the anode. The hole injecting layer can be formed by the vacuum vapor deposition process, the spin coating process, the casting process, or the LB process, as described above. The vacuum vapor deposition process is preferable since a uniform film can be easily obtained and the possibility of formation of pin holes is small. When the hole injecting layer is formed by the vacuum vapor deposition process, in general, it is preferable that the conditions be suitably selected in the following ranges: the temperature of the source of the deposition: 50 to 450°C; the vacuum: 10-⁷ to 10⁻³ torr; the rate of deposition: 0.01 to 50 nm/second; the temperature of the substrate: -50 to 300°C and the thickness of the film: 5 nm to 5 µm; although the conditions of the vacuum vapor deposition are different depending on the compound to be used (i.e. , the material for the hole injecting layer) and the crystal structure and the recombination structure of the target hole injecting layer.

Then, the light emitting layer is formed on the hole injecting layer. A thin film of the organic light emitting material can be formed by using a desired organic light emitting material by a process such as the vacuum vapor deposition process, the sputtering process, the spin coating process, or the casting process, and the formed thin film is used as the light emitting layer. The vacuum vapor deposition process is preferable since a uniform film can be easily obtained and the possibility of formation of pin holes is small. When the light emitting layer is formed by the vacuum vapor deposition process, in general, the conditions of the vacuum vapor deposition process can be selected in the same ranges as those described for the vacuum vapor deposition of the hole injecting layer, although the conditions are different depending on the used compound.
Next, an electron injecting layer is formed on the light emitting layer formed above. Similarly to the hole injecting layer and the light emitting layer, it is preferable that the electron injecting layer be formed by the vacuum vapor deposition process since a uniform film must be obtained. The conditions of the vacuum vapor deposition can be selected in the same ranges as those described for the vacuum vapor deposition of the hole injecting layer and the light emitting layer.
When the vapor deposition process is used, the aromatic amine compound of the present invention can be deposited by vapor in combination with other materials, although the situation may be different depending on which layer in the light emitting region or in the hole transporting region includes the compound. When the spin coating process is used, the compound can be incorporated into the formed layer by using a mixture of the compound with other materials.
A cathode is formed on the electron injecting layer formed above in the last step, and an organic EL device can be obtained.
The cathode is made of a metal and can be formed by the vacuum vapor deposition process or the sputtering process. It is preferable that the vacuum vapor deposition process be used in order to prevent formation of damages on the lower organic layers during the formation of the film.
In the above-mentioned preparation of the organic EL device, it is preferable that the above-mentioned layers from the anode to the cathode be formed successively while the preparation system is kept in a vacuum after being evacuated once.

The method of forming the layers in the organic EL device of the present invention is not particularly limited. A conventionally known process such as the vacuum vapor deposition process and the spin coating process can be used. The organic thin film layer which is used in the organic EL device of the present invention and includes the aromatic amine compound of the present invention can be formed by a known process such as the vacuum vapor deposition process and the molecular beam epitaxy process (the MBE process) or, using a solution prepared by dissolving the compounds into a solvent, by a coating process such as the dipping process, the spin coating process, the casting process, the bar coating process, or the roll coating process.
The thickness of each layer in the organic thin film layer in the organic EL device of the present invention is not particularly limited. In general, an excessively thin layer tends to have defects such as pin holes, and an excessively thick layer requires a high applied voltage, thereby decreasing the efficiency. Therefore, a thickness in the range of several nanometers to 1 µm is preferable.
The organic EL device which can be fablicated as described above emits light when a direct voltage of 5 to 40 V is applied in the condition that the anode is connected to a positive electrode (+) and the cathode is connected to a negative electrode (-). When the connection is reversed, no electric current is observed and no light is emitted at all. When an alternating voltage is applied to the organic EL device, the uniform light emission is observed only in the condition that the polarity of the anode is positive and the polarity of the cathode is negative. When an alternating voltage is applied to the organic EL device, any type of wave shape can be used.

### EXAMPLES

Next, the present invention will be described in more detail by way of examples.

### Synthesis Example 1 (synthesis of Compound 1)

Compound 1 shown below was synthesized through the following reaction process.

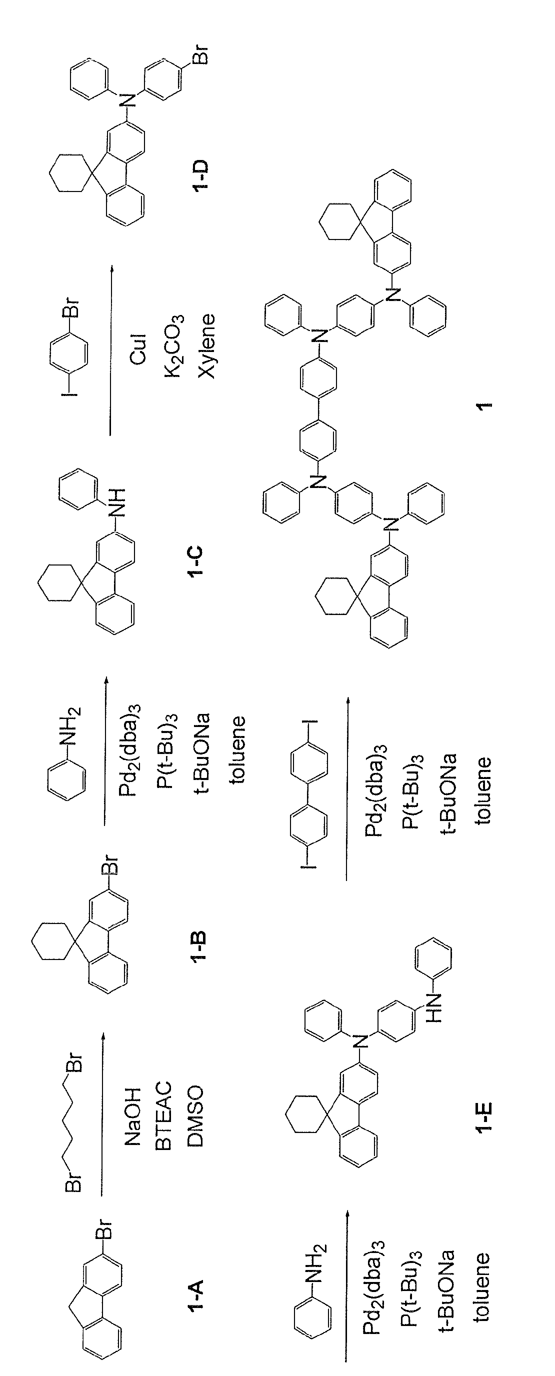

### (1-1) Synthesis of Intermediate (1-B)

Under an ambient atmosphere of argon gas, 100 g of 2-bromofluorene (1-A), 1,200 ml of dimethyl sulfoxide (DMSO), 1.9 g of benzyltriethylammonium chloride, and 130 g of a 50-wt% aqueous solution of sodium hydroxide were loaded into a reaction vessel.
The reaction vessel was placed in a water bath, and 93.8 g of 1,5-dibromopentane were added while the mixture was stirred.
After a reaction for 5 hours, 2,000 ml of water were added, and the whole was extracted with 1,000 ml of toluene. An organic layer was dried with magnesium sulfate, and the solvent was removed by distillation with a rotary evaporator, whereby 97 g of the oil of Intermediate (1-B) were obtained (76% yield).

### (1-2) Synthesis of Intermediate (1-C)

Under an ambient atmosphere of argon gas, 0.16 ml of a 0.66-wt% solution of tri-t-butylphosphine in toluene was added to a solution of 10 g of Intermediate (1-B), 3.6 g of aniline, 0.29 g of tris(benzylideneacetone)dipalladium(0), and 4.3 g of t-butoxysodium in 100 ml of toluene, and the whole was stirred at room temperature for 5 hours. The mixture was subjected to cerite filtration,andthefiltratewasextracted withtoluene. The extract was condensed under reduced pressure, and the resultant coarse product was subjected to column purification, whereby 9.5 g of the pale yellow powder of Intermediate (1-C) were obtained (91% yield).

### (1-3) Synthesis of Intermediate (1-D)

Under an ambient atmosphere of argon gas, a solution of 5.0 g of Intermediate (1-C), 4.4 g of 4-bromoiodobenzene, 0.15 g of copper iodide, and 4.2 g of potassium carbonate in 50 ml of xylene was refluxed with heat for 12 hours. After the mixture had been cooled, water was added to the mixture, and the whole was filtrated. The filtrate was condensed with a rotary evaporator, and the resultant coarse product was subjected to column purification, whereby 4.2 g of the pale yellow powder of Intermediate (1-D) were obtained (56% yield).

### (1-4) Synthesis of Intermediate (1-E)

Under an ambient atmosphere of argon gas, 43 µl of a 0.66-wt% solution of tri-t-butylphosphine in toluene were added to a solution of 4.2 g of Intermediate (1-D), 0.98 g of aniline, 80 mg of tris(benzylideneacetone)dipalladium(0), and 1.2 g of t-butoxysodium in 40 ml of toluene, and the whole was stirred at room temperature for 5 hours. The mixture was subjected to cerite filtration,andthefiltratewasextracted withtoluene. The extract was condensed under reduced pressure, and the resultant coarse product was subjected to column purification, whereby 4.0 g of the pale yellow powder of Intermediate (1-E) were obtained (93% yield). (1-5) Synthesis of Compound 1

Under an ambient atmosphere of argon gas, 18 µl of a 0.66-wt% solution of tri-t-butylphosphine in toluene were added to a solution of 4.0 g of Intermediate (1-E), 1.5 g of 4,4'-diiodobiphenyl, 68 mg of tris(benzylideneacetone)dipalladium(0), and 1.0 g of t-butoxysodium in 30 ml of toluene, and the whole was stirred at room temperature for 5 hours. The mixture was subjected to cerite filtration,and thefiltratewasextracted with toluene. The extract was condensed under reduced pressure, and the resultant coarse product was subjected to column purification, whereby 2.3 g of a pale yellow powder were obtained (55% yield). The mass spectral analysis of the powder confirmed that the powder was a target product and had a ratio m/e of 1,134 with respect to a molecular weight of 1,134.56.

### Synthesis Example 2 (synthesis of Compound 2)

Compound 2 shown below was synthesized through the following reaction process.

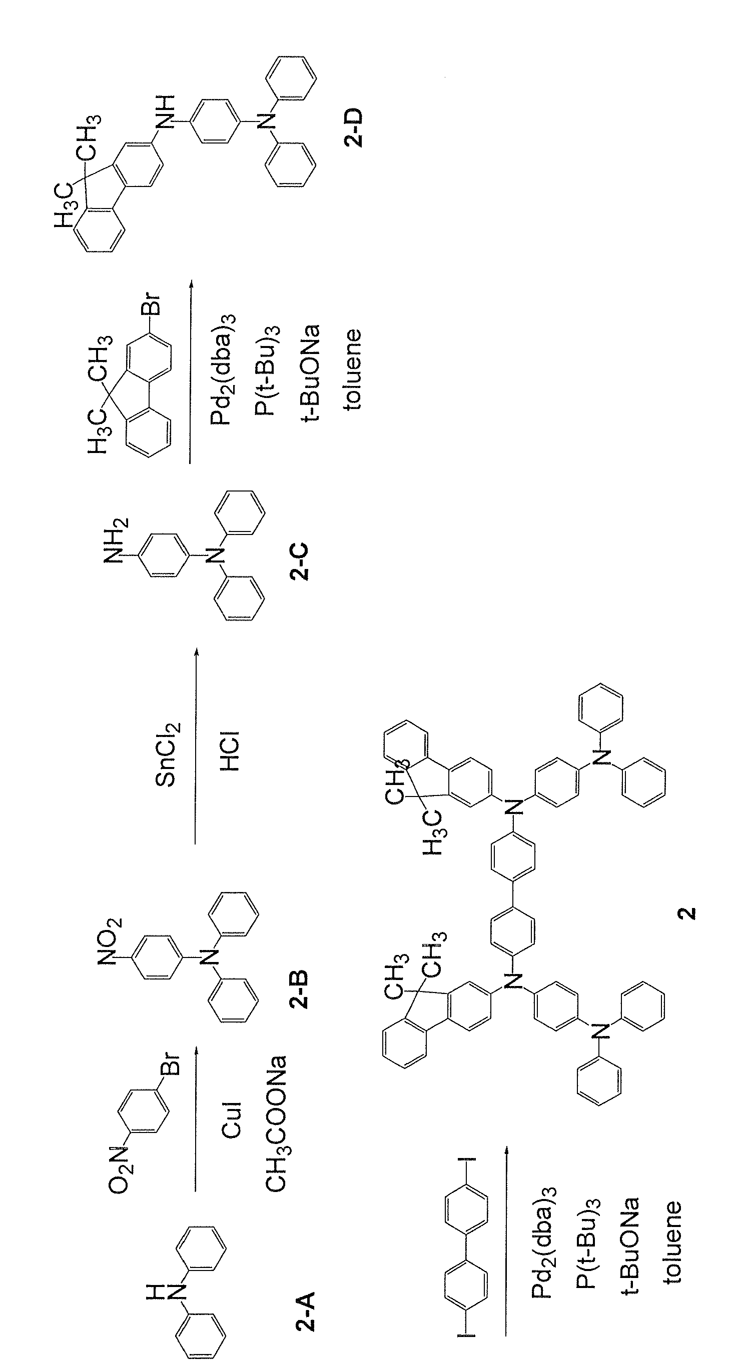

### (2-1) Synthesis of Intermediate (2-B)

Under an ambient atmosphere of argon gas, 10 g of diphenylamine (2-A), 10 g of 4-bromonitrobenzene, 0.94 g of copper iodide, and 8.1 g of sodium acetate were stirred with heat at 200°C for 12 hours. After having been cooled, the mixture was extracted with water and toluene. A water layer was removed, and an organic layer was dried with magnesium sulfate. The dried product was condensed with a rotary evaporator, and the resultant coarse product was subjected to column purification, whereby 11 g of the yellow powder of Intermediate (2-B) were obtained (77% yield).

### (2-2) Synthesis of Intermediate (2-C)

A solution of 34 g of tin (II) chloride dihydrate in 30 ml of concentrated hydrochloric acid was added to a solution of 10 g of Intermediate (2-B) in the mixture of 40 ml of ethanol and 40 ml of toluene, and the whole was refluxed with heat for 5 hours. After the mixture had been cooled, a water layer was removed, and an organic layer was washed with a 10-wt% aqueous solution of sodium hydroxide twice and dried with magnesium sulfate. The solvent was removed by distillation under reduced pressure, and the resultant solid was subjected to column purification, whereby 5.0 g of the brown powder of Intermediate (2-C) were obtained (56% yield).

### (2-3) Synthesis of Intermediate (2-D)

Under an ambient atmosphere of argon gas, 78 µl of a 0.66-wt% solution of tri-t-butylphosphine in toluene were added to a solution of 5.0 g of Intermediate (2-C), 4.4 of 2-bromo-9,9-dimethylfluorene, 0.15 g of tris(benzylideneacetone)dipalladium(0), and 2.2 g of t-butoxysodium in 50 ml of toluene, and the whole was stirred at room temperature for 5 hours. The mixture was subjected to cerite filtration,andthefiltratewasextracted withtoluene. The extract was condensed under reduced pressure, and the resultant coarse product was subjected to column purification, whereby 5.2 g of the pale yellow powder of Intermediate (2-D) were obtained (71% yield).

### (2-4) Synthesis of Compound 2

Under an ambient atmosphere of argon gas, 51 µl of a 0.66-wt% solution of tri-t-butylphosphine in toluene were added to a solution of 5.2 g of Intermediate (2-D), 2.1 g of 4,4'-diiodobiphenyl, 96 mg of tris(benzylideneacetone)dipalladium(0), and 1.4 g of t-butoxysodium in 40 ml of toluene, and the whole was stirred at room temperature for 5 hours. The mixture was subjected to cerite filtration,andthefiltratewasextracted with toluene. Theextract was condensed under reduced pressure, and the resultant coarse product was subjected to column purification, whereby 2.8 g of a pale yellow powder were obtained (52% yield). The mass spectral analysis of the powder confirmed that the powder was a target product and had a ratio m/e of 1,054 with respect to a molecular weight of 1,054.30.

### Synthesis Example 3 (synthesis of Compound 3)

Compound 3 shown below was synthesized through the following reaction process.

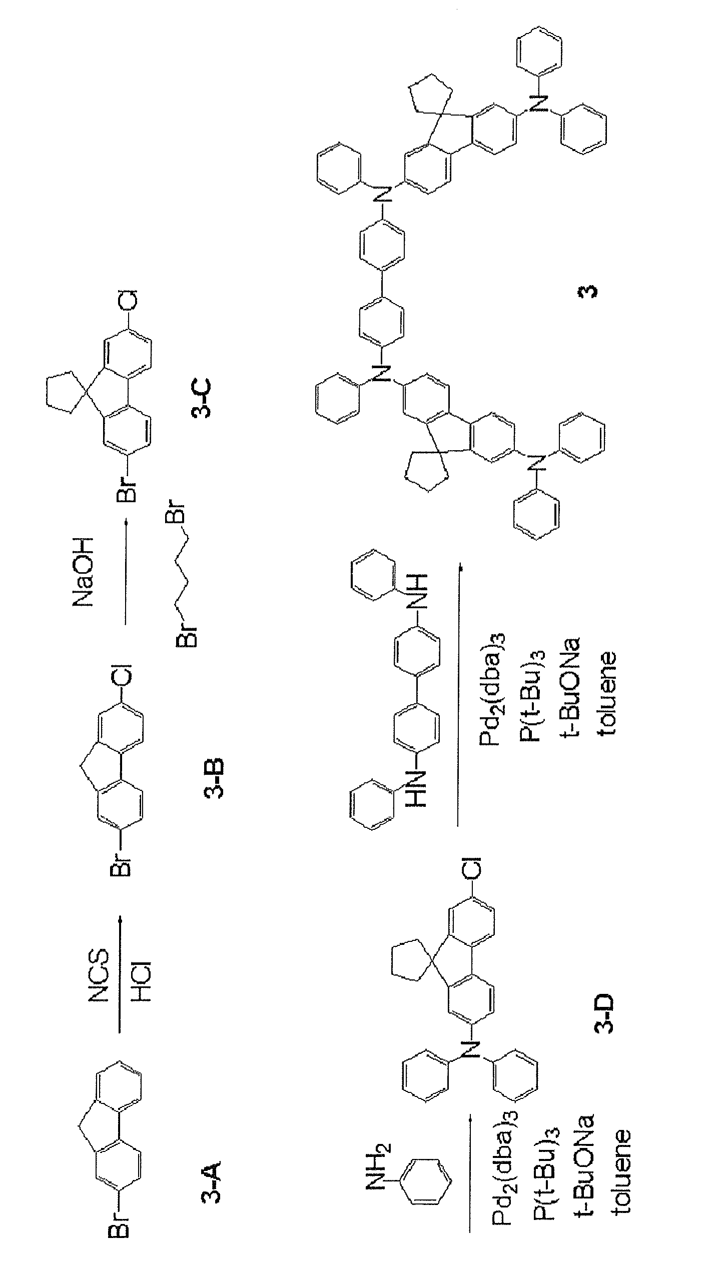

### (3-1) Synthesis of Intermediate (3-B)

2.2 ml of concentrated hydrochloric acid were dropped to a solution of 5.0 g of 2-bromofluorene (3-A) and 3.3 g of N-chlorosuccinimide in 10 ml of acetonitrile under water cooling, and the whole was stirred at room temperature for 5 hours. After the completion of the reaction, the reaction mixture was filtrated. The resultant solid was washed with methanol, whereby 4.9 g of the white powder of Intermediate (3-B) were obtained (86% yield).

### (3-2) Synthesis of Intermediate (3-C)

Under an ambient atmosphere of argon gas, 4.5 g of Intermediate (3-B), 20 ml of DMSO, 80 mg of benzyltriethylammonium chloride, and 3 ml of a 50-wt% aqueous solution of sodium hydroxide were loaded into a reaction solution. The reaction vessel was placed in a water bath, and 3.5 g of 1,4-dibromobutane were added while the mixture was stirred. After a reaction for 5 hours, water was added, and the whole was filtrated. The resultant solid was washed withm ethanol, whereby 4.7 g of the pale yellow solid of Intermediate (3-C) were obtained (88% yield).

### (3-3) Synthesis of Intermediate (3-D)

Under an ambient atmosphere of argon gas, 69 µl of a 0.66-wt% solution of tri-t-butylphosphine in toluene were added to a solution of 4.7 g of Intermediate (3-C), 2.9 g of diphenylamine, 0.13 g of tris(benzylideneacetone)dipalladium(0), and 1.9 g of t-butoxysodium in 30 ml of toluene, and the whole was stirred at room temperature for 5 hours. The mixture was subjected to cerite filtration,andthefiltratewasextracted with toluene. The extract was condensed under reduced pressure, and the resultant coarse product was subjected to column purification, whereby 4.0 g of the pale yellow powder of Intermediate (3-D) were obtained (67% yield).

### (3-4) Synthesis of Compound 3

Under an ambient atmosphere of argon gas, 45 µl of a 0.66-wt% solution of tri-t-butylphosphine in toluene were added to a solution of 4.0 g of Intermediate (3-D), 1.5 g of N,N'-diphenylbenzidine, 83 mg of tris(benzylideneacetone)dipalladium(0), and 1.2 g of t-butoxysodium in 40 ml of toluene, and the whole was refluxed with heat for 5 hours. The mixture was subjected to cerite filtration, and the filtrate was extracted with toluene. The extract was condensed under reduced pressure, and the resultant coarse product was subjected to column purification. After that, the purified product was recrystallized with toluene, whereby 2.2 g of the pale yellow powder of Compound 3 were obtained (21% yield). The mass spectral analysis of the compound confirmed that the compound was a target product and had a ratio m/e of 1,106 with respect to a molecular weight of 1,106.53.

### Synthesis Example 4 (synthesis of Compound 4)

Compound 4 shown below was synthesized through the following reaction process.

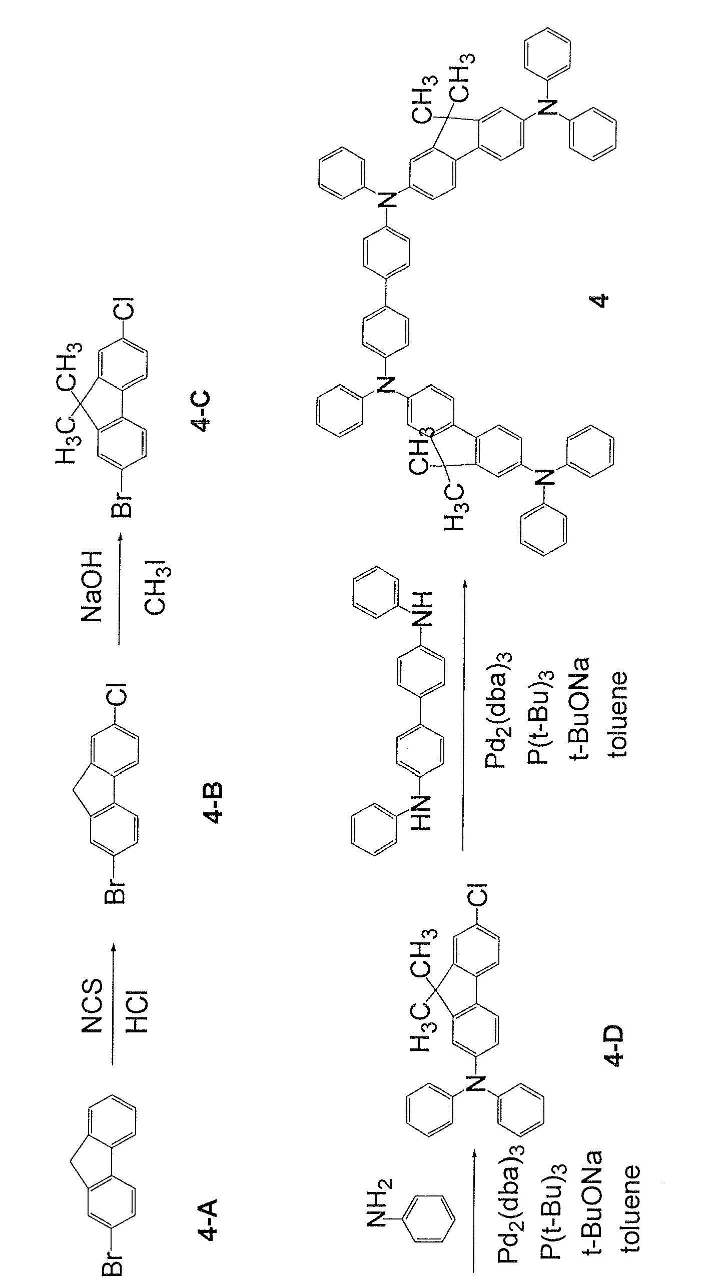

Compound 4 was synthesized in the same manner as in Synthesis Example 3 except that methyl iodide was used instead of 1,4-dibromobutane in the section (3-2) of Synthesis Example 3 (a yield from Compound (4-A) was 18%). The mass spectral analysis of the compound confirmed that the compound was a target product and had a ratiom/e of 1,054 with respect to a molecular weight of 1, 054.50.

### Synthesis Example 5 (synthesis of Compound 5)

Compound 5 shown below was synthesized through the following reaction process.

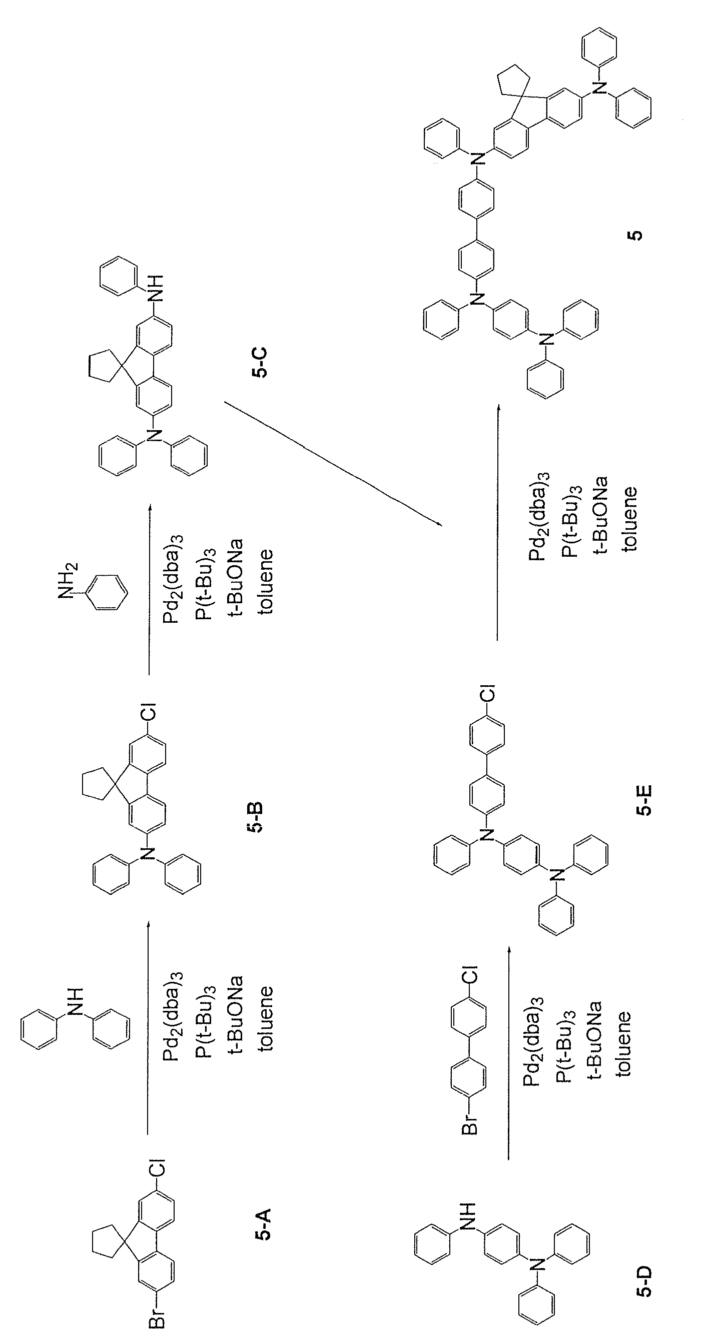

### (5-1) Synthesis of Intermediate (5-C)

Under an ambient atmosphere of argon gas, 58 µl of a 0.66-wt% solution of tri-t-butylphosphine in toluene were added to a solution of 5.0 g of Intermediate (5-B), 1.3 g of aniline, 0.11 g of tris(benzylideneacetone)dipalladium(0), and 1.6 g of t-butoxysodium in 40 ml of toluene, and the whole was refluxed with heat for 5 hours. The mixture was subjected to cerite filtration, and the filtrate was extracted with toluene. The extract was condensed under reduced pressure, and the resultant coarse product was subjected to column purification, whereby 4.2 g of the pale yellow powder of Intermediate (5-C) were obtained (74% yield).

### (5-2) Synthesis of Intermediate (5-D)

4-bromotriphenylamine was synthesized by an Ullmann reaction between diphenylamine and 4-bromoiodobenzene in the presence of a copper catalyst, and was then allowed to react with aniline in the presence of a Pd catalyst, whereby 5.2 g of Intermediate (5-D) were obtained (a yield from 4-bromoiodobenzene was 68%).

### (5-3) Synthesis of Intermediate (5-E)

Under an ambient atmosphere of argon gas, 60 µl of a 0.66-wt% solution of tri-t-butylphosphine in toluene were added to a solution of 5.0 g of Intermediate (5-C), 3.3 g of 4-bromo-4'-chlorobiphenyl, 0.11 g of tris(benzylideneacetone)dipalladium(0), and 1.7 g of t-butoxysodium in 50 ml of toluene, and the whole was stirred at room temperature for 5 hours. The mixture was subjected to cerite filtration, and the filtrate was extracted with toluene. The extract was condensed under reduced pressure, and the resultant coarse product was subjected to column purification, whereby 4.8 g of the pale yellow powder of Intermediate (5-E) were obtained (74% yield).

### (5-4) Synthesis of Compound 5

Under an ambient atmosphere of argon gas, 35 µl of a 0.66-wt% solution of tri-t-butylphosphine in toluene were added to a solution of 4.0 g of Intermediate (5-C), a solution of 3.7 g of Intermediate (5-E), 65 mg of tris(benzylideneacetone)dipalladium(0), and 0.95 g of t-butoxysodium in 40 ml of toluene, and the whole was refluxed with heat for 5 hours. The mixture was subjected to cerite filtration, and the filtrate was extracted with toluene. The extract was condensed under reduced pressure, and the resultant coarse product was subjected to column purification, whereby 4.1 g of the pale yellow powder of Compound 5 were obtained (60% yield). The mass spectral analysis of the powder confirmed that the powder was a target product and had a ratio m/e of 964 with respect to a molecular weight of 964.45.

### Synthesis Example 6 (synthesis of Compound 6)

Compound 6 shown below was synthesized through the following reaction process.

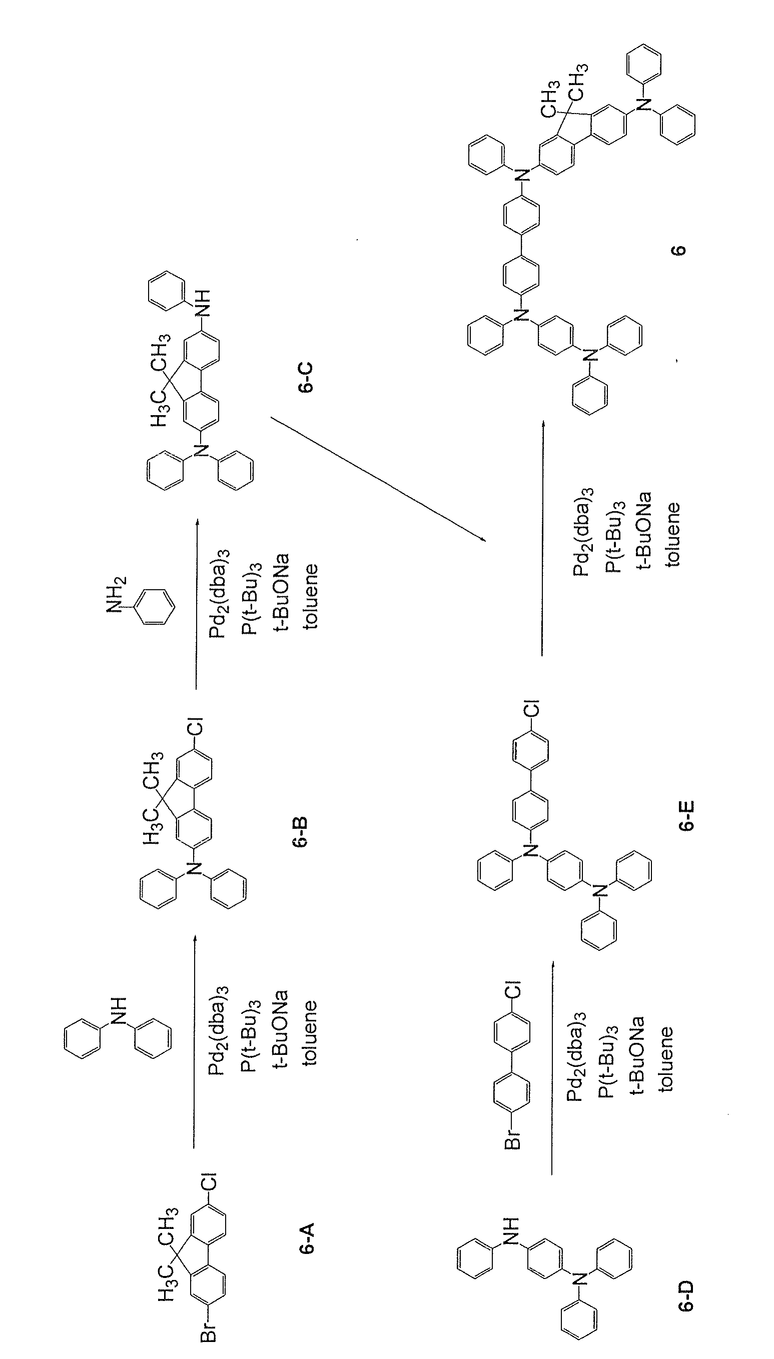

Compound 6 was synthesized in the same manner as in Synthesis Example 5 except that Compound (6-A) was used instead of Compound (5-A) in Synthesis Example 5 (a yield from Compound (6-A) was 15%). The mass spectral analysis of the compound confirmed that the compound was a target product and had a ratio m/e of 938 with respect to a molecular weight of 938.43.

### Synthesis Example 7 (synthesis of Compound 7)

Compound 7 shown below was synthesized through the following reaction process.

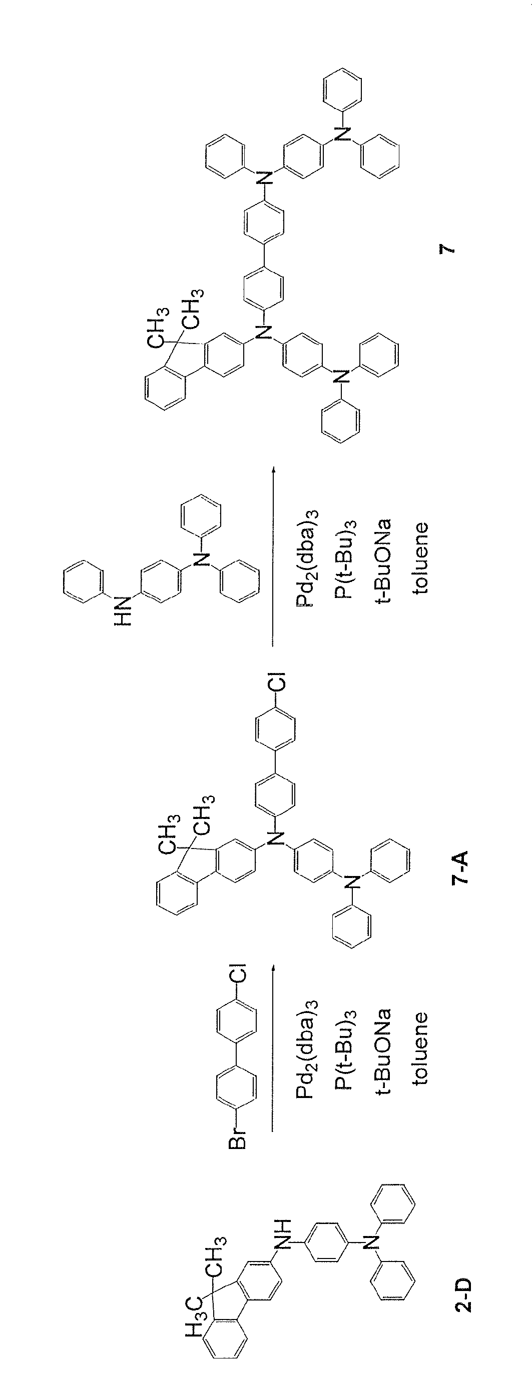

### (7-1) Synthesis of Intermediate (7-A)

Under an ambient atmosphere of argon gas, 45 µl of a 0.66-wt% solution of tri-t-butylphosphine in toluene were added to a solution of 5.0 g of Intermediate (2-D), 2.5 g of 1-bromo-chlorobiphenyl, 0.084 g of tris(benzylideneacetone)dipalladium(0), and 1.2 g of t-butoxysodium in 40 ml of toluene, and the whole was stirred at room temperature for 5 hours. The mixture was subjected to cerite filtration,andthe filtratewasextracted with toluene. Theextract was condensed under reduced pressure, and the resultant coarse product was subjected to column purification, whereby 4.2 g of the pale yellow powder of Intermediate (7-A) were obtained (71% yield).

### (7-2) Synthesis of Compound 7

Under an ambient atmosphere of argon gas, 32 µl of a 0.66-wt% solution of tri-t-butylphosphine in toluene were added to a solution of 4.2 g of Intermediate (7-A), 2.7 g of N,N,N'-triphenyl-1,4-phenylenediamine, 0.060 g of tris(benzylideneacetone)dipalladium(0), and 0.88 g of t-butoxysodium in 40 ml of toluene, and the whole was stirred with heat for 5 hours. The mixture was subjected to cerite filtration, and the filtrate was extracted with toluene. The extract was condensed under reduced pressure, and the resultant coarse product was subjected to column purification, whereby 3.8 g of the pale yellow powder of Compound 7 were obtained (61% yield). The mass spectral analysis of the powder confirmed that the powder was a target product and had a ratio m/e of 938 with respect to a molecular weight of 938.43.

### Synthesis Example 8 (synthesis of Compound 8)

Compound 8 shown below was synthesized through the following reaction process.

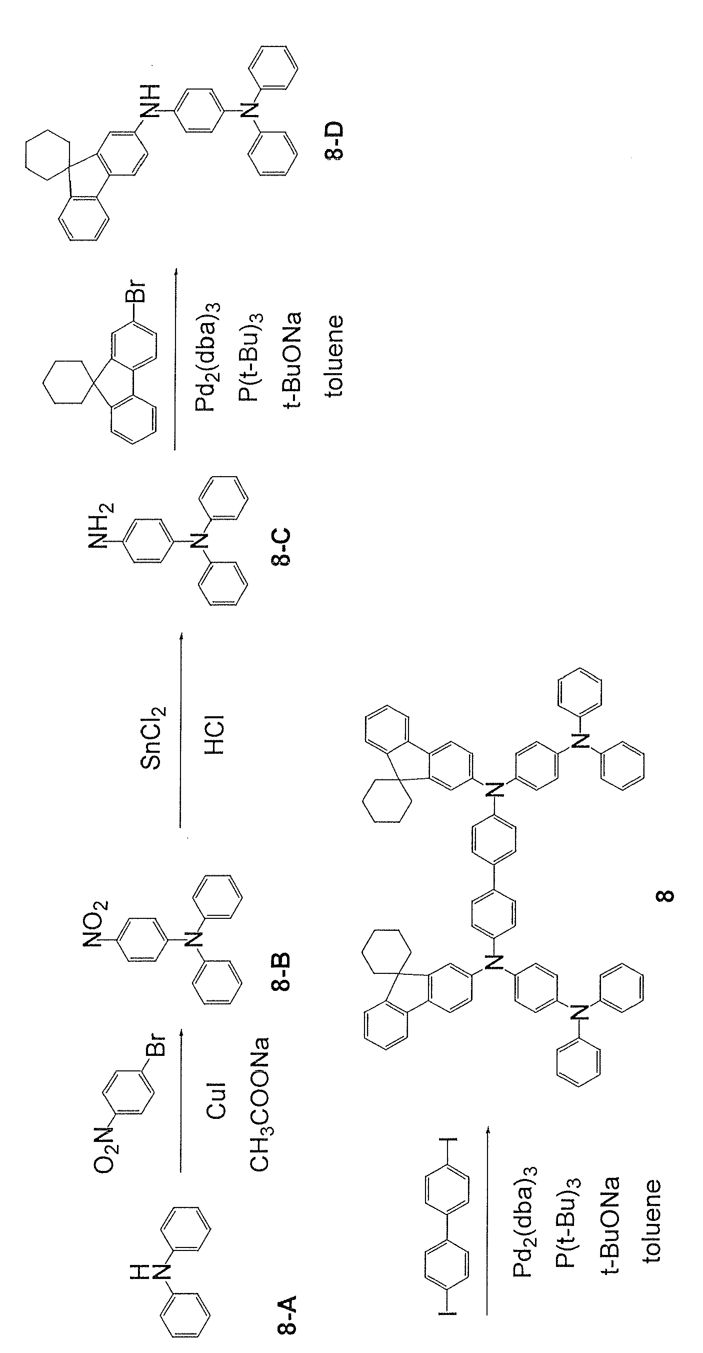

Compound 8 was synthesized in the same manner as in Synthesis Example 2 except that 2-bromospiro[cyclohexane-1,9'-fluorene] was used instead of 2-bromo-9,9-dimethylfluorene in the section (2-3) of Synthesis Example 2 (a yield from Compound (8-A) was 16%). The mass spectral analysis of the compound confirmed that the compound was a target product and had a ratio m/e of 1,135 with respect to a molecular weight of 1,035.56.

### Synthesis Example 9 (synthesis of Compound 9)

Compound 9 shown below was synthesized through the following reaction process.

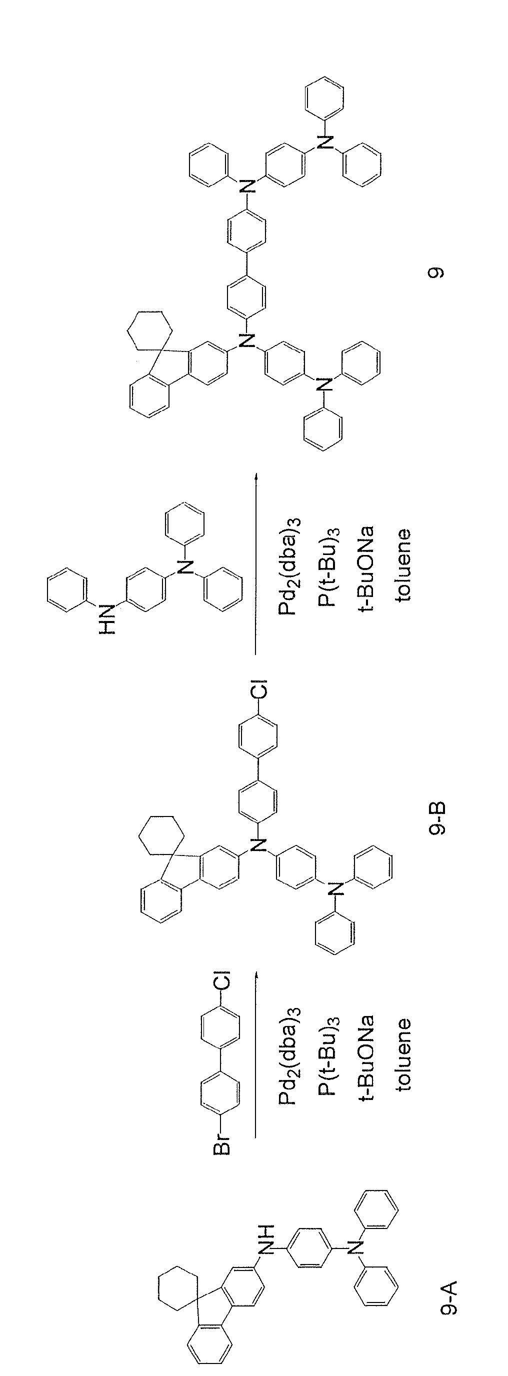

Compound 9 was synthesized in the same manner as in Synthesis Example 7 except that 2' -bromospiro[cyclohexane-1,9'-fluorene] was used instead of Intermediate (2-D) in the section (7-1) of Synthesis Example 7 (a yield from Compound (9-A) was 12%). The mass spectral analysis of the compound confirmed that the compound was a target product and had a ratio m/e of 978 with respect to a molecular weight of 978.47.

### Synthesis Example 10 (synthesis of Compound 10)

Compound 10 shown below was synthesized through the following reaction process.

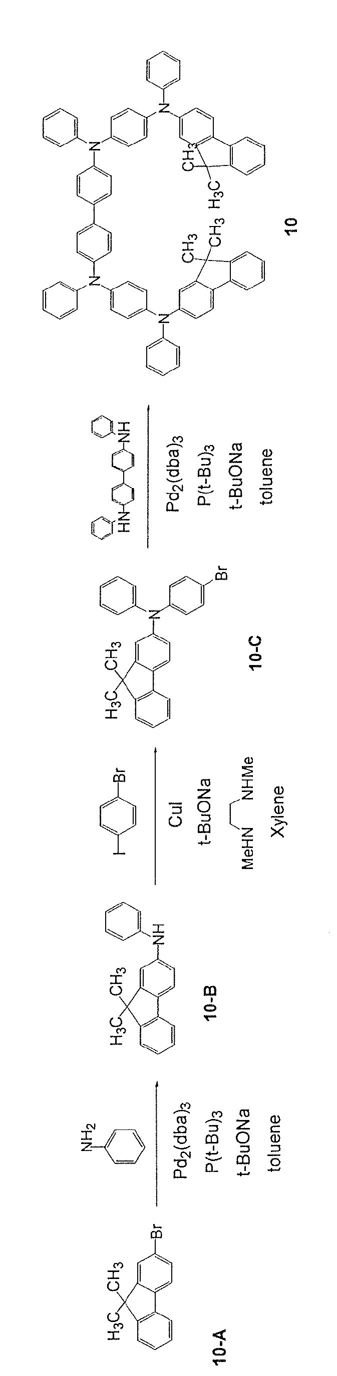

### (1) Synthesis of Intermediate (10-B)

Under an ambient atmosphere of argon gas, 1.0 ml of a 0.66-wt% solution of tri-t-butylphosphine in toluene were added to 27.3 g of 2-bromo-9,9-dimethylfluorene (Compound(10-A)), 11.2g of aniline, 1.83 g of tris(benzylideneacetone)dipalladium(0), and 13.5 g of t-butoxysodium in 200 ml of toluene, and the whole was stirred at room temperature for 5 hours. The mixture was subjected to cerite filtration,andthefiltratewasextracted with toluene. The extract was condensed under reduced pressure, and the resultant coarse product was subjected to column purification, whereby 22.8 g of the white powder of Intermediate (10-B) were obtained.

### (2) Synthesis of Intermediate (10-C)

8.8 g of N, N ' -dimethylethylenediamine were added to a solution of 14.3 g of Intermediate (10-B), 14.1 g of 4-bromoiodobenzene, 7.20 g of t-butoxysodium, and 1.90 g of a copper powder in 50 ml of xylene, and the whole was refluxed with heat for 24 hours Under an ambient atmosphere of argon gas. After having been cooled to room temperature, the mixture was filtrated so that insoluble matter would be removed. Then, the filtrate was condensed. The residue was purified by means of silica gel column chromatography, whereby 16.5 g of Intermediate (10-C) were obtained.

### (3) Synthesis of Compound 10

Under an ambient atmosphere of argon gas, 0.20 ml of a 0.66-wt% solution of trit-butylphosphine in toluene was added to a solution of 9.68 g of Intermediate (10-C), 3.36 g of N,N'-diphenylbenzidine, 0.366 g of tris(benzylideneacetone)dipalladium(0), and 2.69 g of t-butoxysodium in 50 ml of toluene, and the whole was stirred at 80°C for 5 hours. After having been cooled to room temperature, the mixture was subjected to cerite filtration, and the filtrate was extracted with toluene. The extract was condensed under reduced pressure, and the resultant coarse product was subjected to column purification, whereby 8.26 g of the yellow powder of Compound 10 were obtained. The mass spectral analysis of the compound confirmed that the compound was a target product and had a ratio m/e of 1, 054 with respect to a molecular weight of 1,054.50.

### Example 1

A glass substrate provided with an ITO transparent electrode measuring 25 mm wide by 75 mm long by 1.1 mm thick (manufactured by GEOMATEC Co., Ltd.) was subjected to ultrasonic cleaning in isopropyl alcohol for 5 minutes. After that, the substrate was subjected to UV ozone cleaning for 30 minutes. The glass substrate provided with a transparent electrode line after the washing was mounted on the substrate holder of a vacuum vapor deposition apparatus. First, Compound 1 as a hole injecting material was formed into a film having a thickness of 60 nm by resistance heating deposition on a surface on the side where the transparent electrode line was formed in such a manner that the film would cover the transparent electrode. The film of Compound 1 functions as a hole injecting layer. Next, 4,4'-bis[N-(1-naphthyl)-N-phenylamino]biphenyl as a hole transporting material was formed into a film having a thickness of 20 nm (hereinafter abbreviated as "NPD film") by resistance heating deposition on the film of Compound I. The NPD film functions as a hole transporting layer. Further, 9-(2-naphthyl)-10-[4-(1-naphthyl)phenyl]anthracene (hereinafter abbreviated as "AN-1") was formed into a filmhaving a thickness of 40 nm by resistance heating deposition on the NPD film. Simultaneously with the formation, Amine Compound D-1 having a styryl group shown below as a light emitting molecule was deposited from the vapor at a weight ratio between Amine Compound D-1 and AN-1 of 2 : 40. The film functions as a light emitting layer. An Alq film having a thickness of 10 nm was formed on the film. The Alq film functions as an electron injecting layer. After that, Li as a reducing dopant (Li source: manufactured by SAES Getters) and Alq were co-deposited from the vapor, whereby an Alq:Li film (having a thickness of 10 nm) was formed as an electron injecting layer (cathode). Metal Al was deposited from the vapor onto the Alq: Li film to form a metal cathode, whereby an organic EL device was fabricated.
Table 1 shows the results of the measurement of the current density and current efficiency of the resultant device upon energization with a voltage of 13 (V), and shows the luminescent color of the device. In addition, Table 1 shows the half lifetime (hours) of the device having an initial luminance of 1,000 cd/m² upon driving at a constant current.

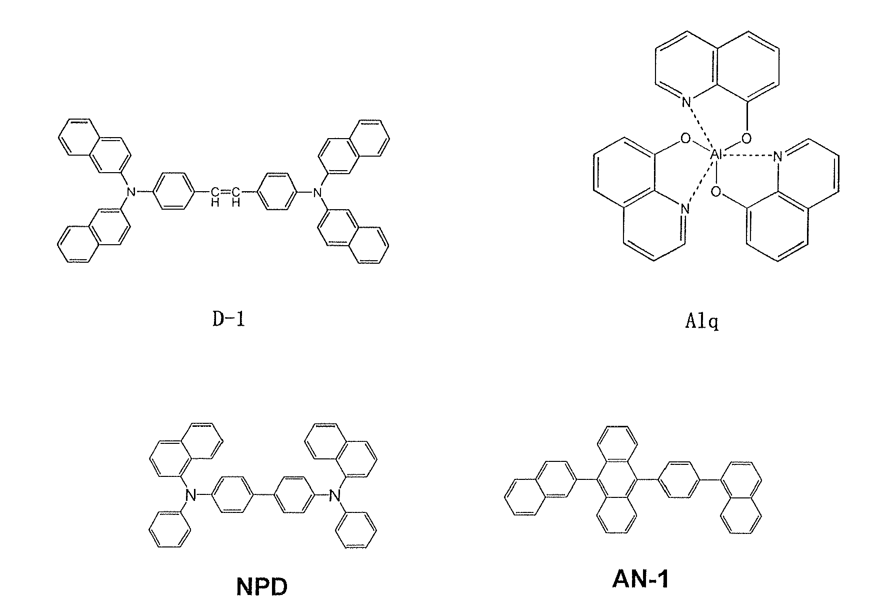

### Examples 2 to 8

Organic EL devices were each fabricated in the same manner as in Example 1 except that any one of the compounds shown in Table 1 was used instead of Compound 1 as a hole injecting material of which a hole injecting layer was formed.
Table 1 shows the results of the measurement of the current density and current efficiency of the resultant device upon energization with a voltage of 13 (V), and shows the luminescent color of the device. In addition, Table 1 shows the half lifetime (hours) of the device having an initial luminance of 1,000 cd/m² upon driving at a constant current.

### Comparative Examples 1 and 2

Organic EL devices were each fabricated in the same manner as in Example 1 except that an N,N'-bis(N,N'-diphenyl-4-aminophenyl)-N,N-diphenyl-4,4'-diamin o-1,1'-biphenyl film (TPD 232 film) shown below (Comparative Example 1) or Compound (A) shown below (Comparative Example 2) was used instead of Compound 1 as a hole injecting material of which a hole injecting layer was formed.
Table 1 shows the results of the measurement of the current density and current efficiency of each of the resultant devices upon energization with a voltage of 13 (V), and shows the luminescent color of each of the devices. In addition, Table 1 shows the half lifetime (hours) of each of the devices each having an initial luminance of 1,000 cd/m² upon driving at a constant current.

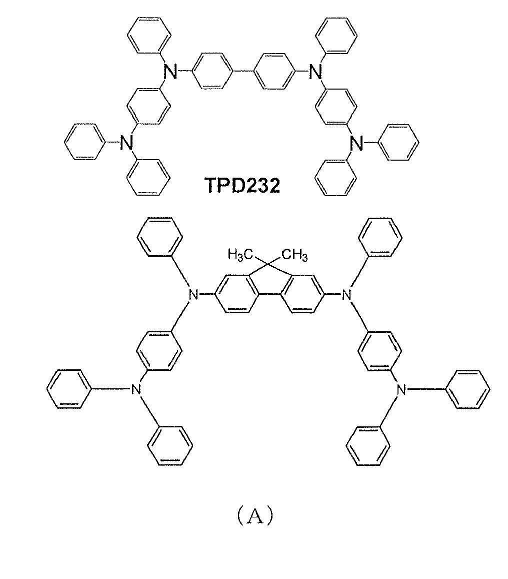

**[Table 1]**

| | Hole injecting material | Current density (mA/cm²) @13V | Current efficiency (cd/A) | Luminescent color | Half lifetime (hours) @1000 (cd/m²) |
|---|---|---|---|---|---|
| Example 1 | Compound 1 | 530 | 4.8 | Blue | 5500 |
| Example 2 | Compound 2 | 570 | 4.7 | Blue | 4200 |
| Example 3 | Compound 5 | 520 | 5.1 | Blue | 4400 |
| Example 4 | Compound 6 | 560 | 5.1 | Blue | 5600 |
| Example 5 | Compound 7 | 570 | 4.8 | Blue | 5000 |
| Example 6 | Compound 8 | 520 | 4.7 | Blue | 5000 |
| Example 7 | Compound 9 | 520 | 5.1 | Blue | 5200 |
| Example 8 | Compound 10 | 572 | 4.8 | Blue | 5000 |
| Comparative example 1 | TPD232 | 250 | 4.8 | Blue | 3000 |
| Comparative example 2 | Compound (A) | 520 | 4.9 | Blue | 2500 |

As shown in Table 1, an organic EL device using the compound of the present invention in its hole injecting layer has a long lifetime, and shows high current efficiency because of its high hole injecting property and high hole transporting property. In addition, TPD 232 has a Tg of 111°C, and Compound (A) has a Tg of 117°C while Compounds 1, 2, 5, and 6 to 9 of the present invention each have a Tg of 130°C or higher. That is, the organic EL device using the compound of the present invention is thermally stable.

### Example 9

An organic EL device was fabricated in the same manner as in Example 9 except that Compound 4 was used instead of compound 3 as a hole transporting material of which a hole transporting layer was formed.
Table 2 shows the results of the measurement of the current density and current efficiency of the resultant device upon energization with a voltage of 13 (V), and shows the luminescent color of the device. In addition, Table 2 shows the half lifetime (hours) of the device having an initial luminance of 1,000 cd/m² upon driving at a constant current.

### Example 10

An organic EL device was fabricated in the same manner as in Example 9 except that Compound 4 was used instead of Compund 3 as a hole transporting material of which a hole transporting layer was formed.
Table 2 shows the results of the measurement of the current density and current efficiency of the resultant device upon energization with a voltage of 13 (V), and shows the luminescent color of the device. In addition, Table 2 shows the half lifetime (hours) of the device having an initial luminance of 1,000 cd/m² upon driving at a constant current.

### Comparative Example 3 to 5

An organic EL device was fabricated in the same manner as in Example 9 except that Compound 4 was used instead of Compound 3 as a hole transporting material of which a hole transporting layer was formed NPD (Comparative example 3), Compound (B) described below (Comparative example 4), and Compound (C) described below (Comparative example 5).
Table 2 shows the results of the measurement of the current density and current efficiency of the resultant device upon energization with a voltage of 13 (V), and shows the luminescent color of the device. In addition, Table 2 shows the half lifetime (hours) of the device having an initial luminance of 1,000 cd/m² upon driving at a constant current.

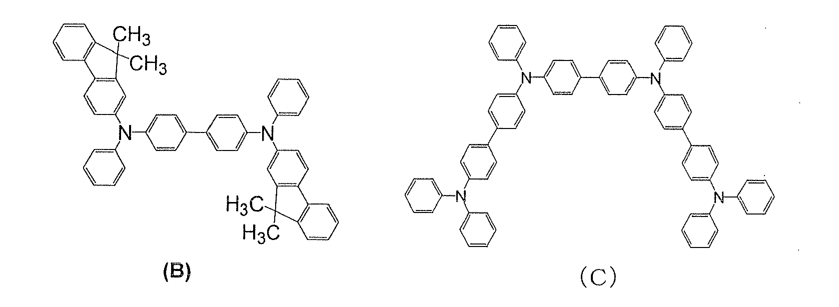

**[Table 2]**

| | Hole transporting material | Current density (mA/cm²) @13V | Current efficiency (cd/A) | Luminescent color | Half lifetime (hours) @1000(cd/m²) |
|---|---|---|---|---|---|
| Example 9 | Compound 3 | 530 | 4.8 | Blue | 5500 |
| Example 10 | Compound 4 | 570 | 4.7 | Blue | 5000 |
| Comparative example 3 | NPD | 250 | 4.8 | Blue | 3000 |
| Comparative example 4 | Compound (B) | 560 | 4.8 | Blue | 3000 |
| Comparative example 5 | Compound (C) | 220 | 4.8 | Blue | 2500 |

As shown in Table 2, the organic EL devices of Examples 9 and 10 each using the compound of the present invention in its hole transporting layer each have high hole injecting property and high hole transporting property, and each have a lifetime much longer than that of Comparative Example 4. In addition, NPD has a Tg of 95°C, and Compound (B) has a Tg of 120°C while Compound 3 of the present invention has a Tg of 157°C, and Compound 4 of the present invention has a Tg of 158°C. That is, each organic EL device using the compound of the present invention is thermally stable.

### Example 11

A glass substrate provided with an ITO transparent electrode measuring 25 mm wide by 75 mm long by 1.1 mm thick (manufactured by GEOMATEC Co., Ltd.) was subjected to ultrasonic cleaning in isopropyl alcohol for 5 minutes. After that, the substrate was subjected to UV ozone cleaning for 30 minutes. The glass substrate provided with a transparent electrode line after the washing was mounted on the substrate holder of a vacuum vapor deposition apparatus. First, a TPD 232 film having a thickness of 60 nm was formed on a surface on the side where the transparent electrode line was formed so as to cover the transparent electrode. The TPD 232 film functions as a hole injecting layer. Next, Compound 3 was formed into a film having a thickness of 20 nm on the TPD 232 film. The film functions as a hole transporting layer. Further, 4,4'-bis(carbazolyl)biphenyl (hereinafter abbreviated as "CBP") was deposited from the vapor, whereby a film having a thickness of 40 nm was formed. Simultaneously with the formation, tris(2-phenylpyridine)iridium (hereinafter abbreviated as "Ir(ppy)3") was added as a phosphorescent Ir metal complex dopant. The concentration of Ir(ppy)3 in a light emitting layer was set to 5 wt%. (1,1'-bisphenyl)-4-olato)bis(2-methyl-8-quinolinolato) aluminum shown below was formed into a film having a thickness of 10 nm (hereinafter abbreviated as "BAlq film") on the film. The BAlq film functions as a hole blocking layer. Further, an Alq film having a thickness of 40 nm was formed on the film. The Alq film functions as an electron injecting layer. After that, LiF as an alkali metal halide was deposited from the vapor to have a thickness of 0.2 nm, and then aluminum was deposited from the vapor to have a thickness of 150 nm. The Al/LiF film serves as a cathode. Thus, an organic EL device was fabricated.
The application of a voltage to the resultant device resulted in the emission of heterogeneous green light. Accordingly, the compound of the present invention can be used also as a material for the hole transporting layer of a phosphorescent device.

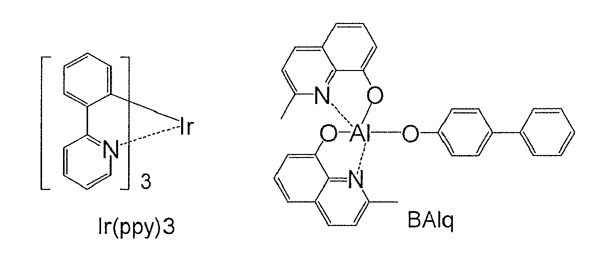

### Example 12

A glass substrate provided with an ITO transparent electrode measuring 25 mm wide by 75 mm long by 1.1 mm thick (manufactured by GEOMATEC Co., Ltd.) was subjected to ultrasonic cleaning in isopropyl alcohol for 5 minutes. After that, the substrate was subjected to UV ozone cleaning for 30 minutes. Polyethylene dioxythiophene/polystyrene sulfonate (PEDOT/PSS) to be used in a hole injecting layer was formed into a film having a thickness of 100 nm by a spin coating method on the substrate. Next, a solution of Compound 4 in toluene was formed into a hole transporting layer by a spin coating method on the PEDOT/PSS film. At this time, the film had a thickness of 10 nm. AN-1 described above was formed into a film having a thickness of 30 nm by resistance heating deposition on the film of Compound 4. Simultaneously with the formation, Amine Compound D-1 having a styryl group as a light emitting molecule was deposited from the vapor at a weight ratio between Amine Compound D-1 and AN-1 of 2 : 40. The film functions as a light emitting layer. An Alq film having a thickness of 10 nm was formed on the film. The Alq film functions as an electron injecting layer. After that, Li as a reducing dopant (Li source: manufactured by SAES Getters) and Alq were co-deposited from the vapor, whereby an Alq:Li film was formed as an electron injecting layer (cathode). Metal Al was deposited from the vapor onto the Alq: Li film to form a metal cathode, whereby an organic EL device was fabricated.
A current having a current density of 10 mA/cm² flowed in the resultant device at a DC voltage of 6.0 V, and the device was observed to emit blue light having an emission luminance of 280 cd/m². The device had a current efficiency of 2.8 cd/A.

### INDUSTRIAL APPLICABILITY

As described above in detail, an organic EL device using the aromatic amine compound of the present invention shows various hues of light emission and has high heat resistance. In particular, when the aromatic amine compound of the present invention is used as a hole injecting/transporting material, hole injecting/transporting property is high, and high emission luminance, high emission efficiency, and a long lifetime can be obtained. Therefore, the organic EL device of the present invention has high practicability, and is useful for the flat luminous element of a wall hanging television or for a light source such as the backlight of a display.

## Claims

1. An aromatic amine compound represented by the following general formula (1): 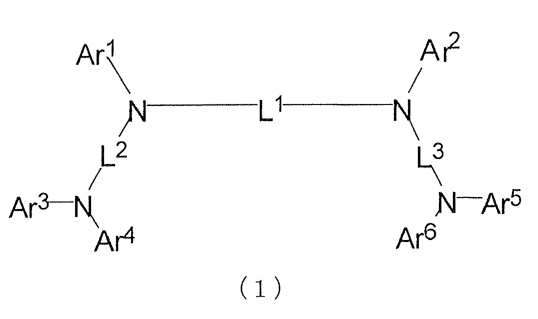 wherein Ar¹ to Ar⁶ each independently represent a substituted or unsubstituted aryl group having 5 to 60 ring carbon atoms, or a substituted or unsubstituted heteroaryl group having 3 to 50 ring carbon atoms; and
L¹ to L³ each independently represent a substituted or unsubstituted arylene group having 5 to 60 ring carbon atoms, or a substituted or unsubstituted heteroarylene group having 3 to 50 ring carbon atoms;
provided that the general formula (1) satisfies at least one of the following conditions (i) and (ii):
(i) at least one of Ar¹ to Ar⁶ represents a substituted or unsubstituted fluorenyl-containing group; and
(ii) at least one of L² and L³ represents a substituted or unsubstituted fluorenylene-containing group.

2. An aromatic amine compound according to claim 1, wherein at least one of Ar¹ to Ar⁶ in the general formula (1) represents a fluorenyl-containing group represented by the following general formula (1-a): 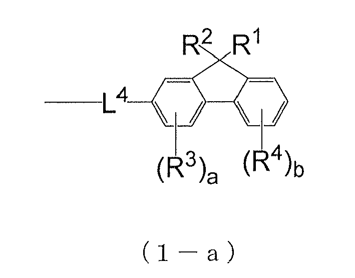 wherein R¹ and R² each independently represent a hydrogen atom or a substituent, and R¹ and R² may bond each other to form a cyclic structure;
R³ and R⁴ each independently represent a substituent, **a** represents an integer of 0 to 3, **b** represents an integer of 0 to 4, and, when multiple R³' s are present, R³' s may bond each other to form a cyclic structure, or, when multiple R⁴'s are present, R⁴'s may bond each other to form a cyclic structure; and
L⁴ represents a single bond, a substituted or unsubstituted arylene group having 5 to 60 ring carbon atoms, or a substituted or unsubstituted heteroarylene group having 3 to 50 ring carbon atoms.

3. An aromatic amine compound according to claim 2, wherein the fluorenyl-containing group represented by the general formula (1-a) is a fluorenyl-containing group represented by the following general formula (1-b): 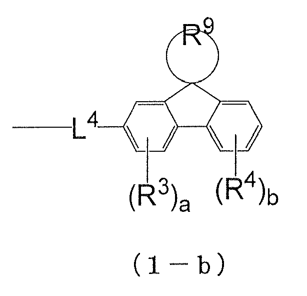 wherein R⁹ represents an atomic group forming a cyclic structure and further, R³, R⁴, **a**, **b**, and L⁴ are the same as described above.

4. An aromatic amine compound according to claim 1, wherein at least one of L² and L³ in the general formula (1) represents a fluorenylene-containing group represented by the following general formula (2-a): 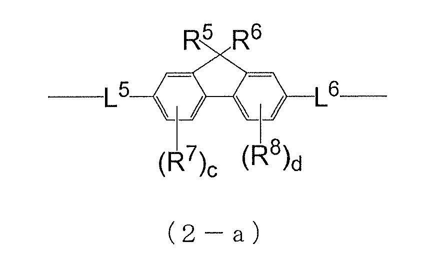 wherein R⁵ and R⁶ each independently represent a hydrogen atom or a substituent, and R⁵ and R⁶ may bond each other to form a cyclic structure;
R⁷ and R⁸ each independently represent a substituent; **c** and **d** each represent an integer of 0 to 3 and further, when multiple R⁷'s are present, R⁷'s may bond each other to form a cyclic structure, or, when multiple R⁸'s are present, R⁸' s may bond each other to form a cyclic structure; and
L⁵ and L⁶ each independently represent a single bond, a substituted or unsubstituted arylene group having 5 to 60 ring carbon atoms, or a substituted or unsubstituted heteroarylene group having 3 to 50 ring carbon atoms.

5. An aromatic amine compound according to claim 3, wherein the fluorenylene-containing group represented by the general formula (2-a) is a fluorenylene-containing group represented by the following general formula (2-b): 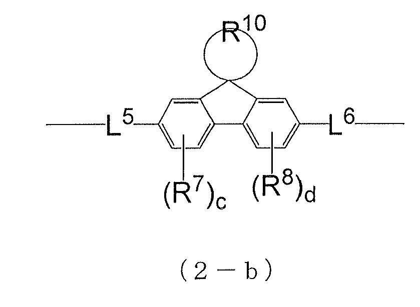 wherein R¹⁰ represents an atomic group forming a cyclic structure and further, R⁷, R⁸, **c, d,** L⁵, and L⁶ are the same as described above.

6. An aromatic amine compound according to claim 1, which is a material for organic electroluminescence device.

7. An aromatic amine compound according to claim 6, wherein the aromatic amine compound comprises a hole transporting material for an organic electroluminescence device or a hole injecting material for an organic electroluminescence device.

8. An aromatic amine compound according to claim 6, which is a light emitting material for an organic electroluminescence device.

9. An organic electroluminescence device which comprises one or more organic thin film layers comprising at least one light emitting layer sandwiched between a cathode and an anode,
wherein at least one of the thin film layers comprises the aromatic amine compound according to claim 1 singly or as its mixture component.

10. An organic electroluminescence device according to claim 9, wherein the organic thin film layers have at least one of a hole transporting region and a hole injecting region; and
the aromatic amine compound is incorporated into at least one of the hole transporting region and the hole injecting region.

11. An organic electroluminescence device according to claim 9, wherein the organic thin film layers have at least one of a hole transporting layer and a hole injecting layer; and
the aromatic amine compound is incorporated into at least one of the hole transporting layer and the hole injecting layer.

12. An organic electroluminescence device according to claim 11, wherein at least one of the hole transporting layer and the hole injecting layer essentially comprises the aromatic amine compound.

13. An organic electroluminescence device according to claim 9, wherein the layer comprising the aromatic amine compound contacts with the anode.

14. An organic electroluminescence device according to claim 9, wherein the layer contacting with the anode comprises the aromatic amine compound as its essential component.

15. An organic electroluminescence device according to claim 9, wherein the organic thin film layers have a layer comprising the aromatic amine compound and a light-emitting material.

16. An organic electroluminescence device according to claim 9, wherein the organic thin film layer has a laminated layers formed of at least one of a hole transporting layer comprising the aromatic amine compound and a hole injecting layer comprising the aromatic amine compound, and further, formed of a light emitting layer composed of a phosphorescent metal complex and a host material.
